# EUROPEAN PATENT SPECIFICATION

(11) **EP 4 004 001 B1**
(45) Date of publication and mention of the grant of the patent: **03.09.2025**
(21) Application number: 20846788.6
(22) Date of filing: 28.07.2020
(51) Int. Cl.: C07D 495/04, C07D 333/00, A61K 31/519, A61K 31/47, A61P 5/04, A61P 5/24, A61P 13/08, A61P 15/00, A61P 15/08, A61P 15/18, A61P 17/10, A61P 17/14, A61P 35/00, A61P 37/08

(54) **SUBSTITUTED PYRIMIDINEDIONE COMPOUNDS AND USES THEREOF**
SUBSTITUIERTE PYRIMIDINDIONVERBINDUNGEN UND VERWENDUNGEN DAVON
COMPOSÉS DE PYRIMIDINEDIONE SUBSTITUÉS ET LEURS UTILISATIONS

(30) Priority: 29.07.2019 CN 201910686491; 14.04.2020 CN 202010290629
(43) Date of publication of application: 01.06.2022
(73) Proprietor: Sunshine Lake Pharma Co., Ltd., Dongguan, Guangdong 523000 (CN)
(72) Inventor: JIN, Chuanfei, Dongguan, Guangdong 523871 (CN); ZHONG, Wenhe, Dongguan, Guangdong 523871 (CN)
(74) Representative: Hoffmann Eitle
(86) International application number: PCT/CN2020/105014
(87) International publication number: WO 2021/018105

(56) References cited:
- WO-A1-00/56739
- WO-A1-2007/011072
- WO-A1-97/40846
- WO-A2-2014/051164
- CN-A- 1 146 206
- CN-A- 1 173 868
- CN-A- 1 768 065
- JP-A- H09 208 496
- US-A- 5 977 132
- US-A1- 2005 222 174
- US-A1- 2005 250 793
- KAZUHIRO MIWA ET AL: "Discovery of 1-{4-[1-(2,6-Difluorobenzyl)-5-[(dimethylamino)methyl]-3-(6-methoxypyridazin-3-yl)-2,4-dioxo-1,2,3,4-tetrahydrothieno[2,3- d ]pyrimidin-6-yl]phenyl}-3-methoxyurea (TAK-385) as a Potent, Orally Active, Non-Peptide Antagonist of the Human Gonadotropin-Releasing Hormone Receptor", JOURNAL OF MEDICINAL CHEMISTRY, vol. 54, no. 14, 28 July 2011 (2011-07-28), pages 4998 - 5012, XP055012890, ISSN: 0022-2623, DOI: 10.1021/jm200216q
- ZHIQIANG GUO , YONGSHENG CHEN , DONGPEI WU , YUN-FEI ZHU , R SCOTT STRUTHERS , JOHN SAUNDERS , QIU XIE , CHEN CHEN: "Synthesis and Structure–Activity Relationships of Thieno[2,3-d]pyrimidine-2,4-dione Derivatives as Potent GnRH Receptor Antagonists,", BIOORGANIC & MEDICINAL CHEMISTRY LETTERS, vol. 13, no. 20, 20 October 2013 (2013-10-20), pages 3617 - 3622, XP002979732, ISSN: 0960-894X, DOI: 10.1016/S0960-894X(03)00746-7
- SATOSHI SASAKI, NOBUO CHO, YOSHI NARA, MASATAKA HARADA, SATOSHI ENDO, NOBUHIRO SUZUKI, SHUICHI FURUYA, AND MASAHIKO FUJINO,: "Discovery of a Thieno[2,3-d]pyrimidine-2,4-dione Bearing a p-Methoxyureidophenyl Moiety at the 6-Position: A Highly Potent and Orally Bioavailable Non-Peptide Antagonist for the Human Luteinizing Hormone-Releasing Hormone Receptor", JOURNAL OF MEDICINAL CHEMISTRY, vol. 46, no. 1, 27 November 2002 (2002-11-27), pages 113 - 124, XP002967759, ISSN: 0022-2623, DOI: 10.1021/jm020180i

## Description

### CROSS-REFERENCE TO RELATED APPLICATION

This application claims the priority and benefits of Chinese Patent Applications No. 201910686491.2, filed with the State Intellectual Property Office of China on July 29, 2019, and No. 202010290629.X, filed with the State Intellectual Property Office of China on April 14, 2020.

### FIELD OF THE INVENTION

The invention pertains to the pharmaceutical field, and it relates to compounds and pharmaceutical compositions used for preventing or treating sex hormone-dependent diseases, and their usage methods and uses. In particular, the invention relates to substituted pyrimidinedione compounds used as gonadotropin releasing hormone receptor antagonists and uses thereof.

### BACKGROUND OF THE INVENTION

Secretion of anterior pituitary hormones undergoes feedback control by peripheral hormones secreted from target organs of the respective hormones and by secretion-regulating hormones from the hypothalamus, which is the upper central organ of the anterior lobe of the pituitary (hereinafter, these hormones are collectively called "hypothalamic hormones" in this specification). Presently, as hypothalamic hormones, the existence of nine kinds of hormones including, for example, thyrotropin releasing hormone (TRH), and gonadotropin releasing hormone (GnRH, sometimes called as LH-RH (luteinizing hormone releasing hormone)) has been confirmed. These hypothalamic hormones are believed to show their actions via the receptors which are considered to exist in the anterior lobe of the pituitary, and efforts to find the receptorgene expression specific to these hormones, including cases of human, have been made. Therefore, antagonists or agonists that specifically and selectively act on these receptors should control the action of hypothalamic hormones and the secretion of anterior pituitary hormones. As a result, such antagonists or agonists are expected to prevent or treat anterior pituitary hormone dependent diseases.

Known compounds possessing GnRH-antagonizing activity are mostly peptide compounds, such as GnRH-derived linear peptides (linear peptides) (US 5,140,009 and US 5,171,835), a bicyclic peptide derivative (Journal of Medicinal Chemistry, Vol. 36, pp. 3265-3273(1993)), decapeptide modified at 5 or 6 position (WO 9846634 A1) and decapeptide modified at 8 position (EP 0277829 B1), and so forth.

K. Miwa et al., J.Med.Chem. 2011, vol. 54, pages 4998-5012; US 2005/222174, US 2005/250793 and CN1146206 disclose compounds useful as gonadotropine releasing hormone receptor antagonists.

Peptide compounds pose a large number of problems to be resolved with respect to oral absorbability, dosage form, dose volume, drug stability, sustained action, metabolic stability *etc.* There is strong demand for an oral GnRH antagonist, especially one based on a non-peptide compound, that has excellent therapeutic effect on sex hormone-dependent cancers, e.g., prostatic cancer, endometriosis, precocious puberty *etc.,* that does not show transient hypophysialgonadotropic action (acute action) and that has excellent oral absorbability.

### SUMMARY OF THE INVENTION

The following contents merely summarizes certain aspects disclosed herein and is not intended to be limiting in nature. There is a more complete description behind about these and other parts. In the event that one or more of the incorporated literature, patents, and similar materials differs from or contradicts this application, this application controls. The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The invention relates to a novel substituted pyrimidinedione compound, which has unexpected excellent GnRH antagonistic activity and may be used as a gonadotropin releasing hormone receptor antagonist to prevent or treat sex hormone-dependent diseases, including but not limited to prostate cancer, endometriosis, hysteromyoma, precocious puberty, *etc.*

The compounds of the invention have stable properties, good safety, favorable pharmacodynamics and good pharmacokinetic properties, such as good brain/plasma ratio, good bioavailability or good metabolic stability, and so on. Therefore, it has good clinical application potentials.

The invention also provides a method for preparing the compound and a pharmaceutical composition containing the compound.

In one aspect, provided herein is a compound having Formula (I) or a stereoisomer, a geometric isomer, a tautomer, an *N*-oxide, a hydrate, a solvate, or a pharmaceutically acceptable salt thereof, 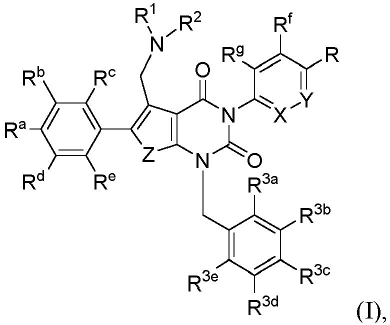 wherein R¹, R², R^{3a}, R^{3b}, R^{3c}, R^{3d}, R^{3e}, R^{a}, R^{b}, R^{c}, R^{d}, R^{e}, R^{f}, R^{g}, R, X, Y and Z are as defined herein.

In some embodiments, X is CR^{x} or N; wherein R^{x} is as defined herein.

In some embodiments, Y is CR^{y} or N; wherein R^{y} is as defined herein.

In some embodiments, Z is NRⁿ, S or O; wherein Rⁿ is as defined herein.

In some embodiments, Rⁿ is H, D, C₁₋₆ alkyl, C₂-C₆ alkenyl, C₂-C₆ alkynyl or C₁-C₆ haloalkyl.

In some embodiments, each R^{b}, R^{c}, R^{d}, R^{e}, R^{f} and R^{g} is independently H, D, F, Cl, Br, I, -CN, -NO₂, -NH₂, -OH, -SH, -COOH, -C(=O)NH₂, -C(=O)NHCH₃, -C(=O)N(CH₃)₂, -C(=O)-(C₁-C₆ alkyl), -C(=O)-(C₁-C₆ alkoxy), C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆-C₁₀ aryl or 5-10 membered heteroaryl.

In some embodiments, R^{x} is H, D, F, Cl, Br, I, -CN, -NO₂, -NH₂, -OH, -SH, -COOH, - C(=O)NH₂, -C(=O)NHCH₃, -C(=O)N(CH₃)₂, -C(=O)-(C₁-C₆ alkyl), -C(=O)-(C₁-C₆ alkoxy), C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆₋₁₀ aryl or 5-10 membered heteroaryl.

In some embodiments, R^{a} is -CN, -NO₂, -OR⁴, -NR⁵R⁶, -NR⁷C(=O)R⁸, - NR⁷C(=O)NR⁵R⁶, -NR⁷S(=O)ₜR⁸, -NR⁷S(=O)ₜNR⁵R⁶, -C(=O)R⁸, -C(=O)NR⁵R⁶, -S(=O)ₜNR⁵R⁶ or -S(=O)ₜR⁸; wherein t is 0, 1 or 2; R⁴, R⁵, R⁶, R⁷ and R⁸ are as defined herein.

In some embodiments, when Y is N, R is 5-10 membered heteroaryl;
when Y is CR^{y}, R is 5-6 membered heteroaryl, wherein R is optionally substituted with 1, 2, 3 or 4 R⁰; R^{y} is H, D, F, Cl, Br, I, -CN, -NO₂, -NH₂, -OH, -SH, -COOH, -C(=O)NH₂, - C(=O)NHCH₃, -C(=O)N(CH₃)₂, -C(=O)-(C₁-C₆ alkyl), -C(=O)-(C₁-C₆ alkoxy), C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆₋₁₀ aryl or 5-10 membered heteroaryl; or
R is H, D, F, Cl, Br, I, -CN, -NO₂, -NH₂, -OH, -SH, -COOH, -C(=O)NH₂, -C(=O)NHCH₃, -C(=O)N(CH₃)₂, -C(=O)-(C₁-C₆ alkyl), -C(=O)-(C₁-C₆ alkoxy), C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆₋₁₀ aryl or 5-10 membered heteroaryl; R^{y} is 5-6 membered heteroaryl, wherein R^{y} is optionally substituted with 1, 2, 3 or 4 R⁰;
wherein R⁰ is as defined herein.

In some embodiments, each R⁰ is independently D, F, Cl, Br, I, -CN, -NO₂, -NH₂, -OH, - SH, -COOH, -C(=O)NH₂, -C(=O)NHCH₃, -C(=O)N(CH₃)₂, -C(=O)-(C₁-C₆ alkyl), -C(=O)-(C₁-C₆ alkoxy), C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino or hydroxy-substituted C₁-C₆ alkyl.

In some embodiments, each R¹ and R² is independently H, D, -OH, C₁-C₆ alkyl, C₂-C₆ alkenyl, C₂-C₆ alkynyl, C₁-C₆ haloalkyl, C₁-C₆ alkoxy, C₁-C₆ haloalkoxy, C₃₋₆ cycloalkyl, 3-8 membered heterocyclyl, C₆₋₁₀ aryl, 5-10 membered heteroaryl, C₃₋₆ cycloalkyl-C₁₋₆ alkylene, (3-8 membered heterocyclyl)-C₁₋₆ alkylene, C₆₋₁₀ aryl-C₁₋₆ alkylene or (5-10 membered heteroaryl)-C₁₋₆ alkylene; wherein each R¹ and R² is independently and optionally substituted with 1, 2, 3 or 4 R^{w}; wherein R^{w} is as defined herein.

In some embodiments, each R^{3a}, R^{3b}, R^{3c}, R^{3d} and R^{3e} is independently H, D, F, Cl, Br, I, -CN, -NO₂, -NH₂, -OH, -SH, -COOH, -C(=O)NH₂, -C(=O)NHCH₃, -C(=O)N(CH₃)₂, -C(=O)-(C₁-C₆ alkyl), -C(=O)-(C₁-C₆ alkoxy), -NHS(=O)₂-C₁-C₆ alkyl, -N(C₁₋₆ alkyl)S(=O)₂-C₁-C₆ alkyl, - S(=O)₂-C₁-C₆ alkyl, C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆₋₁₀ aryl or 5-10 membered heteroaryl.

In some embodiments, R⁴ is H, D, C₁-C₆ alkyl, C₂-C₆ alkenyl, C₂-C₆ alkynyl, C₁-C₆ haloalkyl, C₃₋₆ cycloalkyl, 3-8 membered heterocyclyl, C₆₋₁₀ aryl or 5-10 membered heteroaryl; wherein R⁴ is optionally substituted with 1, 2, 3 or 4 R^{w}; wherein R^{w} is as defined herein.

In some embodiments, each R⁵, R⁶ and R⁷ is independently H, D, C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆₋₁₀ aryl or 5-10 membered heteroaryl; wherein each R⁵, R⁶ and R⁷ is independently and optionally substituted with 1, 2, 3 or 4 R^{w}; wherein R^{w} is as defined herein.

In some embodiments, R⁸ is -OH, C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆₋₁₀ aryl or 5-10 membered heteroaryl; wherein R⁸ is optionally substituted with 1, 2, 3 or 4 R^{w}; wherein R^{w} is as defined herein.

In some embodiments, each R^{w} is independently =O, D, F, Cl, Br, I, -CN, -NO₂, -NH₂, - OH, -SH, -COOH, -C(=O)NH₂, -C(=O)NHCH₃, -C(=O)N(CH₃)₂, -C(=O)-(C₁-C₆ alkyl), -C(=O)-(C₁-C₆ alkoxy), -NHS(=O)₂-(C₁-C₆ alkyl), -N(C₁₋₆ alkyl)S(=O)₂-(C₁-C₆ alkyl), -S(=O)₂-(C₁-C₆ alkyl), C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆₋₁₀ aryl or 5-10 membered heteroaryl.

In some embodiments, each R^{3a}, R^{3b}, R^{3c}, R^{3d} and R^{3e} is independently H, D, F, Cl, Br, I, -CN, -NO₂, -NH₂, -OH, -SH, -COOH, -C(=O)NH₂, -C(=O)NHCH₃, -C(=O)N(CH₃)₂, -C(=O)-(C₁-C₄ alkyl), -C(=O)-(C₁-C₄ alkoxy), -S(=O)₂-(C₁-C₄ alkyl), C₁-C₄ alkyl, C₂-C₄ alkenyl, C₂-C₄ alkynyl, C₁-C₄ haloalkyl, C₁-C₄ alkoxy, C₁-C₄ haloalkoxy, C₁-C₄ alkylthio, C₁-C₄ alkylamino or hydroxy-substituted C₁-C₄ alkyl.

In other embodiments, each R^{3a}, R^{3b}, R^{3c}, R^{3d} and R^{3e} is independently H, D, F, Cl, Br, I, -CN, -NO₂, -NH₂, -OH, -SH, -COOH, -C(=O)NH₂, -C(=O)NHCH₃, -C(=O)N(CH₃)₂, -C(=O)-CH₃, -C(=O)-OCH₃, -S(=O)₂CH₃, methyl, ethyl, n-propyl, i-propyl, t-butyl, vinyl, trifluoromethyl, difluoromethyl, methoxy, trifluoromethoxy, methylamino, dimethylamino or hydroxymethyl.

In some embodiments, the compound provided herein has Formula (II) or a stereoisomer, a geometric isomer, a tautomer, an N-oxide, a hydrate, a solvate, or a pharmaceutically acceptable salt thereof, 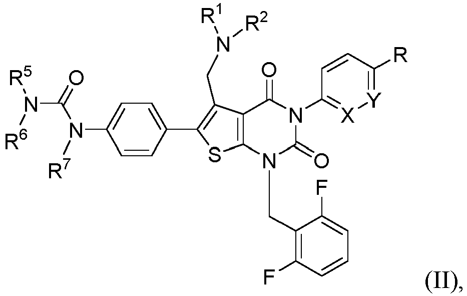 wherein R¹, R², R⁵, R⁶, X⁷, X, Y and R are as defined herein.

In some embodiments, when Y is N, R is pyridyl, pyrimidinyl, thienyl, furyl or 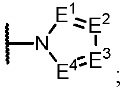 when Y is CR^{y}, R is 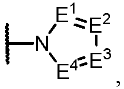 wherein R is optionally substituted with 1, 2, 3 or 4 R⁰; R^{y} is H, D, F, Cl, Br, I, -CN, -NO₂, -NH₂, -OH, -SH, -COOH, -C(=O)NH₂, -C(=O)NHCH₃, - C(=O)N(CH₃)₂, -C(=O)-(C₁-C₄ alkyl), -C(=O)-(C₁-C₄ alkoxy), C₁-C₄ alkyl, C₂-C₄ alkenyl, C₂-C₄ alkynyl, C₁-C₄ haloalkyl, C₁-C₄ alkoxy, C₁-C₄ haloalkoxy, C₁-C₄ alkylthio, C₁-C₄ alkylamino, hydroxy-substituted C₁-C₄ alkyl, C₃₋₆ cycloalkyl, 3-6 membered heterocyclyl, phenyl, naphthyl, pyridyl, pyrimidinyl, thienyl, furyl or 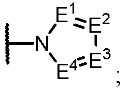 or
R is H, D, F, Cl, Br, I, -CN, -NO₂, -NH₂, -OH, -SH, -COOH, -C(=O)NH₂, -C(=O)NHCH₃, - C(=O)N(CH₃)₂, -C(=O)-(C₁-C₄ alkyl), -C(=O)-(C₁-C₄ alkoxy), C₁-C₄ alkyl, C₂-C₄ alkenyl, C₂-C₄ alkynyl, C₁-C₄ haloalkyl, C₁-C₄ alkoxy, C₁-C₄ haloalkoxy, C₁-C₄ alkylthio, C₁-C₄ alkylamino, hydroxy-substituted C₁-C₄ alkyl, C₃₋₆ cycloalkyl, 3-6 membered heterocyclyl, phenyl, naphthyl, pyridyl, pyrimidinyl, thienyl, furyl or 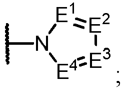 R^{y} is 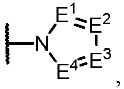 wherein R^{y} is optionally substituted with 1, 2, 3 or 4 R⁰;
wherein each E¹, E², E³ and E⁴ is independently N or CH; wherein R⁰ is as defined herein.

In other embodiments, when Y is N, R is pyridyl, pyrimidinyl, thienyl, furyl, 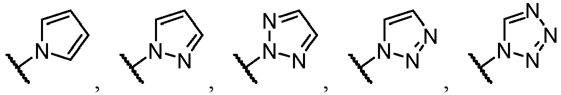 or 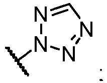
when Y is CR^{y}, R is 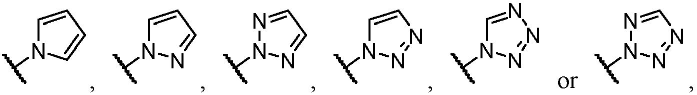 wherein R is optionally substituted with 1, 2, 3 or 4 R⁰; R^{y} is H, D, F, Cl, Br, I, -CN, -NO₂, -NH₂, -OH, -SH, -COOH, -C(=O)NH₂, -C(=O)NHCH₃, -C(=O)N(CH₃)₂, -C(=O)CH₃, -C(=O)OCH₃, methyl, ethyl, n-propyl, i-propyl, t-butyl, vinyl, ethynyl, propynyl, trifluoromethyl, difluoromethyl, methoxy, ethoxy, i-propoxy, trifluoromethoxy, methylthio, methylamino, dimethylamino, hydroxymethyl, C₃₋₆ cycloalkyl, 3-6 membered heterocyclyl, phenyl, naphthyl, pyridyl, pyrimidinyl, thienyl, furyl, 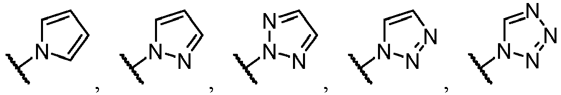 or 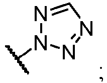 or
R is H, D, F, Cl, Br, I, -CN, -NO₂, -NH₂, -OH, -SH, -COOH, -C(=O)NH₂, -C(=O)NHCH₃, - C(=O)N(CH₃)₂, -C(=O)CH₃, -C(=O)OCH₃, methyl, ethyl, n-propyl, i-propyl, t-butyl, vinyl, ethynyl, propynyl, trifluoromethyl, difluoromethyl, methoxy, ethoxy, i-propoxy, trifluoromethoxy, methylthio, methylamino, dimethylamino, hydroxymethyl, C₃₋₆ cycloalkyl, 3-6 membered heterocyclyl, phenyl, naphthyl, pyridyl, pyrimidinyl, thienyl, furyl, 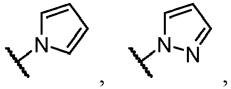 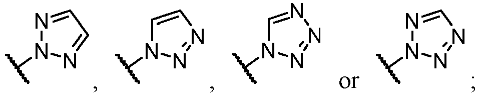 R^{y} is 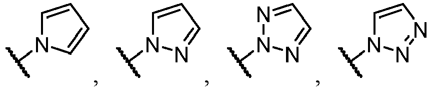 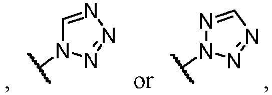 wherein R^{y} is optionally substituted with 1, 2, 3 or 4 R⁰;
wherein R⁰ is as defined herein.

In some embodiments, each R⁰ is independently D, F, Cl, Br, I, -CN, -NO₂, -NH₂, -OH, - SH, -COOH, -C(=O)NH₂, -C(=O)NHCH₃, -C(=O)N(CH₃)₂, -C(=O)-(C₁-C₄ alkyl), -C(=O)-(C₁-C₄ alkoxy), C₁-C₄ alkyl, C₂-C₄ alkenyl, C₂-C₄ alkynyl, C₁-C₄ haloalkyl, C₁-C₄ alkoxy, C₁-C₄ haloalkoxy, C₁-C₄ alkylthio, C₁-C₄ alkylamino or hydroxy-substituted C₁-C₄ alkyl.

In other embodiments, each R⁰ is independently D, F, Cl, Br, I, -CN, -NO₂, -NH₂, -OH, - SH, -COOH, -C(=O)NH₂, -C(=O)NHCH₃, -C(=O)N(CH₃)₂, -C(=O)CH₃, -C(=O)-CH₂CH₃, - C(=O)OCH₃, -C(=O)OCH₂CH₃, methyl, ethyl, n-propyl, i-propyl, t-butyl, vinyl, allyl, propenyl, propynyl, trifluoromethyl, difluoromethyl, methoxy, ethoxy, i-propoxy, trifluoromethoxy, methylamino, dimethylamino or hydroxymethyl.

In some embodiments, each R¹ and R² is independently H, D, -OH, C₁-C₄ alkyl, C₂-C₄ alkenyl, C₂-C₄ alkynyl, C₁-C₄ haloalkyl, C₁-C₄ alkoxy, C₁-C₄ haloalkoxy, C₃₋₆ cycloalkyl, 3-6 membered heterocyclyl, C₆₋₁₀ aryl, 5-6 membered heteroaryl, C₃₋₆ cycloalkyl-C₁₋₄ alkylene, (3-6 membered heterocyclyl)-C₁₋₄ alkylene, C₆₋₁₀ aryl-C₁₋₄ alkylene or (5-6 membered heteroaryl)-C₁₋₄ alkylene; wherein each R¹ and R² is independently and optionally substituted with 1, 2, 3 or 4 R^{w}; wherein R^{w} is as defined herein.

In some embodiments, each R¹ and R² is independently H, D, -OH, methyl, ethyl, n-propyl, i-propyl, n-butyl, t-butyl, 2-methylpropyl, 1-methylpropyl, vinyl, propenyl, allyl, ethynyl, propynyl, trifluoromethyl, difluoromethyl, methoxy, ethoxy, isopropoxy, trifluoromethoxy, cyclopropyl, cyclopentyl, cyclohexyl, oxiranyl, pyrrolidyl, piperidyl, morpholinyl, piperazinyl, phenyl, naphthyl, pyrrolyl, pyridyl, pyrimidinyl, pyrazolyl, triazolyl, thiazolyl, imidazolyl, tetrazolyl, C₃₋₆ cycloalkylmethylene, C₃₋₆ cycloalkylethylene, C₃₋₆ cycloalkylpropylene, (3-6 membered heterocyclyl)methylene, (3-6 membered heterocyclyl)ethylene, phenylmethylene, phenylethylene, phenylpropylene, pyridylmethylene, pyrimidinylmethylene, pyrrolylmethylene, pyrazolylmethylene, triazolylmethylene or tetrazolylmethylene; wherein each R¹ and R² is independently and optionally substituted with 1, 2, 3 or 4 R^{w}; wherein R^{w} is as defined herein.

In some embodiments, each R⁵, R⁶ and R⁷ is independently H, D, C₁-C₄ alkyl, C₂-C₄ alkenyl, C₂-C₄ alkynyl, C₁-C₄ haloalkyl, C₁-C₄ alkoxy, C₁-C₄ haloalkoxy, C₁-C₄ alkylthio, C₁-C₄ alkylamino, hydroxy-substituted C₁-C₄ alkyl, C₃₋₆ cycloalkyl, 3-6 membered heterocyclyl, C₆₋₁₀ aryl or 5-6 membered heteroaryl; wherein each R⁵, R⁶ and R⁷ is independently and optionally substituted with 1, 2, 3 or 4 R^{w}; wherein R^{w} is as defined herein.

In some embodiments, each R⁵, R⁶ and R⁷ is independently H, D, methyl, ethyl, n-propyl, i-propyl, n-butyl, t-butyl, 2-methylpropyl, 1-methylpropyl, vinyl, propenyl, allyl, ethynyl, propynyl, trifluoromethyl, difluoromethyl, 2,2-difluoroethyl, methoxy, ethoxy, i-propoxy, n-propoxy, t-butoxy, trifluoromethoxy, C₁-C₄ alkylthio, C₁-C₄ alkylamino, hydroxy-substituted C₁-C₄ alkyl, C₃₋₆ cycloalkyl, 3-6 membered heterocyclyl, C₆₋₁₀ aryl or 5-6 membered heteroaryl; wherein each R⁵, R⁶ and R⁷ is independently and optionally substituted with 1, 2, 3 or 4 R^{w}; wherein R^{w} is as defined herein.

In other embodiments, each R⁵, R⁶ and R⁷ is independently H, D, methyl, ethyl, n-propyl, i-propyl, n-butyl, t-butyl, 2-methylpropyl, 1-methylpropyl, vinyl, propenyl, allyl, ethynyl, propynyl, trifluoromethyl, difluoromethyl, 2,2-difluoroethyl, methoxy, ethoxy, i-propoxy, n-propoxy, t-butoxy, trifluoromethoxy, C₁-C₄ alkylthio, C₁-C₄ alkylamino, hydroxy-substituted C₁-C₄ alkyl, cyclopropyl, cyclopentyl, cyclohexyl, 3-6 membered heterocyclyl, C₆₋₁₀ aryl or 5-6 membered heteroaryl; wherein each R⁵, R⁶ and R⁷ is independently and optionally substituted with 1, 2, 3 or 4 R^{w}; wherein R^{w} is as defined herein.

In still other embodiments, each R⁵, R⁶ and R⁷ is independently H, D, methyl, ethyl, n-propyl, i-propyl, n-butyl, t-butyl, 2-methylpropyl, 1-methylpropyl, vinyl, propenyl, allyl, ethynyl, propynyl, trifluoromethyl, difluoromethyl, 2,2-difluoroethyl, methoxy, ethoxy, i-propoxy, n-propoxy, t-butoxy, trifluoromethoxy, methylthio, methylamino, dimethylamino, hydroxymethyl, cyclopropyl, cyclopentyl, cyclohexyl, oxiranyl, pyrrolidinyl, piperidinyl, morpholinyl, piperazinyl, phenyl, naphthyl, pyrrolyl, pyrazinyl, pyrimidinyl, pyrazolyl, triazolyl, thiazolyl, imidazolyl or tetrazolyl; wherein each R⁵, R⁶ and R⁷ is independently and optionally substituted with 1, 2, 3 or 4 R^{w}; wherein R^{w} is as defined herein.

In some embodiments, each R^{w} is independently =O, D, F, Cl, Br, I, -CN, -NO₂, -NH₂, - OH, -SH, -COOH, -C(=O)NH₂, -C(=O)NHCH₃, -C(=O)N(CH₃)₂, -C(=O)-(C₁-C₄ alkyl), -C(=O)-(C₁-C₄ alkoxy), -NHS(=O)₂-(C₁-C₄ alkyl), -N(C₁₋₄ alkyl)S(=O)₂-(C₁-C₄ alkyl), -S(=O)₂-(C₁-C₄ alkyl), C₁-C₄ alkyl, C₂-C₄ alkenyl, C₂-C₄ alkynyl, C₁-C₄ haloalkyl, C₁-C₄ alkoxy, C₁-C₄ haloalkoxy, C₁-C₄ alkylthio, C₁-C₄ alkylamino, hydroxy-substituted C₁-C₄ alkyl, C₃₋₆ cycloalkyl, 3-6 membered heterocyclyl, C₆₋₁₀ aryl or 5-6 membered heteroaryl.

In some embodiments, each R^{w} is independently =O, D, F, Cl, Br, I, -CN, -NO₂, -NH₂, - OH, -SH, -COOH, -C(=O)NH₂, -C(=O)NHCH₃, -C(=O)N(CH₃)₂, -C(=O)CH₃, -C(=O)CH₂CH₃, - C(=O)OCH₃, -C(=O)OCH₂CH₃, -NHS(=O)₂CH₃, -N(CH₃)S(=O)₂CH₃, -NHS(=O)₂CH₂CH₃, - N(CH₂CH₃)S(=O)₂CH₂CH₃, -N(CH₂CH₃)S(=O)₂CH₃, -N(CH₃)S(=O)₂CH₂CH₃, -S(=O)₂CH₃, methyl, ethyl, n-propyl, i-propyl, n-butyl, t-butyl, 2-methylpropyl, 1-methylpropyl, vinyl, propenyl, allyl, ethynyl, propynyl, trifluoromethyl, difluoromethyl, methoxy, ethoxy, isopropoxy, n-propoxy, t-butoxy, trifluoromethoxy, C₁-C₄ alkylthio, C₁-C₄ alkylamino, hydroxy-substituted C₁-C₄ alkyl, C₃₋₆ cycloalkyl, 3-6 membered heterocyclyl, C₆₋₁₀ aryl or 5-6 membered heteroaryl.

In some embodiments, the compound disclosed herein has one of the following structures or a stereoisomer, a geometric isomer, a tautomer, an N-oxide, a hydrate, a solvate, or a pharmaceutically acceptable salt thereof:

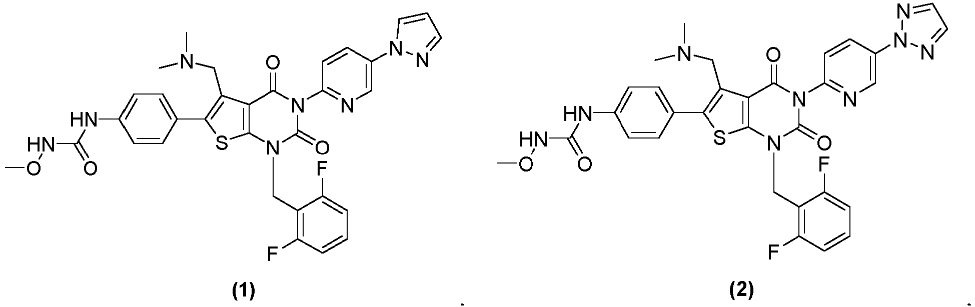

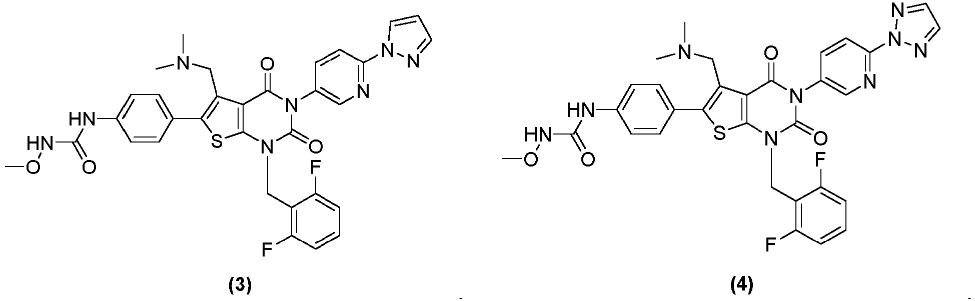

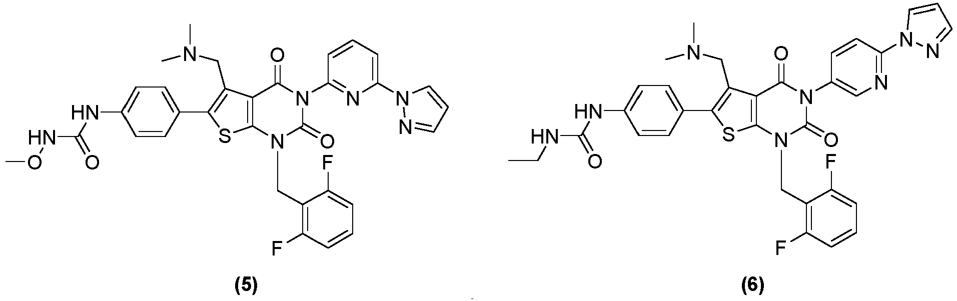

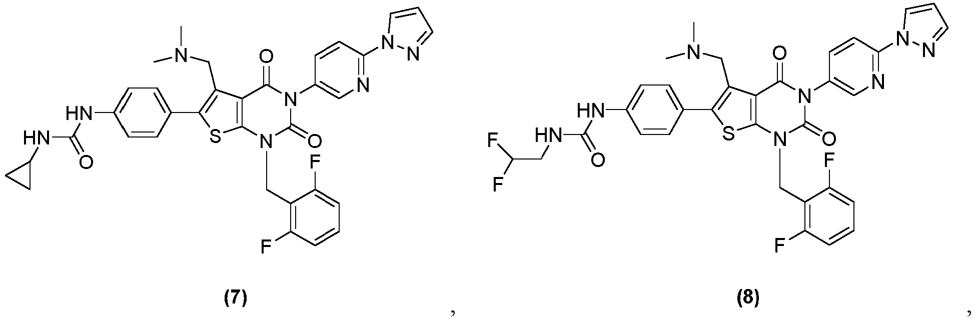

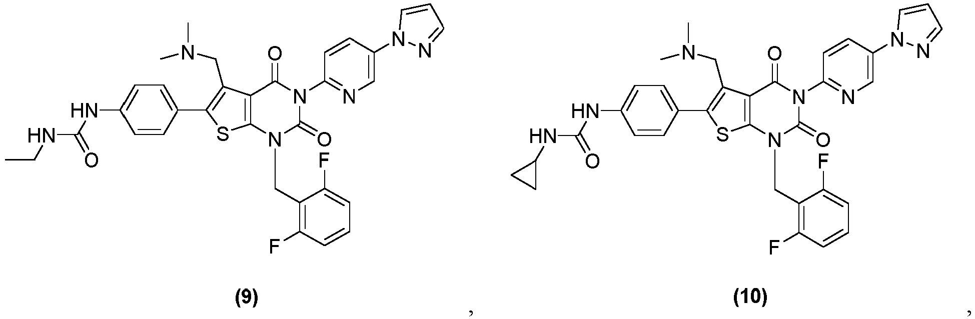

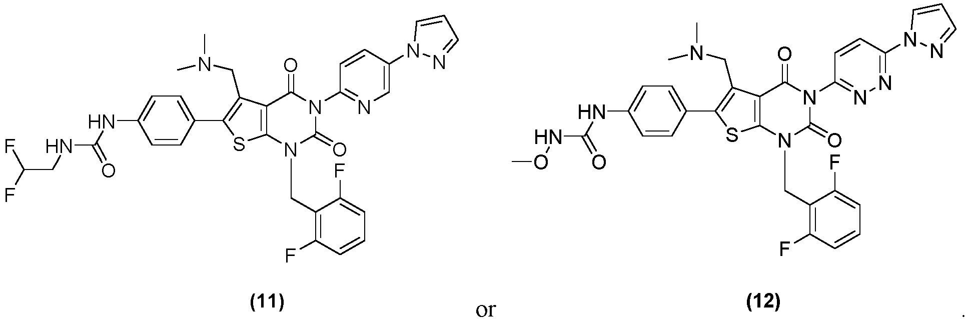

**In** other aspect, provided herein is a pharmaceutical composition comprising the compound disclosed herein.

**In** some embodiments, the pharmaceutical composition disclosed herein further comprises a pharmaceutically acceptable excipient, a carrier, an adjuvant or a combination thereof.

**In** other aspect, the present invention relates to use of the compound or the pharmaceutical composition disclosed herein in the manufacture of a medicament for preventing or treating sex hormone-dependent diseases.

**In** some embodiments, the sex hormone-dependent disease described herein is sex hormone-dependent cancer, osseous metastasis from sex hormone-dependent cancer, prostatic hypertrophy, hysteromyoma, endometriosis, uterine fibroids, precocious puberty, amenorrhea, premenstrual syndrome, algomenorrhea, multilocular ovary syndrome, polycystic ovary syndrome, acne, alopecia, Aizheimer's disease, infertility, irritable bowel syndrome, a benign or malignant tumor or flush independent of hormone and sensitive to LH-RH (luteinizing hormone releasing hormone).

In one aspect, the present invention relates to use of the compound or the pharmaceutical composition disclosed herein in the manufacture of a medicament, the medicament may be used as a reproduction regulator, a contraceptive, an ovulation inducer, or the medicament may be used to prevent postoperative recurrence of sex hormone-dependent cancer.

In other aspect, the present invention relates to use of the compound or the pharmaceutical composition disclosed herein in the manufacture of a medicament for antagonizing gonadotropinreleasing hormone (GnRH).

In other aspect, the present invention relates to a method of preventing or treating a sex hormone-dependent disease in a subject comprising administering to the subject a therapeutically effective amount of the compound or the pharmaceutical composition disclosed herein.

In some embodiments, the sex hormone-dependent disease is sex hormone-dependent cancer, osseous metastasis from sex hormone-dependent cancer, prostatic hypertrophy, hysteromyoma, endometriosis, uterine fibroids, precocious puberty, amenorrhea, premenstrual syndrome, algomenorrhea, multilocular ovary syndrome, polycystic ovary syndrome, acne, alopecia, infertility or irritable bowel syndrome.

In other aspect, the present invention relates to the compound or the pharmaceutical composition disclosed herein for use in preventing or treating a sex hormone-dependent disease in a subject.

In some embodiments, wherein the sex hormone-dependent disease is sex hormone-dependent cancer, osseous metastasis from sex hormone-dependent cancer, prostatic hypertrophy, hysteromyoma, endometriosis, uterine fibroids, precocious puberty, amenorrhea, premenstrual syndrome, algomenorrhea, multilocular ovary syndrome, polycystic ovary syndrome, acne, alopecia, infertility or irritable bowel syndrome.

In other aspect, provided herein is a method of preparing, separating or purifying the compound of Formula (I) or (II).

The biological test results shown that the compound of the invention has strong antagonistic activity against gonadotropin releasing hormone (GnRH), so the compound provided herein may be as a better GnRH antagonist.

Any embodiment disclosed herein can be combined with other embodiments as long as they are not contradictory to one another, even though the embodiments are described under different aspects of the invention. In addition, any technical feature in one embodiment can be applied to the corresponding technical feature in other embodiments as long as they are not contradictory to one another, even though the embodiments are described under different aspects of the invention.

### DETAILED DESCRIPTION OF THE INVENTION DEFINITIONS AND GENERAL TERMINOLOGY

Reference will now be made in detail to certain embodiments of the invention, examples of which are illustrated in the accompanying structures and formulas. One skilled in the art will recognize many methods and materials similar or equivalent to those described herein, which could be used in the practice of the present invention. The present invention is in no way limited to the methods and materials described herein. In the event that one or more of the incorporated literature, patents, and similar materials differs from or contradicts this application, including but not limited to defined terms, term usage, described techniques, or the like, this application controls.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable sub-combination.

As used herein, the following definitions shall apply unless otherwise indicated. For purposes of this invention, the chemical elements are identified in accordance with the Periodic Table of the Elements, CAS version, and the Handbook of Chemistry and Physics, 75th Ed. 1994. Additionally, general principles of organic chemistry are described in "Organic Chemistry", Thomas Sorrell, University Science Books, Sausalito: 1999, and Smith et al., "March's Advanced Organic Chemistry", John Wiley & Sons, New York: 2007, the entire contents of which are hereby incorporated by reference.

The grammatical articles "a", "an" and "the", as used herein, are intended to include "at least one" or "one or more" unless otherwise indicated herein or clearly contradicted by the context. Thus, the articles are used herein to refer to one or more than one (*i.e.* at least one) of the grammatical objects of the article. By way of example, "a component" means one or more components, and thus, possibly, more than one component is contemplated and may be employed or used in an implementation of the described embodiments.

As used herein, "patient" refers to a human (including adults and children) or other animal. In one embodiment, "patient" refers to a human.

"Stereoisomers" refers to compounds which have identical chemical constitution, but differ with regard to the arrangement of the atoms or groups in space. Stereoisomers include enantiomer, diastereoisomers, conformer (rotamer), geometric (cis/trans) isomer, atropisomer, *etc.*

The term "tautomer" or "tautomeric form" refers to structural isomers of different energies which are interconvertible via a low energy barrier. Where tautomerization is possible (*e.g.* in solution), a chemical equilibrium of tautomers can be reached. For example, proton tautomers (also known as prototropic tautomers) include interconversions via migration of a proton, such as keto-enol and imine-enamine isomerizations.

"Pharmaceutically acceptable" as used herein, refers to those compounds, materials, compositions, and/or dosage forms which are, within the scope of sound medical judgment, suitable for use in contact with the tissues of patients without excessive toxicity, irritation, allergic response, or other problem or complication commensurate with a reasonable benefit/risk ratio, and are effective for their intended use.

The terms "optionally substituted with..." and "unsubstituted or substituted with" can be used interchangeably, *i.e.* the structure is unsubstituted or substituted with one or more of the substituents described in the present invention, the substituents disclosed herein include, but are not limited to, D, F, Cl, Br, I, N₃, -OH, -NH₂, -NO₂, -CN, -SH, -COOH, -C(=O)NH₂, - C(=O)NHCH₃, -C(=O)N(CH₃)₂, -C(=O)-alkyl, -C(=O)-alkoxy, -NHS(=O)₂-alkyl, - N(alkyl)S(=O)₂-alkyl, -S(=O)₂-alkyl, alkyl, alkoxy, alkylthio, alkylamino, alkenyl, alkynyl, haloalkyl, haloalkoxy, hydroxy-substituted alkyl, cycloalkyl, heterocyclyl, aryl, heteroaryl, cycloalkyl-alkylene, heterocyclyl-alkylene, aryl-alkylene, heteroaryl-alkylene, and so on.

At various places in the present specification, substituents of compounds disclosed herein are disclosed in groups or in ranges. It is specifically intended that the invention include each and every individual sub-combination of the members of such groups and ranges. For example, the term "C₁-C₆ alkyl" is specifically intended to individually disclose methyl, ethyl, C₃ alkyl, C₄ alkyl, C₅ alkyl, and C₆ alkyl.

At various places in the present specification, linking substituents are described. Where the structure clearly requires a linking group, the Markush variables listed for that group are understood to be linking groups. For example, if the structure requires a linking group and the Markush group definition for that variable lists "alkyl" or "aryl" then it is understood that the "alkyl" or "aryl" represents a linking alkylene group or arylene group, respectively.

The terms "halogen" and "halo" can be used interchangeably, which refer to Fluoro (F), Chloro (Cl), Bromo (Br), or Iodo (I).

The term "alkyl" or "alkyl group" refers to a saturated linear or branched-chain monovalent hydrocarbon group of 1-20 carbon atoms, wherein the alkyl group is optionally substituted with one or more substituents described herein. In some embodiments, the alkyl group contains 1-6 carbon atoms. In other embodiments, the alkyl group contains 1-4 carbon atoms. In still other embodiments, the alkyl group contains 1-3 carbon atoms. Examples of the alkyl group include, but are not limited to, methyl (Me, -CH₃), ethyl (Et, -CH₂CH₃), *n*-propyl (*n*-Pr, *-* CH₂CH₂CH₃), *i*-propyl (*i*-Pr, -CH(CH₃)₂), *n*-butyl (*n*-Bu, -CH₂CH₂CH₂CH₃), *i*-butyl (*i*-Bu, - CH₂CH(CH₃)₂), *s*-butyl (*s*-Bu, -CH(CH₃)CH₂CH₃), *t*-butyl (*t*-Bu, -C(CH₃)₃), and the like.

The term "alkylene" refers to a saturated divalent hydrocarbon group derived from a straight or branched chain saturated hydrocarbon by the removal of two hydrogen atoms. Unless otherwise specified, the alkylene group contains 1-10 carbon atoms. In some embodiments, the alkylene group contains 1-6 carbon atoms. In other embodiments, the alkylene group contains 1-4 carbon atoms. In still other embodiments, the alkylene group contains 1-2 carbon atoms. Some non-limiting examples of the alkylene group include methylene (-CH₂-), ethylene (-CH₂CH₂-), isopropylene (*i*-propylene, -CH(CH₃)CH₂-), and the like. Wherein the alkylene group is optionally substituted with one or more substituents described herein.

The term "alkenyl" refers to linear or branched-chain monovalent hydrocarbon radical of 2 to 12 carbon atoms with at least one site of unsaturation, *i.e.,* a carbon-carbon, sp² double bond, wherein the alkenyl radical may be optionally substituted independently with one or more substituents described herein, and includes radicals having *"cis"* and *"trans"* orientations, or alternatively, "*E*" and "*Z*" orientations. In some embodiments, the alkenyl contains 2 to 8 carbon atoms. In other embodiments, the alkenyl contains 2 to 6 carbon atoms. In still other embodiments, the alkenyl contains 2 to 4 carbon atoms. Some non-limiting examples of the alkenyl group include ethenyl or vinyl (-CH=CH₂), allyl (-CH₂CH=CH₂), 1-propenyl (*i.e.* propenyl, -CH=CH-CH₃), and the like.

The term "alkynyl" refers to a linear or branched monovalent hydrocarbon radical of 2 to 12 carbon atoms with at least one site of unsaturation, *i.e.,* a carbon-carbon, sp triple bond, wherein the alkynyl radical may be optionally substituted independently with one or more substituents described herein. In some embodiments, the alkynyl contains 2 to 8 carbon atoms. In other embodiments, the alkynyl contains 2 to 6 carbon atoms. In still other embodiments, the alkynyl contains 2 to 4 carbon atoms. Examples of such groups include, but are not limited to, ethynyl (-C≡CH), propargyl (-CH₂C≡CH), 1-propynyl (*i.e.* propynyl, -C≡C-CH₃), and the like.

The term "alkoxy" refers to an alkyl group, as previously defined, attached to the parent molecular moiety via an oxygen atom. Unless otherwise specified, the alkoxy group contains 1-12 carbon atoms. In some embodiments, the alkoxy group contains 1-6 carbon atoms. In other embodiments, the alkoxy group contains 1-4 carbon atoms. In still other embodiments, the alkoxy group contains 1-3 carbon atoms. The alkoxy group may be optionally substituted with one or more substituents disclosed herein. Examples of the alkoxy group include, but are not limited to, methoxy (MeO, -OCH₃), ethoxy (EtO, -OCH₂CH₃), 1-propoxy (*n*-PrO, *n*-propoxy, - OCH₂CH₂CH₃), 2-propoxy (*i*-PrO, *i*-propoxy, -OCH(CH₃)₂), 1-butoxy (*n*-BuO, *n*-butoxy, - OCH₂CH₂CH₂CH₃), 2-methyl-1-propoxy (*i*-BuO, *i*-butoxy, -OCH₂CH(CH₃)₂), 2-butoxy (*s*-BuO, *s*-butoxy, -OCH(CH₃)CH₂CH₃), 2-methyl-2-propoxy (*t*-BuO, *t*-butoxy, -OC(CH₃)₃), and the like.

The term "alkylthio" refers to an alkyl group, as previously defined, attached to the parent molecular moiety via a sulfur atom. Unless otherwise specified, the alkylthio group contains 1-12 carbon atoms. In some embodiments, the alkylthio group contains 1-6 carbon atoms. In other embodiments, the alkylthio group contains 1-4 carbon atoms. In still other embodiments, the alkylthio group contains 1-3 carbon atoms. The alkylthio group may be optionally substituted with one or more substituents disclosed herein. Examples of the alkylthio group include, but are not limited to, methylthio (MeS, -SCH₃), ethylthio (EtS, -SCH₂CH₃), and the like.

The term "alkamino" or "alkylamino" refers to an amino group is substituted with one or two alkyl groups, including "*N*-alkylamino" and "*N,N*-dialkylamino", wherein the alkyl group is as defined herein. Suitable alkylamino group may be monoalkylamino or dialkylamino. Examples of such group include, but are not limited to, *N*-methylamino (methylamino), *N*-ethylamino (ethylamino), *N,N*-dimethylamino (dimethylamino), *N,N*-diethylamino (diethylamino), and the like. And wherein the alkylamino radical is optionally substituted with one or more substituents described herein.

The term "hydroxy-substituted alkyl" refers to an alkyl group substituted with one or more hydroxy groups, wherein the alkyl is as defined herein. Examples of such group include, but are not limited to, hydroxymethyl, hydroxyethyl (*e.g*. 2-hydroxyethyl), 2-hydroxy-1-propyl, 3-hydroxy-1-propyl, 2,3-dihydroxypropyl, and the like.

The term "haloalkyl" or "haloalkoxy" refer to an alkyl, or alkoxy, is substituted with one or more halogen atoms, wherein the alkyl and alkoxy groups are as defined herein. Some non-limiting examples of such groups include trifluoromethyl, difluoromethyl, 2,2-difluoroethyl, trifluoromethoxy, and the like. In some embodiments, C₁-C₆ haloalkyl include fluoro substituted C₁-C₆ alkyl; In other embodiments, C₁-C₄ haloalkyl include fluoro substituted C₁-C₄ alkyl; In still other embodiments, C₁-C₂ haloalkyl include fluoro substituted C₁-C₂ alkyl.

The term "cycloalkyl" refers to a saturated monocyclic, bicyclic, or tricyclic ring system having 3 to 12 carbon atoms. In some embodiments, the cycloalkyl contains 3 to 10 carbon atoms, such as C₃₋₁₀ cycloalkyl. In other embodiments, the cycloalkyl contains 3 to 8 carbon atoms, such as C₃₋₈ cycloalkyl. In yet other embodiments, the cycloalkyl contains 3 to 6 carbon atoms, such as C₃₋₆ cycloalkyl. Examples of cycloalkyl groups include, but are not limited to, cyclopropyl, cyclobutyl, cyclopentyl, cyclohexyl, cycloheptyl, cyclooctyl, and the like. Wherein, as described in the present invention, C₃₋₈ cycloalkyl includes C₃₋₆ cycloalkyl; the C₃₋₆ cycloalkyl includes cyclopropyl, cyclobutyl, cyclopentyl and cyclohexyl. Wherein the cycloalkyl group may be optionally substituted with one or more substituents disclosed herein.

The term "heterocyclyl" refers to a saturated or partially unsaturated monocyclic, bicyclic or tricyclic ring system containing 3-12 ring atoms, wherein at least one ring member is selected from nitrogen, sulfur and oxygen; and wherein the heterocyclyl is nonaromatic, and the aromatic ring does not exist in the heterocyclyl system. Unless otherwise specified, the heterocyclyl group may be carbon or nitrogen linked, and a -CH₂- group can be optionally replaced by a -C(=O)-group. The sulfur of the ring can be optionally oxygenized to S-oxide. The nitrogen of the ring can be optionally oxygenized to N-oxide. The term "heterocyclyl" can be used interchangeably with the term "heterocycle". As described in the invention, the heterocyclyl may be consisted of 3-8 atoms or 3-6 atoms, the atom is optionally selected from C, N, O or S and at least one atom is N, O or S; wherein the heterocyclyl consisted of 3-8 atoms includes the heterocyclyl consisted of 3-6 atoms; the heterocyclyl consisted of 3-6 atoms includes the heterocyclyl consisted of 3-5 atoms. Specifically, the heterocyclyl consisted of 3-6 atoms includes, but is not limited to, oxiranyl, aziridinyl, azetidinyl, oxetanyl, pyrrolidyl, tetrahydrofuryl, tetrahydrothienyl, thiazolidinyl, pyrazolidyl, pyrazolinyl, oxazolidinyl, imidazolidinyl, piperidyl, piperazinyl or morpholinyl, *etc.* The heterocyclyl group may be optionally substituted with one or more substituents disclosed herein.

The term "aryl" refers to monocyclic, bicyclic and tricyclic carbocyclic ring systems having a total of six to fourteen ring members, or six to twelve ring members, or six to ten ring members, wherein at least one ring in the system is aromatic, and the aryl group has a single point or multipoint of attachment to the rest of the molecule. The term "aryl" can be used interchangeably with the term "aromatic ring" herein. Some non-limiting examples of the aryl group include phenyl, 2,3-dihydro-1*H*-indenyl, naphthalenyl and anthracenyl, *etc.* The aryl group may be optionally substituted with one or more substituents disclosed herein. Unless otherwise specified, the group "C₆₋₁₀ aryl" refers to an aryl group having 6-10 ring carbon atoms.

The term "heteroaryl" refers to a monocyclic, bicyclic or tricyclic ring system having 5 to 12 ring members, or 5 to 10 ring members, or 5 to 6 ring members, wherein at least one ring in the system is aromatic, and in which at least one ring contains 1, 2, 3 or 4 heteroatoms selected from nitrogen, oxygen and sulfur, and wherein the heteroaryl has a single point or multipoint of attachment to the rest of the molecule. When -CH₂- group exists in heteroaryl, the -CH₂- group can be optionally replaced with -C(=O)-. Unless otherwise specified, the heteroaryl group can attach to the rest of the molecular via any reasonable attachments (such as C or N). The term "heteroaryl" and "heteroaromatic ring" or "heteroaromatic compound" can be used interchangeably herein. The heteroaryl group may be optionally substituted with one or more substituents disclosed herein. In some embodiments, the heteroaryl group is a heteroaryl group consisting of 5-10 atoms, which contains 1-9 ring carbon atoms and 1, 2, 3 or 4 ring heteroatoms selected from O, S and N; in other embodiments, the heteroaryl group is a heteroaryl group consisting of 5-6 atoms, which contains 1-5 ring carbon atoms and 1, 2, 3 or 4 ring heteroatoms selected from O, S and N; some examples of the heteroaryl group consisting of 5-6 atoms include, but are not limited to, furyl, imidazolyl, isoxazolyl, oxazolyl, pyrrolyl, pyrazolyl, pyridyl, pyrimidinyl, pyridazinyl, pyrazinyl, thienyl, thiazolyl, triazolyl, tetrazolyl, and so on.

The term "j-k membered" refers to the ring group consisted of j to k ring atoms, the ring atoms include carbon atom and/or heteroatoms such as O, N, S, P, and so on; the j and k are each independently any non-zero natural number, and k > j; the "j-k" includes j, k and any natural number between them. For example, "3-8 membered", "3-6 membered", "5-10 membered" or "5-6 membered" refers to the ring group consisted of 3-8, 3-6, 5-10 or 5-6 ring atoms, the ring atoms include carbon atom and/or heteroatoms such as O, N, S, P, and so on.

The terms "cycloalkylalkylene", "heterocyclylalkylene", "arylalkylene", "heteroarylalkylene" refer to that the cycloalkyl, heterocyclyl, aryl, heteroaryl are each independently connected to the rest of the molecular via alkylene, wherein the cycloalkyl, heterocyclyl, aryl, heteroaryl and alkylene are as defined herein. For example, some examples of the arylalkylene include, but are not limited to, phenylmethylene, phenylethylene, phenylpropylene, and so on. The cycloalkylalkylene, heterocyclylalkylene, arylalkylene, heteroarylalkylene group are each independently and optionally substituted with one or more substituents described herein.

The term "prodrug" refers to a compound that is transformed in vivo into a compound of Formula (I) or Formula (II). Such a transformation can be affected, for example, by hydrolysis of the prodrug form in blood or enzymatic transformation to the parent form in blood or tissue. Prodrugs of the compounds disclosed herein may be, for example, esters. Some common esters which have been utilized as prodrugs are phenyl esters, aliphatic (C₁₋₂₄) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound.

A "metabolite" is a product produced through metabolism in the body of a specified compound or salt thereof. The metabolites of a compound may be identified using routine techniques known in the art and their activities determined using tests such as those described herein. Such products may result for example from oxidation, reduction, hydrolysis, amidation, deamidation, esterification, deesterification, enzyme cleavage, and the like, of the administered compound.

A "pharmaceutically acceptable salts" refers to organic or inorganic salts of a compound disclosed herein. Pharmaceutically acceptable salts are well known in the art. For example, S. M. Berge et al., describe pharmaceutically acceptable salts in detail in J. Pharmaceutical Sciences, 1977, 66: 1-19, which is incorporated herein by reference. Some non-limiting examples of pharmaceutically acceptable and nontoxic salts include salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid and malonic acid or by using other methods used in the art such as ion exchange. This invention also envisions the quaternization of any basic nitrogen-containing groups of the compounds disclosed herein. Water or oil soluble or dispersable products may be obtained by such quaternization. Further pharmaceutically acceptable salts include, when appropriate, nontoxic ammonium, quaternary ammonium, and amine cations formed using counterions such as halide, hydroxide, carboxylate, sulfate, phosphate, nitrate, C₁-C₈ sulfonate or aryl sulfonate.

The term "solvate" refers to an association or complex of one or more solvent molecules and a compound disclosed herein. Some non-limiting examples of the solvent that form solvates include water, isopropanol, ethanol, methanol, dimethylsulfoxide (DMSO), ethyl acetate, acetic acid, ethanolamine or a combination thereof. The term "hydrate" refers to the complex where the solvent molecule is water.

As used herein, the term "treat", "treating" or "treatment" of any disease or disorder refers in one embodiment, to ameliorating the disease or disorder (*i.e.,* slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (*e.g.,* stabilization of a discernible symptom), physiologically, (*e.g.,* stabilization of a physical parameter), or both. In yet another embodiment, "treat", "treating" or "treatment" refers to preventing or delaying the onset or development or progression of the disease or disorder.

The term "therapeutically effective amount" refers to an amount of the compound when administered to a subject to treat a disease which is sufficient for the treatment of the disease. The "therapeutically effective amount" may vary with the compound, disease and severity, and the condition, age, weight, gender, *etc.* of the subject to be treated.

Unless otherwise stated, all suitable isotopic changes, stereoisomers, tautomers, solvates, or pharmaceutically acceptable salts of the compounds disclosed herein are within the scope of the invention.

All stereoisomers of the structure disclosed herein are considered within the scope of the invention whether the stereochemistry of the structure is indicated or not, and which are interpreted as disclosed compounds of the invention and included in the invention. When the stereochemistry of a structure is indicated by solid wedge or dash line, the stereoisomer of the structure is definite.

The compound of Formula (I) or Formula (II) can be existing in salt forms. In some embodiments, the salt is a pharmaceutically acceptable salt. The phrase "pharmaceutically acceptable" refers to that the substance or composition must be chemically and/or toxicologically compatible with the other ingredients comprising a formulation, and/or the mammal being treated therewith. In other embodiments, the salt may not be a pharmaceutically acceptable salt, may be an intermediate used for preparing and/or purifying the compound of Formula (I) or Formula (II) and/or isolating an enantiomer from the compound of Formula (I) or Formula (II).

Any formula given herein is also intended to represent isotopically unenriched forms as well as isotopically enriched forms of the compounds. The isotope-enriched compound has the structure described by the general formula given in the present invention, except that one or more atoms are replaced by atoms having the selected atomic weight or mass number. Examples of isotopes that can be incorporated into compounds of the invention include isotopes of hydrogen, carbon, nitrogen, oxygen, sulfur, phosphorous, fluorine, and chlorine, such as ²H (deuterium, D), ³H, ¹¹C, ¹³C, ¹⁴C, ¹⁵N, ¹⁷O, ¹⁸O, ¹⁸F, ³¹P, ³²P, ³⁵S, ³⁶Cl, ¹²⁵I, respectively.

In other aspect, provided herein is a preparation of intermediate of the compound of Formula (I) or Formula (II).

### PHARMACEUTICAL COMPOSITION OF THE COMPOUND OF THE INVENTION AND PREPARATIONS AND ADMINISTRATION

The invention provides a pharmaceutical composition containing a therapeutic effective amount of the compound of Formula (I) or Formula (II) or an independent stereoisomer thereof, a racemic mixture or non-racemic mixture of the stereoisomer thereof, or a pharmaceutically acceptable salt or solvent thereof. In some embodiments of the invention, the pharmaceutical composition further comprises at least one pharmaceutically acceptable carrier, adjuvant or excipient, and optionally other treating and/or preventing ingredients.

A suitable carrier, adjuvant or excipient is well known for the technical personnel in the field and was described in detail in Ansel H. C. et al., Ansel's Pharmaceutical Dosage Forms and Drug Delivery Systems (2004) Lippincott, Williams & Wilkins, Philadelphia; Gennaro A. R. et al., Remington: The Science and Practice of Pharmacy (2000) Lippincott, Williams & Wilkins, Philadelphia; and Rowe R. C., Handbook of Pharmaceutical Excipients (2005) Pharmaceutical Press, Chicago.

Suitable pharmaceutically acceptable excipients will vary depending upon the particular dosage form chosen. In addition, suitable pharmaceutically acceptable excipients may be chosen for a particular function that they may serve in the composition. Suitable pharmaceutically acceptable excipients include the following types of excipients: diluents, fillers, binders, disintegrants, lubricants, glidants, granulating agents, coating agents, wetting agents, solvents, cosolvents, suspending agents, emulsifiers, sweeteners, flavoring agents, flavor masking agents, coloring agents, anticaking agents, humectants, chelating agents, plasticizers, viscosity increasing agents, antioxidants, preservatives, stabilizers, surfactants, and buffering agents. The skilled artisan will appreciate that certain pharmaceutically acceptable excipients may serve more than one function and may serve alternative functions depending on how much of the excipient is present in the formulation and what other ingredients are present in the formulation.

The pharmaceutical compositions of the invention are prepared using techniques and methods known to those skilled in the art. Some of the methods commonly used in the art are described in Remington's Pharmaceutical Sciences (Mack Publishing Company).

Therefore, another aspect of the present invention is related to a method for preparing a pharmaceutical composition, the pharmaceutical composition contains the compound disclosed herein and pharmaceutically acceptable excipient, carrier, adjuvant, vehicle or a combination thereof, the method comprises mixing various ingredients. The pharmaceutical composition containing the compound disclosed herein can be prepared at for example environment temperature and under barometric pressure.

The compound of the invention will typically be formulated into a dosage form adapted for administration to the patient by the desired route of administration. For example, dosage forms include those adapted for (1) oral administration such as tablets, capsules, caplets, pills, troches, powders, syrups, elixirs, suspensions, solutions, emulsions, sachets, and cachets; (2) parenteral administration such as sterile solutions, suspensions, and powders for reconstitution; (3) transdermal administration such as transdermal patches; (4) rectal administration such as suppositories; (5) inhalation such as aerosols, solutions, and dry powders; and (6) topical administration such as creams, ointments, lotions, solutions, pastes, sprays, foams, and gels.

In some embodiments, the compounds disclosed herein can be prepared to oral. In other embodiments, the compounds disclosed herein can be prepared to inhalation. In the still other embodiments, the compounds disclosed herein can be prepared to nasal administration. In the yet other embodiments, the compounds disclosed herein can be prepared to transdermal administration. In the still yet other embodiments, the compounds disclosed herein can be prepared to topical administration.

The pharmaceutical compositions provided herein may be provided as compressed tablets, tablet triturates, chewable lozenges, rapidly dissolving tablets, multiple compressed tablets, enteric-coating tablets, sugar-coated, or film-coated tablets.

The pharmaceutical compositions provided herein may be provided as soft or hard capsules, which can be made from gelatin, methylcellulose, starch, or calcium alginate.

The pharmaceutical compositions provided herein may be administered parenterally by injection, infusion, or implantation, for local or systemic administration. Parenteral administration, as used herein, include intravenous, intraarterial, intraperitoneal, intrathecal, intraventricular, intraurethral, intrasternal, intracranial, intramuscular, intrasynovial, and subcutaneous administration.

The pharmaceutical compositions provided herein may be formulated in any dosage forms that are suitable for parenteral administration, including solutions, suspensions, emulsions, micelles, liposomes, microspheres, nanosystems, and solid forms suitable for solutions or suspensions in liquid prior to injection. Such dosage forms can be prepared according to conventional methods known to those skilled in the art of pharmaceutical science (*see,* Remington: The Science and Practice of Pharmacy, supra).

In another aspect, the pharmaceutical composition of the invention is prepared to a dosage form adapted for administration to a patient by inhalation, for example as a dry powder, an aerosol, a suspension, or a solution composition.

Pharmaceutical compositions adapted for transdermal administration may be presented as discrete patches intended to remain in intimate contact with the epidermis of the patient for a prolonged period of time. For example, the active ingredient may be delivered from the patch by iontophoresis as generally described in Pharmaceutical Research, 3(6), 318(1986).

Pharmaceutical compositions adapted for topical administration may be formulated as ointments, creams, suspensions, lotions, powders, solutions, pastes, gels, sprays, aerosols or oils.

### USE OF THE COMPOUNDS AND PHARMACEUTICAL COMPOSITIONS

The compounds and pharmaceutical compositions provided herein have excellent GnRH antagonizing activity and low toxicity (for example, acute toxicity, chronic toxicity, genetic toxicity, reproduction toxicity, cardiotoxicity, drug interaction, and carcinogenicity). And also, the compound or pharmaceutical composition is excellent in oral absorbability, action sustainability, stability and pharmacokinetics. In addition, the compound or pharmaceutical composition is scarcely affected by influenced by plasma ingredients. The compound or pharmaceutical composition of the present invention can therefore be safely used in a mammal (*e.g.,* human, monkey, bovine, horse, dog, cat, rabbit, rat, mouse, *etc.*) for the preventing and/or treating diseases depending on male or female hormones, diseases due to excess of these hormones, *etc.,* by suppressing gonadotropin secretion with its GnRH receptor-antagonizing action to control plasma sex hormone concentrations.

In particular, the compound or pharmaceutical composition of the present invention is useful for preventing and/or treating sex hormone-dependent cancers (*e.g*., prostatic cancer, uterine cancer, breast cancer, pituitary tumor, *etc*.)*,* bone metastasis of sex hormone-dependent cancer, prostatic hypertrophy, hysteromyoma, endometriosis, metrofibroma, precocious puberty, amenorrhea, premenstrual syndrome, dysmenorrhea, multilocular ovary syndrome, polycystic ovary syndrome, acne, alopecia, Alzheimer's disease (Alzheimer's disease, senile dementia of Alzheimer type and a mixed type thereof), and the like. The compound of the present invention is also useful for the regulation of reproduction in males and females (*e.g*., pregnancy regulators, menstruation cycle regulators, *etc*.)*.* The compound or pharmaceutical composition of the present invention can be also used as a male or female contraceptive, or as a female ovulation inducer. Based on its rebound effect after withdrawal, the compound of the present invention can be used to treat infertility. And the compound or pharmaceutical composition of this invention can be used as an agent for preventing and/or treating benign or malignant tumor which is hormone independent and LH-RH sensitive. Moreover, the compound or pharmaceutical composition of the present invention can be used as an agent for preventing and/or treating irritable bowel syndrome and for preventing postoperative recurrence of sex hormone-dependent cancer (an agent for preventing postoperative recurrence of prostatic cancer; an agent for preventing postoperative recurrence of breast cancer or ovarian cancer in the condition before or after menopause; especially, an agent for preventing postoperative recurrence of breast cancer or ovarian cancer in the condition before menopause).

In addition, the compound or pharmaceutical composition of the present invention is useful for regulation of animal estrus, improvement of meat quality and promotion of animal growth in the field of animal husbandry. The compound of the present invention is also useful as a fish spawning promoter.

The compound or pharmaceutical composition of the present invention can be also used to suppress the transient rise in plasma testosterone concentration (flare phenomenon) observed in administration of a GnRH super-agonist such as leuprorelin acetate. The compound of the present invention can be used in combination with a GnRH super-agonist such as leuprorelin acetate, gonadorelin, buserelin, triptorelin, goserelin, nafarelin, histrelin, deslorelin, meterelin, lecirelin, and the like. Among others, preferred is leuprorelin acetate.

It is also beneficial to use the compound or pharmaceutical composition of the present invention in conjunction with at least one member selected from the steroidal or nonsteroidal antiandrogen agent or antiestrogen agent, chemotherapeutic agent, GnRH antagonistic peptide, α-reductase inhibitor, α-receptor inhibitor, aromatase inhibitor, 17β-hydroxysteroid dehydrogenase inhibitor, adrenal androgen production inhibitor, kinase inhibitor, drug for hormone therapy, and drug inhibiting cell growth factor or its receptor, among others.

The "chemotherapeutic agent" mentioned above includes ifosfamide, adriamycin, peplomycin, cisplatin, cyclophosphamide, 5-FU, UFT, methotrexate, mitomycin C, mitoxantrone, *etc.* The "GnRH antagonistic peptide" mentioned above includes non-oral GnRH antagonistic peptides such as cetrorelix, ganirelix, abarelix, *etc.*

Besides being useful for human treatment, these compounds are also useful for veterinary treatment of animals such as companion animals, exotic animals and farm animals. In other embodiments, the animals disclosed herein include horses, dogs, and cats.

In some embodiments, the compound of the invention or the pharmaceutical composition thereof may be administered once or according to a dosing regimen wherein a number of doses are administered at varying intervals of time for a given period of time. The compounds of the present invention may be administered either simultaneously with, or before or after, one or more other therapeutic agents. The compounds of the present invention may be administered separately, by the same or different route of administration, or together in the same pharmaceutical composition as the other agents.

### GENERAL SYNTHETIC PROCEDURES

The following examples are provided so that the invention might be more fully understood. However, it should be understood that these embodiments merely provide a method of practicing the present invention, and the present invention is not limited to these embodiments.

Generally, the compounds disclosed herein may be prepared by methods described herein, wherein the substituents are as defined for Formula (I) or Formula (II) above, except where further noted. The following non-limiting schemes and examples are presented to further exemplify the invention.

Persons skilled in the art will recognize that the chemical reactions described may be readily adapted to prepare a number of other compounds disclosed herein, and alternative methods for preparing the compounds disclosed herein are deemed to be within the scope disclosed herein. For example, the synthesis of non-exemplified compounds according to the invention may be successfully performed by modifications apparent to those skilled in the art, *e.g.,* by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds disclosed herein.

In the examples described below, unless otherwise indicated all temperatures are set forth in degrees Celsius. Reagents were purchased from commercial suppliers such as Aldrich Chemical Company, Arco Chemical Company and Alfa Chemical Company, and were used without further purification unless otherwise indicated. Common solvents were purchased from commercial suppliers such as Shantou Xi Long Chemical Factory, Guangdong Guanghua Reagent Chemical Factory Co. Ltd., Guangzhou Reagent Chemical Factory, Tianjin YuYu Fine Chemical Ltd., Tianjin Fuchen Chemical Reagent Factory, Wuhan XinHuaYuanm Technology Development Co. Ltd., Qingdao Tenglong Reagent Chemical Ltd., and Qingdao Ocean Chemical Factory.

Anhydrous THF, dioxane, toluene, and ether were obtained by refluxing the solvent with sodium. Anhydrous CH₂Cl₂ and CHCl₃ were obtained by refluxing the solvent with CaH₂. EtOAc, PE, hexane, DMAC and DMF were treated with anhydrous sodium sulfate prior to use.

The reactions set forth below were done generally under a positive pressure of nitrogen or argon or with a drying tube (unless otherwise stated) in anhydrous solvents, and the reaction flasks were typically fitted with rubber septa for the introduction of substrates and reagents via syringe. Glassware was oven dried and/or heat dried.

Column chromatography was conducted using a silica gel column. Silica gel (300-400 mesh) was purchased from Qingdao Ocean Chemical Factory.

¹H NMR spectra were recorded by Bruker 400 MHz or 600 MHz NMR spectrometer. ¹H NMR spectra were obtained by using CDCl₃, DMSO-*d₆*, CD₃OD or acetone-*d₆* solutions (in ppm), with TMS (0 ppm) or chloroform (7.26 ppm) as the reference standard. When peak multiplicities are reported, the following abbreviations are used: s (singlet), d (doublet), t (triplet), q (quartet), m (multiplet), br (broadened), brs (broadened singlet), dd (doublet of doublets), dt (doublet of triplets), tt (triplet of triplets). Coupling constants *J*, when given, were reported in Hertz (Hz).

Low resolution mass spectrum (MS) data measurement condition: Agilent 6120 Quadrupole HPLC-M (column type: Zorbax SB-C18, 2.1 × 30 mm, 3.5 micron, 6 min, flow rate 0.6 mL / min. Mobile phase: in the proportion of 5% - 95% (CH₃CN containing 0.1% of formic acid) in (H₂O containing 0.1% of formic acid), using electrospray ionization (ESI), UV detection, at 210 nm / 254 nm.

Pure compound was detected by Agilent 1260 pre-HPLC or Calesep pump 250 pre-HPLC (NOVASEP 50/80 mm DAC) with UV detection at 210/254 nm.

The following abbreviations are used throughout the specification:

| | | | |
|---|---|---|---|
| Py | pyridine | h | hour(s) |
| CDCl₃ | deuterated chloroform | mmol | millimolar |
| DMSO | dimethylsulfoxide | nM, nmol/L | nanomoles per liter |
| DMSO-*d₆* | deuterated dimethylsulfoxide | M, mol/L, | moles per liter |
| AIBN | azobisisobutyronitrile | mg | milligram |
| NBS | N-bromosuccinimide | g | gram |
| EA | ethyl acetate | kg | kilogram |
| Saline | physiological saline | mL, ml | milliliter |
| min | minute(s) | Pd/C | palladium on carbon |

The following synthetic schemes describe the steps for preparing the compounds disclosed herein, unless otherwise specified, wherein, R^{j} is C₁-C₆ alkyl, C₁-C₆ haloalkyl, C₁-C₆ alkoxy or C₃₋₆ cycloalkyl, each R¹, R², X, Y and E¹ is as defined herein.

### Scheme 1

A compound of Formula (15') can be prepared by the following procedures:

A compound of Formula (1) can react with ethyl cyanoacetate of formula (2) to get a compound of Formula (3); and then the compound of Formula (3) can undergo a closing reaction in the presence of sulfur powder to give a compound of Formula (4). The compound of Formula (4) can react with phenyl chloroformate in the presence of pyridine to get a compound of Formula (5). The compound of Formula (5) can react with a compound of Formula (6) to get a compound of Formula (7); and then the compound of Formula (7) can undergo a closing reaction in the presence of sodium alcoholate, such as sodium methoxide, to give a compound of Formula (8). The compound of Formula (8) can react with a compound of Formula (9) to get a compound of Formula (10). The compound of Formula (10) can be brominated to get a compound of Formula (11); the compound of Formula (11) can react with a substituted amine of Formula (11a) to get a compound of Formula (12); the nitro group of the compound of Formula (12) can be reduced to get a compound of Formula (13); the compound of Formula (13) can react with a compound of Formula (14') to get a compound of Formula (15'). The reaction process is as follows:

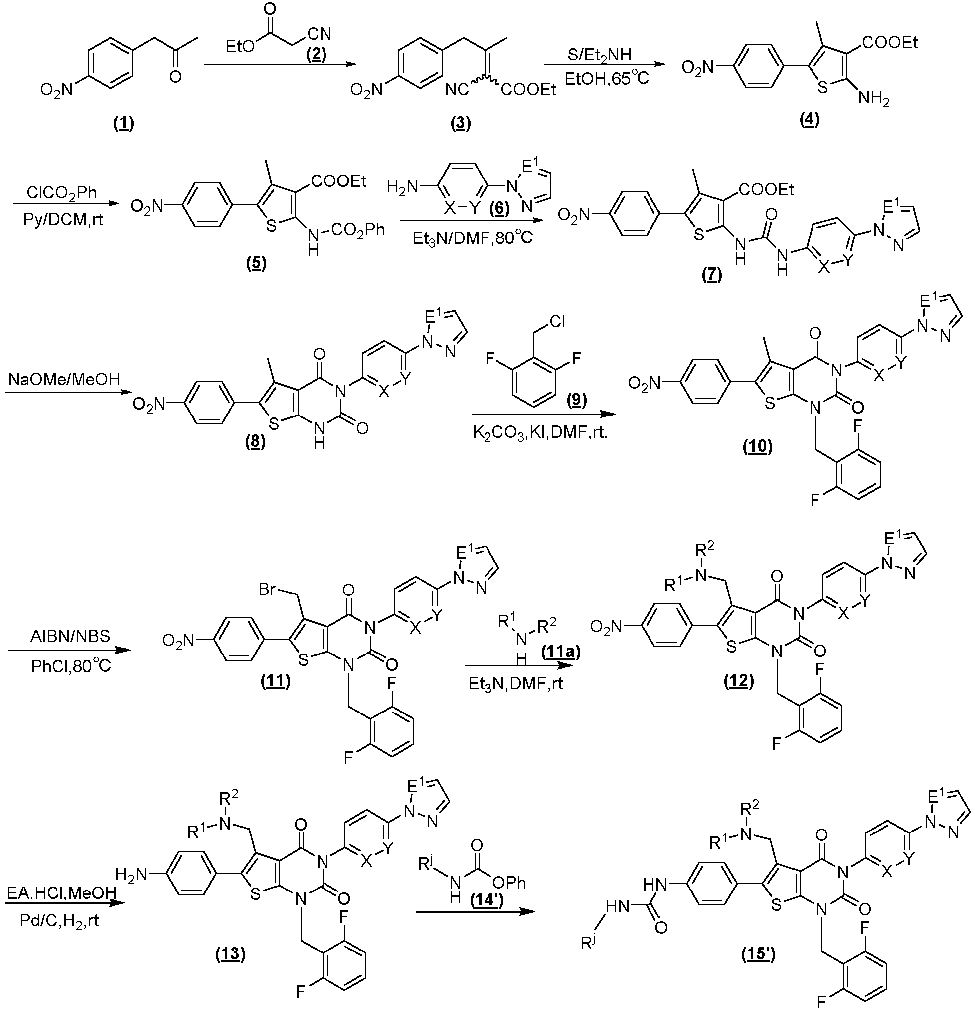

The following examples are provided to further illustrate the compounds, pharmaceutical compositions and their applications thereof.

### EXAMPLES

### Example 1 1-(4-(3-(5-(1H-pyrazol-1-yl)pyrid-2-yl)-1-(2,6-difluorobenzyl)-5-((dimethyl amino)methyl)-2,4-dioxo-1,2,3,4-tetrahydrothieno[2,3-d]pyrimidin-6-yl)phenyl)-3-methoxyurea

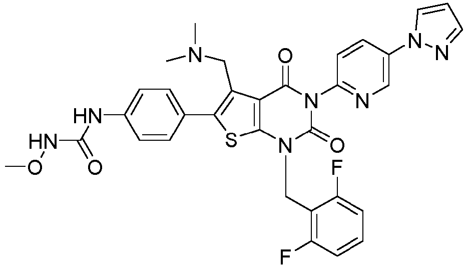

### Step 1) Synthesis of ethyl (E/Z)-2-cyano-3-methyl-4-(4-nitrophenyl)but-2-enoate

To a 250 mL one-neck round bottom flask were added 4-nitrophenylacetone (5.3 g, 29.6 mmol) and ethyl cyanoacetate (9.64 mL, 88.8 mmol), and then heptanoic acid (4.37 mL, 29.6 mmol), benzylamine (3.6 mL, 32 mmol) and methylbenzene (50 mL) were added, and the flask was equipped with a water separator, the mixture was stirred at 130 °C for 12 hours; after the reaction was completed, the mixture was concentrated *in vacuo,* the residue was purified by silica gel column chromatography eluted with (petroleum ether / ethyl acetate (v/v) = 18/1) to give the title compound as a light yellow oil (7.24 g, 89.2%).
MS (ESI, neg. ion) *m*/*z:* 273.3 [M-H]⁻.

### Step 2) Synthesis of ethyl 2-amino-4-methyl-5-(4-nitrophenyl)thiophene-3-carboxylate

To a 250 mL one-neck round bottom flask were added ethyl (*E*/*Z*)-2-cyano-3-methyl-4-(4-nitrophenyl)but-2-enoate (7.2 g, 26.2 mmol), sulfur powder (1.68 g, 52.4 mmol) and ethanol (80 mL), and diethylamine (6.8 mL, 65 mmol) was added, the mixture was stirred for 15 min, and the flask was transferred to an oil bath at 65 °C, the reaction was continued for 3 hours; the reaction was terminated, the mixture was concentrated *in vacuo,* the residue was purified by silica gel column chromatography eluted with (petroleum ether / dichloromethane (v/v) = 5/1) to give the title compound as a brown-red solid (5.23 g, 65.1%).
MS (ESI, pos. ion) *m*/*z:* 307.1 [M+H]⁺;
¹H NMR (600 MHz, CDCl₃) *δ* (ppm) 8.22 (d, *J* = 8.9 Hz, 2H), 7.48 (d, *J* = 8.9 Hz, 2H), 6.27 (s, 2H), 4.33 (q, *J* = 7.1 Hz, 2H), 2.40 (s, 3H), 1.38 (t, *J* = 7.1 Hz, 3H).

### Step 3) Synthesis of ethyl 4-methyl-5-(4-nitrophenyl)-2-((phenoxycarbonyl)amino)thiophene -3-carboxylate

At 25 °C, to a 250 mL one-neck round bottom flask were added ethyl 2-amino-4-methyl-5-(4-nitrophenyl)thiophene-3-carboxylate (10.5 g, 34.3 mmol), pyridine (9.7 mL, 120 mmol) and dichloromethane (100 mL), and then phenyl chloroformate (5.2 mL, 41 mmol) was added, the mixture was further stirred for 2 hours; the reaction was terminated, and water (50 mL) added, the mixture was separated into two layers, the organic layer was dried over anhydrous sodium sulfate (1.5 g), and filtered; the filtrate was concentrated *in vacuo,* the residue was purified by silica gel column chromatography eluted with (petroleum ether / ethyl acetate (v/v) = 30/1) to give the title compound as a yellow solid (12.25 g, 83.8%).
MS (ESI, pos. ion) *m*/*z:* 427.1 [M+H]⁺;
¹H NMR (600 MHz, CDCl₃) *δ* (ppm) 11.04 (s, 1H), 8.27 (d, *J* = 8.8 Hz, 2H), 7.55 (d, *J =* 8.8 Hz, 2H), 7.41 (d, *J* = 8.3 Hz, 2H), 7.29 - 7.27 (m, 1H), 7.23 (d, *J* = 8.6 Hz, 2H), 4.43 (q, *J* = 7.1 Hz, 2H), 2.44 (s, 3H), 1.45 (t, *J* = 7.1 Hz, 3H).

### Step 4) Synthesis of ethyl 2-(3-(5-(1H-pyrazol-1-yl)pyrid-2-yl)ureido)-4-methyl-5-(4-nitro phenyl)thiophene-3-carboxylate

To a 100 mL one-neck round bottom flask were added ethyl 4-methyl-5-(4-nitrophenyl)-2-((phenoxycarbonyl)amino)thiophene-3-carboxylate (2.5 g, 5.86 mmol) and tetrahydrofuran (20 mL), and then triethylamine (2.5 mL, 18 mmol) and 5-pyrazolylpyridin-2-amine (1.2 g, 7.5 mmol) were added, the mixture was stirred in an oil bath at 70 °C for 10 hours; the reaction was terminated, the mixture was filtered, the filter cake was washed with tetrahydrofuran (10 mL × 2) and dried to give the title compound as a light yellow solid (1.78 g, 61.6%).
MS (ESI, pos. ion) *m*/*z:* 493.1 [M+H]⁺.

### Step 5) Synthesis of 3-(5-(1H-pyrazol-1-yl)pyrid-2-yl)-5-methyl-6-(4-nitrophenyl)thieno[2,3-d] pyrimidine-2,4(1H,3H)-dione

To a 100 mL one-neck round bottom flask were added ethyl 2-(3-(5-(1*H*-pyrazol-1-yl)pyrid-2-yl)ureido)-4-methyl-5-(4-nitrophenyl)thiophene-3-carboxylate (1.7 g, 3.45 mmol) and ethanol (20 mL), and then sodium methoxide (0.37 g, 6.9 mmol) was added, the mixture was stirred in an oil bath at 80 °C for 5 hours; the reaction was terminated, the mixture was concentrated *in vacuo,* and water (20 mL) was added, the resulting mixture was stirred for 10 min and filtered, the filter cake was dried to give the title compound as a brick red solid (1.4 g, 90.9%).
MS (ESI, pos. ion) *m*/*z:* 447.0 [M+H]⁺.

### Step 6) Synthesis of 3-(5-(1H-pyrazol-1-yl)pyrid-2-yl)-1-(2,6-difluorobenzyl)-5-methyl-6- (4-nitrophenyl)thieno[2,3-d]pyrimidine-2,4(1H,3H)-dione

At 25 °C, to a 100 mL one-neck round bottom flask were added 3-(5-(1*H*-pyrazol-1-yl)pyrid-2-yl)-5-methyl-6-(4-nitrophenyl)thieno[2,3-*d*]pyrimidine-2,4(1*H*,3*H*)-dione (1.4 g, 3.14 mmol), potassium iodide (1.04 g, 6.27 mmol), potassium carbonate (0.876 g, 6.28 mmol) and*N,N-*dimethylformamide (20 mL), and then 2,6-difluorobenzyl chloride (1.02 g, 6.27 mmol) was added, the mixture was further stirred for 14 hours; the reaction was terminated by quenching with water (150 mL), and then the resulting mixture was extracted with ethyl acetate (40 mL × 2). The combined organic layers were dried over anhydrous sodium sulfate (2 g) and filtered, the filtrate was concentrated *in vacuo* and the residue was purified by silica gel chromatography eluted with (petroleum ether / ethyl acetate (v/v) = 2/1) to give the title compound as a yellow solid (1.6 g, 89.1%).
MS (ESI, pos. ion) *m*/*z:* 573.1 [M+H]⁺;
¹H NMR (400 MHz, DMSO-*d₆*) *δ* (ppm) 9.11 (d, *J =* 2.6 Hz, 1H), 8.67 (d, *J* = 2.4 Hz, 1H), 8.45 (dd, *J =* 8.6, 2.6 Hz, 1H), 8.33 (d, *J =* 8.7 Hz, 2H), 7.88 (s, 1H), 7.76 (d, *J =* 8.7 Hz, 2H), 7.63 (d, *J* = 8.6 Hz, 1H), 7.52 - 7.41 (m, 1H), 7.15 (t, *J =* 8.2 Hz, 2H), 6.66 (s, 1H), 5.31 (s, 2H), 2.56 (s, 3H).

### Step 7) Synthesis of 3-(5-(1H-pyrazol-1-yl)pyrid-2-yl)-5-(bromomethyl)-1-(2,6-difluorobenzyl)-6-(4-nitrophenyl)thieno[2,3-d]pyirimidine-2,4(1H,3H)-dione

To a 100 mL one-neck round bottom flask were added 3-(5-(1*H*-pyrazol-1-yl)pyrid-2-yl)-1-(2,6-difluorobenzyl)-5-methyl-6-(4-nitrophenyl)thieno[2,3-*d*]pyrimidine-2,4(1*H*,3*H*)-dione (200 mg, 0.35 mmol), azobisisobutyronitrile (0.030 g, 0.18 mmol), *N*-bromosuccinimide (0.127 g 0.70 mmol) and chlorobenzene (10 mL), the mixture was stirred in an oil bath at 80 °C under N₂ for 10 hours; the reaction was terminated, and saturated sodium bicarbonate aqueous solution (20 mL) was added, the mixture was separated into two layers, the organic layer was dried over anhydrous sodium sulfate (1 g) and filtered; the filtrate was concentrated *in vacuo* to give the title compound as a light yellow solid (0.21 g, 92.3%).

### Step 8) Synthesis of 3-(5-(1H-pyrazol-1-yl)pyrid-2-yl)-1-(2,6-difluorobenzyl)-5-((dimethyl amino)methyl)-6-(4-nitrophenyl)thieno[2,3-d]pyrimidine-2,4(1H,3H)-dione

At 25 °C, to a 100 mL one-neck round bottom flask were added 3-(5-(1*H*-pyrazol-1-yl)pyrid-2-yl)-5-(bromomethyl)-1-(2,6-difluorobenzyl)-6-(4-nitrophenyl)thieno[2,3-*d*]pyrimidine-2,4(1*H*,3*H*)-dione (210 mg, 0.32 mmol), dimethylamine hydrochloride (0.159 g, 1.93 mmol) and *N,N*-dimethylformamide (10 mL), and then triethylamine (0.18 mL, 1.3 mmol) was added, the mixture was further stirred for 16 hours; the reaction was terminated, and water (20 mL) was added, a light yellow solid precipitated, the mixture was filtered, the filter cake was purified by silica gel chromatography eluted with (dichloromethane / methanol (v/v) = 30/1) to give the title compound as a light yellow solid (0.14 g, 70.8%).
MS (ESI, pos. ion) *m*/*z:* 616.4 [M+H]⁺;
¹H NMR (400 MHz, CDCl₃) *δ* (ppm) 9.03 (d, *J* = 2.6 Hz, 1H), 8.33 - 8.22 (m, 3H), 8.00 (d, *J =* 2.4 Hz, 1H), 7.89 *(d, J* = 8.7 Hz, 2H), 7.80 (s, 1H), 7.48 (d, *J =* 8.5 Hz, 1H), 7.36 - 7.32 (m, 1H), 6.95 (t, *J =* 9.7 Hz, 2H), 6.55 (s, 1H), 5.39 (s, 2H), 3.74 (s, 2H), 2.20 (s, 6H).

### Step 9) Synthesis of 3-(5-(1H-pyrazol-1-yl)pyrid-2-yl)-1-(2,6-difluorobenzyl)-5-((dimethyl amino)methyl)-6-(4-aminophenyl)thieno[2,3-d]pyrimidine-2,4(1H,3H)-dione

At 25 °C, to a 100 mL one-neck round bottom flask were added 3-(5-(1*H*-pyrazol-1-yl)pyrid-2-yl)-1-(2,6-difluorobenzyl)-5-((dimethylamino)methyl)-6-(4-nitrophenyl)thieno[2,3-*d*]pyrimidine-2,4(1*H*,3*H*)-dione (0.6 g, 0.97 mmol), hydrogen chloride-ethyl acetate (2 mL, 2 M) and methanol (10 mL), and Pd/C (0.18 g) was added, the mixture was stirred under H₂ for 3 hours; the reaction was terminated, the mixture was concentrated to remove the most solvent, and methanol (10 mL) was added, the resulting mixture was adjusted with sodium bicarbonate solid to pH = 8, the mixture was loaded after mixing the mixture with silica gel, then eluting silica gel column by an elution solvent (dichloromethane / methanol (v/v) = 30/1) to give the title compound as a light yellow solid (0.55 g, 96.6%).
MS (ESI, pos. ion) *m*/*z:* 586.1 [M+H]⁺;
¹H NMR (400 MHz, CDCl₃) *δ* (ppm) 9.03 (d, *J =* 2.6 Hz, 1H), 8.27 (dd, *J =* 8.5, 2.7 Hz, 1H), 8.01 (d, *J* = 2.4 Hz, 1H), 7.81 (s, 1H), 7.52 (d, *J* = 8.5 Hz, 1H), 7.42 - 7.39 (m, 1H), 7.32 (d, *J* = 8.4 Hz, 2H), 6.94 (t*, J* = 8.1 Hz, 2H), 6.73 (d, *J =* 8.4 Hz, 2H), 6.56 (s, 1H), 5.38 (s, 2H), 3.68 (s, 2H), 2.15 (s, 6H).

### Step 10) Synthesis of 1-(4-(3-(5-(1H-pyrazol-1-yl)pyrid-2-yl)-1-(2,6-difluorobenzyl)-5-((dimethylamino)methyl)-2,4-dioxo-1,2,3,4-tetrahydrothieno[2,3-d]pyrimidin-6-yl)phenyl)-3-methoxyurea

To a 100 mL one-neck round bottom flask were added 3-(5-(1*H*-pyrazol-1-yl)pyrid-2-yl)-1-(2,6-difluorobenzyl)-5-((dimethylamino)methyl)-6-(4-aminophenyl)thieno[2,3-*d*]pyrimidine-2,4(1*H*,3*H*)-dione (0.55 g, 0.94 mmol), phenyl *N*-methoxycarbamate (0.785 g, 4.70 mmol) and N,N-dimethylformamide (10 mL), and then triethylamine (0.65 mL, 4.7 mmol) and 4-dimethylaminopyridine (0.012 g, 0.094 mmol) were added, the mixture was stirred in an oil bath at 60 °C for 12 hours; the reaction was terminated, and water (15 mL) was added, the mixture was filtered, the filter cake was purified by silica gel chromatography eluted with (dichloromethane / methanol (v/v) = 30/1) to give the title compound as a white solid (0.365 g, 59.0%).
MS (ESI, pos. ion) *m*/*z:* 658.9 [M+H]⁺;
¹H NMR (400 MHz, DMSO-*d₆*) *δ* (ppm) 9.74 (s, 1H), 9.29 (s, 1H), 9.14 (d, *J =* 2.5 Hz, 1H), 8.70 (d*, J* = 2.4 Hz, 1H), 8.49 (dd, *J =* 8.6, 2.6 Hz, 1H), 7.89 (s, 1H), 7.80 (d, *J =* 8.5 Hz, 2H), 7.70 (d, *J* = 8.5 Hz, 1H), 7.52 - 7.49 (m, 1H), 7.44 (d, *J* = 8.4 Hz, 2H), 7.17 (t, *J* = 8.2 Hz, 2H), 6.67 (s, 1H), 5.41 (s, 2H), 4.39 (s, 2H), 3.64 (s, 3H), 2.60 (s, 6H).

### Example 2 1-(4-(3-(5-(2H-1,2,3-triazol-2-yl)pyrid-2-yl)-1-(2,6-difluorobenzyl)-5-((dimethylamino)methyl)-2,4-dioxo-1,2,3,4-tetrahydrothieno[2,3-d]pyrimidin-6-yl)phenyl)-3-methoxyurea

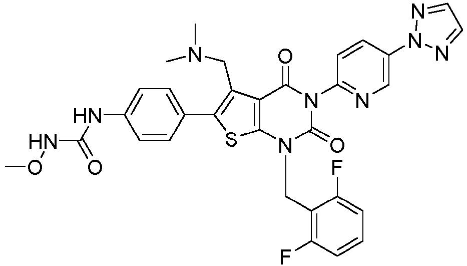

### Step 1) Synthesis of ethyl 2-(3-(5-(2H-1,2,3-triazol-2-yl)pyrid-2-yl)ureido)-4-methyl-5-(4-nitrophenyl)thiophene-3-carboxylate

The title compound in this step was prepared according to the method described in **Example 1, Step 4,** *i.e.* ethyl 4-methyl-5-(4-nitrophenyl)-2-((phenoxycarbonyl)amino) thiophene-3-carboxylate (500 mg, 1.17 mmol), 5-(triazol-2-yl)pyridine-2-amine (0.28 g, 1.7 mmol) and triethylamine (0.5 mL, 3.6 mmol) in tetrahydrofuran (10 mL) reacted together to give the title compound as a yellow solid (0.33 g, 57.3%).
MS (ESI, pos. ion) *m*/*z:* 494.1 [M+H]⁺.

### Step 2) Synthesis of 3-(5-(2H-1,2,3-triazol-2-yl)pyrid-2-yl)-5-methyl-6-(4-nitrophenyl) thieno[2,3-d]pyrimidine-2,4(1H,3H)-dione

The title compound in this step was prepared according to the method described in **Example 1, Step 5,** *i.e.* ethyl 2-(3-(5-(2*H*-1,2,3-triazol-2-yl)pyrid-2-yl)ureido)-4-methyl-5-(4-nitrophenyl)thiophene-3-carboxylate (2.0 g, 4.06 mmol) and sodium methoxide (0.68 g, 12.6 mmol) in ethanol (20 mL) reacted together to give the title compound as a red solid (1.75 g, 96.3%).
MS (ESI, pos. ion) *m*/*z:* 448.0 [M+H]⁺;
¹H NMR (400 MHz, DMSO-*d₆*) *δ* (ppm) 9.16 (d, *J =* 2.4 Hz, 1H), 8.44 (dd, *J =* 8.5, 2.5 Hz, 1H), 8.24 (d, *J =* 6.1 Hz, 4H), 7.67 (d, *J =* 8.7 Hz, 2H), 7.43 (d, *J =* 8.5 Hz, 1H), 2.53 (s, 3H).

### Step 3) Synthesis of 3-(5-(2H-1,2,3-triazol-2-yl)pyrid-2-yl)-1-(2,6-difluorobenzyl)-5-methyl- 6-(4-nitrophenyl)thieno[2,3-d]pyrimidine-2,4(1H,3H)-dione

The title compound in this step was prepared according to the method described in **Example 1, Step 6,** *i.e.* 3-(5-(2*H*-1,2,3-triazol-2-yl)pyrid-2-yl)-5-methyl-6-(4-nitrophenyl)thieno [2,3-*d*]pyrimidine-2,4(1*H*,3*H*)-dione (3.6 g, 8.05 mmol), potassium iodide (2.67 g, 16.1 mmol), potassium carbonate (2.25 g, 16.1 mmol) and 2,6-difluorobenzyl choride (2.62 g, 16.1 mmol) in *N,N*-dimethylformamide (40 mL) reacted together to give the title compound as a light yellow solid (4.1 g, 88.8%).
MS (ESI, pos. ion) *m*/*z:* 574.1 [M+H]⁺.

### Step 4) Synthesis of 3-(5-(2H-1,2,3-triazol-2-yl)pyrid-2-yl)-5-(bromomethyl)-1-(2,6-difluoro benzyl)-6-(4-nitrophenyl)thieno[2,3-d]pyrimidine-2,4(1H,3H)-dione

The title compound in this step was prepared according to the method described in **Example 1, Step** 7, *i.e.* 3-(5-(2*H*-1,2,3-triazol-2-yl)pyrid-2-yl)-1-(2,6-difluorobenzyl)-5-methyl-6-(4-nitrophenyl)thieno[2,3-*d*]pyrimidine-2,4(1*H*,3*H*)-dione (1.8 g, 3.14 mmol), azobisisobutyronitrile (0.13 g, 0.77 mmol) and *N*-bromosuccinimide (0.74 g, 4.1 mmol) in chlorobenzene (40 mL) reacted together to give the title compound as a light yellow solid (1.84 g, 89.8%).

### Step 5) Synthesis of 3-(5-(2H-1,2,3-triazol-2-yl)pyrid-2-yl)-1-(2,6-difluorobenzyl)-5-((dimethylamino)methyl)-6-(4-nitrophenyl)thieno[2,3-d]pyrimidine-2,4(1H,3H)-dione

The title compound in this step was prepared according to the method described in **Example 1, Step 8,** *i.e.* 3-(5-(2*H*-1,2,3-triazol-2-yl)pyrid-2-yl)-5-(bromomethyl)-1-(2,6-difluorobenzyl)-6-(4-nitrophenyl)thieno[2,3-*d*]pyrimidine-2,4(1*H*,3*H*)-dione (1.84 g, 2.82 mmol), dimethylamine hydrochloride (1.39 g, 16.9 mmol) and triethylamine (2.37 mL, 16.9 mmol) in *N,N-*dimethylformamide (30 mL) reacted together and the reaction mixture was purified by silica gel chromatography eluted with (dichloromethane / methanol (v/v) = 40/1) to give the title compound as a light yellow solid (1.54 g, 88.6%).
MS (ESI, pos. ion) *m*/*z:* 617.2 [M+H]⁺;
¹H NMR (400 MHz, CDCl₃) *δ* (ppm) 9.44 (d, *J =* 2.4 Hz, 1H), 8.58 (dd, *J =* 8.6, 2.6 Hz, 1H), 8.29 (d, *J* = 8.7 Hz, 2H), 7.91 - 7.89 (m, 4H), 7.51 (d, *J* = 8.5 Hz, 1H), 7.38 - 7.30 (m, 1H), 6.95 (t, *J* = 8.1 Hz, 2H), 5.39 (s, 2H), 3.73 (s, 2H), 2.20 (s, 6H).

### Step 6) Synthesis of 3-(5-(2H-1,2,3-triazol-2-yl)pyrid-2-yl)-1-(2,6-difluorobenzyl)-5-((dimethylamino)methyl)-6-(4-aminophenyl)thieno[2,3-d]pyrimidine-2,4(1H,3H)-dione

The title compound in this step was prepared according to the method described in **Example 1, Step 9,** *i.e.* 3-(5-(2*H*-1,2,3-triazol-2-yl)pyrid-2-yl)-1-(2,6-difluorobenzyl)-5-((dimethylamino)methyl)-6-(4-nitrophenyl)thieno[2,3-d]pyrimidine-2,4(1*H*,3*H*)-dione (1.25 g, 2.03 mmol), Pd/C (0.375 g) and hydrogen chloride-ethyl acetate solution (1.5 mL, 2 M) in methanol (15 mL) reacted together and the reaction mixture was purified by silica gel chromatography eluted with (dichloromethane / methanol (v/v) = 30/1) to give the title compound as a light yellow solid (1.15 g, 96.7%).
MS (ESI, pos. ion) *m*/*z:* 587.2 [M+H]⁺;
¹H NMR (400 MHz, CDCl₃) *δ* (ppm) 9.41 (d, *J =* 2.4 Hz, 1H), 8.56 (dd, *J =* 8.6, 2.6 Hz, 1H), 7.88 (s, 2H), 7.71 (d, *J* = 8.5 Hz, 1H), 7.35 - 7.29 (m, 1H), 7.23 (d, *J* = 8.4 Hz, 2H), 6.92 (t, *J* = 8.1 Hz, 2H), 6.72 (d, *J =* 8.4 Hz, 2H), 5.33 (s, 2H), 3.99 (s, 3H), 2.32 (s, 6H).

### Step 7) Synthesis of 1-(4-(3-(5-(2H-1,2,3-triazol-2-yl)pyrid-2-yl)-1-(2,6-difluorobenzyl)- 5-((dimethylamino)methyl)-2,4-dioxo-1,2,3,4-tetrahydrothieno[2,3-d]pyrimidin-6-yl)phenyl)-3-methoxyurea

The title compound in this step was prepared according to the method described in **Example 1, Step 10**, *i.e.* 3-(5-(2*H*-1,2,3-triazol-2-yl)pyrid-2-yl)-1-(2,6-difluorobenzyl)-5-((dimethylamino)methyl)-6-(4-aminophenyl)thieno[2,3-*d*]pyrimidine-2,4(1*H*,3*H*)-dione (1.15 g, 1.96 mmol), phenyl *N*-methoxycarbamate (1.31 g, 7.84 mmol), triethylamine (1.36 mL, 9.78 mmol) and 4-dimethylaminopyridine (0.024 g, 0.19 mmol) in *N,N*-dimethylformamide (15 mL) reacted together and the reaction mixture was purified by silica gel chromatography eluted with (dichloromethane / methanol (v/v) = 20/1) to give the title compound as a white solid (0.885 g, 68.4%).
MS (ESI, pos. ion) *m*/*z:* 660.1 [M+H]⁺;
¹H NMR (400 MHz, CDCl₃) *δ* (ppm) 9.43 (d, *J =* 2.5 Hz, 1H), 8.56 (dd, *J =* 8.5, 2.6 Hz, 1H), 7.88 (s, 2H), 7.65 (s, 1H), 7.57 - 7.50 (m, 4H), 7.36 - 7.29 (m, 1H), 6.93 (t, *J* = 8.1 Hz, 2H), 5.35 (s, 2H), 3.81 (s, 3H), 3.69 (s, 2H), 2.14 (s, 6H).

### Example 3 1-(4-(3-(6-(1H-pyrazol-1-yl)pyrid-3-yl)-1-(2,6-difluorobenzyl)-5-((dimethyl amino)methyl)-2,4-dioxo-1,2,3,4-tetrahydrothieno[2,3-d]pyrimidin-6-yl)phenyl)-3-methoxyurea

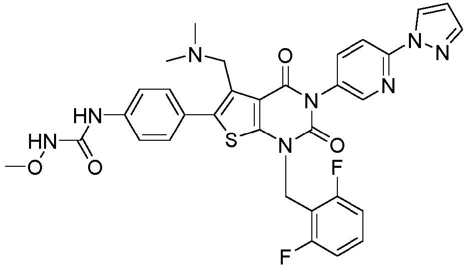

### Step 1) Synthesis of ethyl 2-(3-(6-(1H-pyrazol-1-yl)pyrid-3-yl)ureido)-4-methyl-5- (4-nitrophenyl)thiophene-3-carboxylate

The title compound in this step was prepared according to the method described in **Example 1, Step 4,** *i.e.* ethyl 4-methyl-5-(4-nitrophenyl)-2-((phenoxycarbonyl)amino) thiophene-3-carboxylate (500 mg, 1.17 mmol), 6-(pyrazol-1-yl)pyridine-3-amine (0.2 g, 1.25 mmol) and triethylamine (0.5 mL, 3.6 mmol) in tetrahydrofuran (6 mL) reacted together to give the title compound as a light yellow solid (0.495 g, 85.9%).
MS (ESI, pos. ion) *m*/*z:* 493.2 [M+H]⁺;
¹H NMR (400 MHz, CDCl₃) *δ* (ppm) 11.21 (s, 1H), 8.53 (s, 1H), 8.44 (s, 1H), 8.29 (d, *J =* 8.8 Hz, 2H), 8.19 (d, *J =* 8.9 Hz, 1H), 8.00 (d*, J =* 9.1 Hz, 1H), 7.76 (s, 1H), 7.59 (d, *J =* 8.7 Hz, 2H), 7.45 (s, 1H), 6.49 (s, 1H), 4.41 (q, *J* = 7.1 Hz, 2H), 2.45 (s, 3H), 1.45 (t, *J =* 7.1 Hz, 3H).

### Step 2) Synthesis of 3-(6-(1H-pyrazol-1-yl)pyrid-3-yl)-5-methyl-6-(4-nitrophenyl)thieno [2,3-d]pyrimidine-2,4(1H, 3H)-dione

The title compound in this step was prepared according to the method described in **Example 1, Step 5,** *i.e.* ethyl 2-(3-(6-(1*H*-pyrazol-1-yl)pyrid-3-yl)ureido)-4-methyl-5- (4-nitrophenyl)thiophene-3-carboxylate (0.32 g, 0.65 mmol) and sodium methoxide (0.11 g, 2.04 mmol) in tetrahydrofuran (10 mL) and methanol (5 mL) reacted together to give the title compound as a light yellow solid (0.25 g, 87.5%).
MS (ESI, pos. ion) *m*/*z:* 447.1 [M+H]⁺;
¹H NMR (400 MHz, DMSO-*d₆*) *δ* (ppm) 8.65 (s, 1H), 8.27 - 8.18 (m, 3H), 7.96 (d, *J* = 8.5 Hz, 1H), 7.85 (s, 1H), 7.77 (d, *J =* 8.5 Hz, 1H), 7.67 (d, *J =* 8.7 Hz, 2H), 6.60 (s, 1H), 2.55 (s, 3H).

### Step 3) Synthesis of 3-(6-(1H-pyrazol-1-yl) pyrid-3-yl )-1-(2,6-difluorobenzyl)-5-methyl-6-(4-nitrophenyl)thieno[2,3-d]pyrimidine-2,4(1H,3H)-dione

The title compound in this step was prepared according to the method described in **Example 1, Step 6,** *i.e.* 3-(6-(1*H*-pyrazol-1-yl)pyrid-3-yl)-5-methyl-6-(4-nitrophenyl)thieno [2,3-d]pyrimidine-2,4(1*H*,3*H*)-dione (1.25 g, 2.8 mmol), potassium iodide (0.93 g, 5.6 mmol), potassium carbonate (0.78 g, 5.6 mmol) and 2,6-difluorobenzyl choride (0.68 g, 4.2 mmol) in *N,N-*dimethylformamide (15 mL) reacted together to give the title compound as a light yellow solid (1.16 g, 72.4%).
MS (ESI, pos. ion) *m*/*z:* 572.9 [M+H]⁺;
¹H NMR (400 MHz, DMSO-*d₆*) *δ* (ppm) 8.67 (s, 1H), 8.43 (s, 1H), 8.34 (d, *J =* 8.4 Hz, 2H), 8.08 (d, *J* = 8.5 Hz, 1H), 7.98 (d, *J* = 8.8 Hz, 1H), 7.89 (s, 1H), 7.77 (d, *J* = 8.5 Hz, 2H), 7.53 - 7.43 (m, 1H), 7.16 (t, *J =* 8.0 Hz, 2H), 6.63 (s, 1H), 5.32 (s, 2H), 2.56 (s, 3H).

### Step 4) Synthesis of 3-(6-(1H-pyrazol-1-yl) pyrid-3-yl )-5-(bromomethyl)-1-(2,6-difluoro benzyl)-6-(4-nitrophenyl)thieno[2,3-d]pyrimidine-2,4(1H,3H)-dione

The title compound in this step was prepared according to the method described in **Example 1, Step** 7, *i.e.* 3-(6-(1*H*-pyrazol-1-yl)pyrid-3-yl)-1-(2,6-difluorobenzyl)-5-methyl- 6-(4-nitrophenyl)thieno[2,3-*d*]pyrimidine-2,4(1*H*,3*H*)-dione (0.15 g, 0.26 mmol), azobisisobutyronitrile (0.01 g, 0.06 mmol) and *N*-bromosuccinimide (0.07 g, 0.4 mmol) in chlorobenzene (5 mL) reacted together to give the title compound as a light red solid (0.17 g, 99.6%).
MS (ESI, pos. ion) *m*/*z:* 650.8 [M+H]⁺.

### Step 5) Synthesis of 3-(6-(1H-pyrazol-1-yl) pyrid-3-yl )-1-(2,6-difluorobenzyl)-5-((dimethyl amino)methyl)-6-(4-nitrophenyl)thieno[2,3-d]pyrimidine-2,4(1H,3H)-dione

The title compound in this step was prepared according to the method described in **Example 1, Step 8,** *i.e.* 3-(6-(1*H*-pyrazol-1-yl)pyrid-3-yl)-5-(bromomethyl)-1-(2,6-difluoro benzyl)-6-(4-nitrophenyl)thieno[2,3-*d*]pyrimidine-2,4(1*H*,3*H*)-dione (1.08 g, 1.66 mmol), dimethylamine hydrochloride (0.34 g, 4.1 mmol) and triethylamine (0.8 mL, 5.7 mmol) in *N,N-*dimethylformamide (8 mL) reacted together and the reaction mixture was purified by silica gel chromatography eluted with (dichloromethane / methanol (v/v) = 40/1) to give the title compound as a light yellow solid (0.7 g, 68.6%).
MS (ESI, pos. ion) *m*/*z:* 616.2 [M+H]⁺;
¹H NMR (400 MHz, CDCl₃) *δ* (ppm) 8.61 (d, *J* = 2.2 Hz, 1H), 8.37 (d, *J* = 2.0 Hz, 1H), 8.31 (d, *J* = 8.7 Hz, 2H), 8.15 (d, *J* = 8.7 Hz, 1H), 7.87 (d, *J* = 8.7 Hz, 2H), 7.81 - 7.75 (m, 2H), 7.41 - 7.34 (m, 1H), 6.98 (t, *J =* 8.1 Hz, 2H), 6.50 (s, 1H), 5.43 (s, 2H), 3.76 (s, 2H), 2.22 (s, 6H).

### Step 6) Synthesis of 3-(6-(1H-pyrazol-1-yl)pyrid-3-yl)-1-(2,6-difluorobenzyl)-5-((dimethyl amino)methyl)-6-(4-aminophenyl)thieno[2,3-d]pyrimidine-2,4(1H,3H)-dione

The title compound in this step was prepared according to the method described in **Example 1, Step 9,** *i.e.* 3-(6-(1*H*-pyrazol-1-yl)pyrid-3-yl)-1-(2,6-difluorobenzyl)-5-((dimethyl amino)methyl)-6-(4-nitrophenyl)thieno[2,3-*d*]pyrimidine-2,4(1*H*,3*H*)-dione (1.08 g, 1.75 mmol), Pd/C (0.33 g) and hydrogen chloride-ethyl acetate solution (1.5 mL, 2 M) in methanol (8 mL) reacted together and the reaction mixture was purified by silica gel chromatography eluted with (dichloromethane / methanol (v/v) = 20/1) to give the title compound as an off-white solid (1.0 g, 97.3%).
MS (ESI, pos. ion) *m*/*z:* 586.2 [M+H]⁺;
¹H NMR (400 MHz, CDCl₃) *δ* (ppm) 8.60 (d, *J* = 2.2 Hz, 1H), 8.39 (d, *J* = 2.1 Hz, 1H), 8.13 (d, *J* = 8.7 Hz, 1H), 7.82 (dd, *J* = 8.7, 2.2 Hz, 1H), 7.77 (s, 1H), 7.36 - 7.29 (m, 2H), 7.28 (s, 1H), 6.95 (t, *J* = 8.1 Hz, 2H), 6.73 (d, *J =* 8.4 Hz, 2H), 6.50 - 6.46 (m, 1H), 5.39 (s, 2H), 3.74 (s, 2H), 2.17 (s, 6H).

### Step 7) Synthesis of 1-(4-(3-(6-(1H-pyrazol-1-yl)pyrid-3-yl)-1-(2,6-difluorobenzyl)-5-((dimethylamino)methyl)-2,4-dioxo-1,2,3,4-tetrahydrothieno[2,3-d]pyrimidin-6-yl)phenyl)-3-methoxyurea

The title compound in this step was prepared according to the method described in **Example 1, Step 10**, *i.e.* 3-(6-(1*H*-pyrazol-1-yl)pyrid-3-yl)-1-(2,6-difluorobenzyl)-5-((dimethyl amino)methyl)-6-(4-aminophenyl)thieno[2,3-d]pyrimidine-2,4(1*H*,3*H*)-dione (1.0 g, 1.71 mmol), phenyl *N*-methoxycarbamate (1.43 g, 8.55 mmol), triethylamine (2.61 mL, 18.8 mmol) and 4-dimethylaminopyridine (0.02 g, 0.19 mmol) in *N,N-*dimethylformamide (10 mL) reacted together and the reaction mixture was purified by silica gel chromatography eluted with (dichloromethane / methanol (v/v) = 20/1) to give the title compound as a white solid (0.75 g, 66.7%).
MS (ESI, pos. ion) *m*/*z:* 659.2 [M+H]⁺;
¹H NMR (400 MHz, CDCl₃) *δ* (ppm) 8.60 (d, *J* = 2.4 Hz, 1H), 8.38 (d, *J* = 2.3 Hz, 1H), 8.14 (d, *J* = 8.7 Hz, 1H), 7.83 - 7.74 (m, 2H), 7.67 (s, 1H), 7.58 (d, *J* = 8.5 Hz, 2H), 7.50 (d, *J* = 8.5 Hz, 2H), 7.39 - 7.29 (m, 2H), 6.96 (t, *J* = 8.1 Hz, 2H), 6.49 (d, *J* = 1.7 Hz, 1H), 5.40 (s, 2H), 3.84 (s, 3H), 3.71 (s, 2H), 2.15 (s, 6H).

### Example 4 1-(4-(3-(6-(2H-1,2,3-triazol-2-yl) pyrid-3-yl)-1-(2,6-difluorobenzyl)-5-((dimethylamino)methyl)-2,4-dioxo-1,2,3,4-tetrahydrothieno[2,3-d]pyrimidin-6-yl)phenyl)-3-methoxyurea

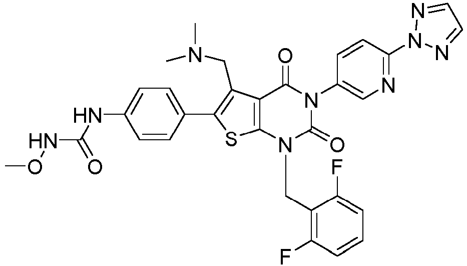

### Step 1) Synthesis of ethyl 2-(3-(6-(2H-1,2,3-triazol-2-yl)pyrid-3-yl)ureido)-4-methyl-5-(4-nitrophenyl)thiophene-3-carboxylate

The title compound in this step was prepared according to the method described in **Example 1, Step 4**, *i.e.* ethyl 4-methyl-5-(4-nitrophenyl)-2-((phenoxycarbonyl)amino)thiophene -3-carboxylate (3.5 g, 8.2 mmol), 6-(triazol-2-yl)pyridine-3-amine (1.2 g, 7.4 mmol) and triethylamine (2.1 mL, 15.1 mmol) in tetrahydrofuran (35 mL) reacted together to give the title compound as a yellow solid (2.7 g, 73.0%).
MS (ESI, neg. ion) *m*/*z:* 492.2 [M-H]⁻.

### Step 2) Synthesis of 3-(6-(2H-1,2,3-triazol-2-yl)pyrid-3-yl)-5-methyl-6-(4-nitrophenyl) thieno[2,3-d]pyrimidine-2,4(1H,3H)-dione

The title compound in this step was prepared according to the method described in **Example 1, Step 5,** *i.e.* ethyl 2-(3-(6-(2*H*-1,2,3-triazol-2-yl)pyrid-3-yl)ureido)-4-methyl-5-(4-nitrophenyl)thiophene-3-carboxylate (1.4 g, 2.8 mmol) and sodium methoxide (0.46 g, 8.51 mmol) in ethanol (50 mL) reacted together to give the title compound as a yellow solid (1.1 g, 87.9%).
MS (ESI, pos. ion) *m*/*z:* 448.2 [M+H]⁺;
¹H NMR (400 MHz, DMSO-*d₆*) *δ* (ppm) 8.90 (d, *J* = 1.0 Hz, 1H), 8.38 (d, *J* = 2.0 Hz, 1H), 8.24 (d, *J* = 8.9 Hz, 2H), 8.17 *(d, J* = 8.5 Hz, 1H), 8.03 *(d, J* = 1.0 Hz, 1H), 7.94 (dd, *J* = 8.5, 2.4 Hz, 1H), 7.67 (d, *J* = 8.9 Hz, 2H), 2.55 (s, 3H).

### Step 3) Synthesis of 3-(6-(2H-1,2,3-triazol-2-yl)pyrid-3-yl)-1-(2,6-difluorobenzyl)-5-methyl- 6-(4-nitrophenyl)thieno[2,3-d]pyrimidine-2,4(1H,3H)-dione

The title compound in this step was prepared according to the method described in **Example 1, Step 6,** *i.e.* 3-(6-(2*H*-1,2,3-triazol-2-yl)pyrid-3-yl)-5-methyl-6-(4-nitrophenyl) thieno[2,3-*d*]pyrimidine-2,4(1*H*,3*H*)-dione (1.1 g, 2.46 mmol), potassium iodide (0.82 g, 4.94 mmol), potassium carbonate (0.68 g, 4.92 mmol) and 2,6-difluorobenzyl choride (0.8 g, 4.92 mmol) in N,N-dimethylformamide (50 mL) reacted together to give the title compound as a yellow solid (1.3 g, 92.2%).
MS (ESI, pos. ion) *m*/*z:* 573.9 [M+H]⁺;
¹H NMR (400 MHz, DMSO-*d₆*) *δ* (ppm) 8.93 (d, *J* = 1.1 Hz, 1H), 8.60 (d, *J* = 2.2 Hz, 1H), 8.35 (d, *J* = 8.8 Hz, 2H), 8.31 (d, *J* = 8.7 Hz, 1H), 8.15 (dd, *J* = 8.6, 2.4 Hz, 1H), 8.05 (d, *J* = 1.1 Hz, 1H), 7.77 (d, *J* = 8.8 Hz, 2H), 7.53 - 7.43 (m, 1H), 7.16 (t, *J* = 8.2 Hz, 2H), 5.33 (s, 2H), 2.54 (s, 2H).

### Step 4) Synthesis of 3-(6-(2H-1,2,3-triazol-2-yl)pyrid-3-yl)-5-(bromomethyl)-1-(2,6-difluorobenzyl)-6-(4-nitrophenyl)thieno[2,3-d]pyrimidine-2,4(1H,3H)-dione

The title compound in this step was prepared according to the method described in **Example 1, Step 7,** *i.e.* 3-(6-(2*H*-1,2,3-triazol-2-yl)pyrid-3-yl)-1-(2,6-difluorobenzyl)-5- methyl-6-(4-nitrophenyl)thieno[2,3-*d*]pyrimidine-2,4(1*H*,3*H*)-dione (1.3 g, 2.3 mmol), azobisisobutyronitrile (0.2 g, 1.22 mmol) and N-bromosuccinimide (0.61 g, 3.43 mmol) in chlorobenzene (100 mL) reacted together to give the title compound as a yellow solid (1.48 g, 98.7%).

### Step 5) Synthesis of 3-(6-(2H-1,2,3-triazol-2-yl)pyrid-3-yl)-1-(2,6-difluorobenzyl)-5-((dimethyl amino)methyl)-6-(4-nitrophenyl)thieno[2,3-d]pyrimidine-2,4(1H,3H)-dione

The title compound in this step was prepared according to the method described in **Example 1, Step 8,** *i.e.* 3-(6-(2*H*-1,2,3-triazol-2-yl)pyrid-3-yl)-5-(bromomethyl)-1-(2,6-difluoro benzyl)-6-(4-nitrophenyl)thieno[2,3-d]pyrimidine-2,4(1*H*,3*H*)-dione (2.6 g, 3.99 mmol), dimethylamine hydrochloride (0.65 g, 7.97 mmol) and triethylamine (2.2 mL, 15.7 mmol) in *N,N-*dimethylformamide (30 mL) reacted together and the reaction mixture was purified by silica gel chromatography eluted with (dichloromethane / methanol (v/v) = 40/1) to give the title compound as a yellow solid (2.2 g, 89.5%).
MS (ESI, pos. ion) *m*/*z:* 617.2 [M+H]⁺;
¹H NMR (400 MHz, CDCl₃) *δ* (ppm) 8.64 (s, 1H), 8.49 (d, *J* = 1.8 Hz, 1H), 8.38 (d, *J* = 8.6 Hz, 1H), 8.31 (d, *J* = 8.6 Hz, 2H), 7.92 (dd, *J* = 8.6, 2.1 Hz, 1H), 7.87 (s, 2H), 7.85 (s, 1H), 7.42 - 7.34 (m, 1H), 6.99 (t, *J =* 8.1 Hz, 2H), 5.43 (s, 2H), 3.75 (s, 2H), 2.20 (s, 6H).

### Step 6) Synthesis of 3-(6-(2H-1,2,3-triazol-2-yl)pyrid-3-yl)-1-(2,6-difluorobenzyl)-5-((dimethyl amino)methyl)-6-(4-aminophenyl)thieno[2,3-d]pyrimidine-2,4(1H,3H)-dione

The title compound in this step was prepared according to the method described in **Example 1, Step 9,** *i.e.* 3-(6-(2*H*-1,2,3-triazol-2-yl)pyrid-3-yl)-1-(2,6-difluorobenzyl)-5-((dimethylamino)methyl)-6-(4-nitrophenyl)thieno[2,3-*d*]pyrimidine-2,4(1*H*,3*H*)-dione (2.0 g, 3.24 mmol), Pd/C (0.6 g) and hydrogen chloride-ethyl acetate solution (5 mL, 2 M) in methanol (40 mL) reacted together and the reaction mixture was purified by silica gel chromatography eluted with (dichloromethane / methanol (v/v) = 20/1) to give the title compound as a grey solid (1.74 g, 91.7%).
MS (ESI, pos. ion) *m*/*z:* 586.8 [M+H]⁺;
¹H NMR (400 MHz, CDCl₃) *δ* (ppm) 8.71 (s, 1H), 8.63 (s, 1H), 8.36 (d, *J* = 8.2 Hz, 2H), 7.85 (s, 1H), 7.38 - 7.32 (m, 1H), 7.16 (d, *J* = 8.1 Hz, 2H), 6.95 (t, *J* = 8.1 Hz, 2H), 6.76 (d, *J* = 8.3 Hz, 2H), 5.38 (s, 2H), 4.04 (s, 2H), 2.63 (s, 6H).

### Step 7) Synthesis of 1-(4-(3-(6-(2H-1,2,3-triazol-2-yl) pyrid-3-yl )-1-(2,6-difluorobenzyl)-5 - ((dimethylamino)methyl)-2,4-dioxo-1,2,3,4-tetrahydrothieno[2,3-d]pyrimidin-6-yl)phenyl)-3-methoxyurea

The title compound in this step was prepared according to the method described in **Example 1, Step 10,** *i.e.* 3-(6-(2*H*-1,2,3-triazol-2-yl)pyrid-3-yl)-1-(2,6-difluorobenzyl)- 5-((dimethylamino)methyl)-6-(4-aminophenyl)thieno[2,3-d]pyrimidine-2,4(1*H*,3*H*)-dione (0.9 g, 1.53 mmol), phenyl N-methoxycarbamate (1.0 g, 5.99 mmol), triethylamine (0.9 mL, 6.42 mmol) in *N*,*N*-dimethylformamide (20 mL) reacted together and the reaction mixture was purified by silica gel chromatography eluted with (dichloromethane / methanol (v/v) = 20/1) to give the title compound as a white solid (0.61 g, 60.3%).
MS (ESI, pos. ion) *m*/*z:* 660.4 [M+H]⁺;
¹H NMR (400 MHz, DMSO-*d₆*) *δ* (ppm) 9.63 (s, 1H), 9.09 (s, 1H), 8.91 (s, 1H), 8.59 (d, *J =* 2.0 Hz, 1H), 8.29 (d, *J =* 8.6 Hz, 1H), 8.14 (dd, *J =* 8.6, 2.3 Hz, 1H), 8.04 (s, 1H), 7.73 (d, *J =* 8.6 Hz, 2H), 7.53 (d, *J =* 8.5 Hz, 2H), 7.49 - 7.43 (m, 1H), 7.15 (t, *J =* 8.2 Hz, 2H), 5.31 (s, 2H), 3.64 (s, 3H), 3.63 (s, 2H), 2.06 (s, 6H).

### Example 5 1-(4-(3-(6-(1H-pyrazol-1-yl)pyrid-2-yl)-1-(2,6-difluorobenzyl)-5-((dimethyl amino)methyl)-2,4-dioxo-1,2,3,4-tetrahydrothieno[2,3-d]pyrimidin-6-yl)phenyl)-3-methoxyurea

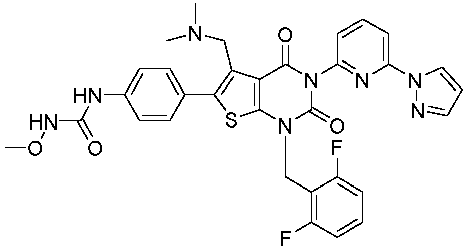

### Step 1) Synthesis of ethyl 2-(3-(6-(1H-pyrazol-1-yl)pyrid-2-yl)ureido)-4-methyl-5-(4-nitro phenyl)thiophene-3-carboxylate

The title compound in this step was prepared according to the method described in **Example 1, Step 4,** *i.e.* ethyl 4-methyl-5-(4-nitrophenyl)-2-((phenoxycarbonyl)amino) thiophene-3-carboxylate (8.0 g, 16.9 mmol), 6-(pyrazol-1-yl)pyridine-2-amine (2.8 g, 17.0 mmol) and triethylamine (7.0 mL, 50.4 mmol) in tetrahydrofuran (30 mL) reacted together to give the title compound as a light yellow solid (1.2 g, 14.0%).
¹H NMR (400 MHz, DMSO-*d₆*) *δ* (ppm) 8.51 (d, *J =* 2.4 Hz, 1H), 8.29 (d, *J =* 8.8 Hz, 2H), 7.95 (t, *J =* 8.0 Hz, 1H), 7.83 (s, 1H), 7.71 (d, *J =* 8.8 Hz, 3H), 7.58 (d, *J =* 8.0 Hz, 1H), 6.59 (s, 1H), 4.32 (q, *J =* 7.2 Hz, 2H), 2.40 (s, 3H), 1.33 (t, *J =* 7.2 Hz, 3H).

### Step 2) Synthesis of 3-(6-(1H-pyrazol-1-yl)pyrid-2-yl)-5-methyl-6-(4-nitrophenyl) thieno[2,3-d]pyrimidine-2,4(1H,3H)-dione

The title compound in this step was prepared according to the method described in **Example 1, Step 5,** *i.e.* ethyl 2-(3-(6-(1*H*-pyrazol-1-yl)pyrid-2-yl)ureido)-4-methyl-5-(4-nitro phenyl)thiophene-3-carboxylate (0.22 g, 0.45 mmol) and sodium methoxide (72 mg, 1.09 mmol) in ethanol (10 mL) reacted together to give the title compound as a light yellow solid (0.19 g, 95.3%).
MS (ESI, pos. ion) *m*/*z:* 447.0 [M+H]⁺;
¹H NMR (400 MHz, DMSO-*d₆*) *δ* (ppm) 8.49 (d, *J* = 2.0 Hz, 1H), 8.23 (d, *J* = 8.8 Hz, 2H), 8.05 (t, *J* = 8.0 Hz, 1H), 7.89 (d, *J* = 8.0 Hz, 1H), 7.84 (s, 1H), 7.67 (d, *J* = 8.8 Hz, 2H), 7.16 (d, *J* = 7.6 Hz, 1H), 6.55 (s, 1H), 2.53 (s, 3H).

### Step 3) Synthesis of 3-(6-(1H-pyrazol-1-yl)pyrid-2-yl)-1-(2,6-difluorobenzyl)-5-methyl- 6-(4-nitrophenyl)thieno[2,3-d]pyrimidine-2,4(1H,3H)-dione

The title compound in this step was prepared according to the method described in **Example 1, Step 6,** *i.e.* 3-(6-(1*H*-pyrazol-1-yl)pyrid-2-yl)-5-methyl-6-(4-nitrophenyl) thieno[2,3-*d*]pyrimidine-2,4(1*H*,3*H*)-dione (190 mg, 0.43 mmol), potassium iodide (85 mg, 0.51 mmol), potassium carbonate (71 mg, 0.51 mmol) and 2,6-difluorobenzyl choride (0.1 g, 0.6 mmol) in *N,N-*dimethylformamide (5 mL) reacted together to give the title compound as a light yellow solid (0.2 g, 82.1%).
MS (ESI, pos. ion) *m*/*z:* 573.2 [M+H]⁺,
¹H NMR (400 MHz, CDCl₃) *δ* (ppm) 8.48 (d, *J* = 2.4 Hz, 1H), 8.29 (d, *J* = 8.8 Hz, 2H), 8.10 (d, *J* = 8.0 Hz, 1H), 8.01 (t, *J* = 8.0 Hz, 1H), 7.73 (s, 1H), 7.56 (d, *J* = 8.8 Hz, 2H), 7.37-7.32 (m, 1H), 7.23 (d, *J* = 7.6 Hz, 1H), 6.95 (t, *J =* 8.0 Hz, 2H), 6.43 (s, 1H), 5.39 (s, 2H), 2.57 (s, 3H).

### Step 4) Synthesis of 3-(6-(1H-pyrazol-1-yl)pyrid-2-yl)-5-(bromomethyl)-1-(2,6-difluoro benzyl)-6-(4-nitrophenyl)thieno[2,3-d]pyrimidine-2,4(1H,3H)-dione

The title compound in this step was prepared according to the method described in **Example 1, Step 7,** *i.e.* 3-(6-(1*H*-pyrazol-1-yl)pyrid-2-yl)-1-(2,6-difluorobenzyl)-5-methyl-6- (4-nitrophenyl)thieno[2,3-*d*]pyrimidine-2,4(1*H*,3*H*)-dione (0.19 g, 0.33 mmol), azobisisobutyronitrile (33 mg, 0.2 mmol) and *N*-bromosuccinimide (0.12 g, 0.7 mmol) in chlorobenzene (8 mL) reacted together to give the title compound as a light yellow solid (0.21 g, 99%).

### Step 5) Synthesis of 3-(6-(1H-pyrazol-1-yl)pyrid-2-yl)-1-(2,6-difluorobenzyl)-5-((dimethyl amino)methyl)-6-(4-nitrophenyl)thieno[2,3-d]pyrimidine-2,4(1H,3H)-dione

The title compound in this step was prepared according to the method described in **Example 1, Step 8,** *i.e.* 3-(6-(1*H*-pyrazol-1-yl)pyrid-2-yl)-5-(bromomethyl)-1-(2,6-difluoro benzyl)-6-(4-nitrophenyl)thieno[2,3-*d*]pyrimidine-2,4(1*H*,3*H*)-dione (3.6 g, 5.5 mmol), dimethylamine hydrochloride (0.9 g, 10.9 mmol) and triethylamine (3.8 mL, 27.1 mmol) in *N,N-*dimethylformamide (30 mL) reacted together and the reaction mixture was purified by silica gel chromatography eluted with (dichloromethane / methanol (v/v) = 40/1) to give the title compound as a light yellow solid (2.2 g, 65%).
MS (ESI, pos. ion) *m*/*z:* 616.3 [M+H]⁺;
¹H NMR (400 MHz, CDCl₃) *δ* (ppm) 8.47 (d, *J* = 2.4 Hz, 1H), 8.28 (d, *J* = 8.8 Hz, 2H), 8.09 (d, *J* = 8.4 Hz, 1H), 8.00 (d, *J* = 8.4 Hz, 1H), 7.86 (d, *J* = 8.8 Hz, 2H), 7.73 (s, 1H), 7.38 - 7.30 (m, 1H), 7.24 (d, *J* = 7.6 Hz, 1H), 6.96 (t, *J =* 8.0 Hz, 2H), 6.43 - 6.40 (m, 1H), 5.40 (s, 2H), 2.95 (s, 2H), 2.20 (s, 6H).

### Step 6) Synthesis of 3-(6-(1H-pyrazol-1-yl)pyrid-2-yl)-1-(2,6-difluorobenzyl)-5-((dimethylamino)methyl)-6-(4-aminophenyl)thieno[2,3-d]pyrimidine-2,4(1H,3H)-dione

The title compound in this step was prepared according to the method described in **Example 1, Step 9,** *i.e.* 3-(6-(1*H*-pyrazol-1-yl)pyrid-2-yl)-1-(2,6-difluorobenzyl)-5-((dimethyl amino)methyl)-6-(4-nitrophenyl)thieno[2,3-*d*]pyrimidine-2,4(1*H*,3*H*)-dione (2.2 g, 3.58 mmol), Pd/C (0.208 g) and hydrogen chloride-ethyl acetate solution (2.5 mL, 2 M) in methanol (10 mL) reacted together and the reaction mixture was purified by silica gel chromatography eluted with (dichloromethane / methanol (v/v) = 20/1) to give the title compound as a light yellow solid (1.72 g, 82%).
MS (ESI, pos. ion) *m*/*z:* 586.2 [M+H]⁺;
¹H NMR (400 MHz, DMSO-*d₆*) *δ* (ppm) 8.48 (d, *J* = 2.4 Hz, 1H), 8.20 (t, *J* = 8.0 Hz, 1H), 8.04 (d, *J* = 8.0 Hz, 1H), 7.87 (s, 1H), 7.51 - 7.45 (m, 1H), 7.42 (d, *J* = 7.6 Hz, 1H), 7.23 (d, *J* = 8.4 Hz, 2H), 7.14 (t, *J =* 8.4 Hz, 2H), 6.62 (d, *J =* 8.4 Hz, 2H), 6.58 (d, *J =* 1.6 Hz, 1H), 5.44 (s, 2H), 5.30 (s, 2H), 2.04 (s, 6H).

### Step 7) Synthesis of 1-(4-(3-(6-(1H-pyrazol-1-yl)pyrid-2-yl)-1-(2,6-difluorobenzyl)-5-((dimethylamino)methyl)-2,4-dioxo-1,2,3,4-tetrahydrothieno[2,3-d]pyrimidin-6-yl)phenyl)-3-methoxyurea

The title compound in this step was prepared according to the method described in **Example 1, Step 10,** *i.e.* 3-(6-(1*H*-pyrazol-1-yl)pyrid-2-yl)-1-(2,6-difluorobenzyl)-5-((dimethyl amino)methyl)-6-(4-aminophenyl)thieno[2,3-*d*]pyrimidine-2,4(1*H*,3*H*)-dione (1.1 g, 1.8 mmol), phenyl *N*-methoxycarbamate (1.8 g, 10.8 mmol), triethylamine (1.2 mL, 8.55 mmol) and 4-dimethylaminopyridine (0.02 g, 0.19 mmol) in *N,N*-dimethylformamide (10 mL) reacted together and the reaction mixture was purified by silica gel chromatography eluted with (dichloromethane / methanol (v/v) = 20/1) to give the title compound as a white solid (0.61 g, 51.6%).
MS (ESI, pos. ion) *m*/*z:* 659.2 [M+H]⁺;
¹H NMR (400 MHz, CDCl₃) *δ* (ppm) 8.49 (d, *J =* 2.4 Hz, 1H), 8.07 (d, *J =* 8.0 Hz, 1H), 7.99 (t, *J* = 8.0 Hz, 1H), 7.72 (s, 1H), 7.66 (s, 1H), 7.56 (d, *J* = 8.8 Hz, 2H), 7.48 (d, *J* = 8.8 Hz, 2H), 7.37 (s, 1H), 7.35 - 7.29 (m, 1H), 7.25 (s, 1H), 6.94 (t, *J =* 8.0 Hz, 2H), 6.43 - 6.40 (m, 1H), 5.37 (s, 2H), 3.80 (s, 3H), 3.70 (s, 2H), 2.14 (s, 6H).

### Example 6 1-(4-(3-(6-(1H-pyrazol-1-yl)pyrid-3-yl)-1-(2,6-difluorobenzyl)-5-((dimethyl amino)methyl)-2,4-dioxo-1,2,3,4-tetrahydrothieno[2,3-d]pyrimidin-6-yl)phenyl)-3-ethyl urea

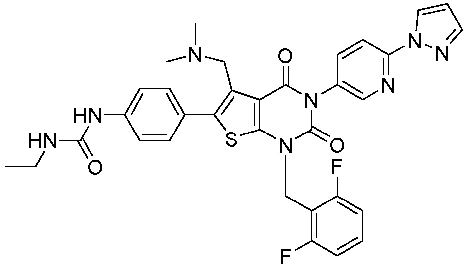

The title compound in this step was prepared according to the method described in **Example 1, Step** 10, *i.e.* 3-(6-(1*H*-pyrazol-1-yl)pyrid-3-yl)-1-(2,6-difluorobenzyl)-5-((dimethyl amino)methyl)-6-(4-aminophenyl)thieno[2,3-*d*]pyrimidine-2,4(1*H*,3*H*)-dione (0.5 g, 0.85 mmol), phenyl *N*-ethylcarbamate (0.7 g, 4.26 mmol), triethylamine (0.59 mL, 4.26 mmol) and 4-dimethylaminopyridine (10.5 mg, 0.085 mmol) in *N*,*N*-dimethylformamide (10 mL) reacted together and the reaction mixture was purified by silica gel chromatography eluted with (dichloromethane / methanol (v/v) = 20/1) to give the title compound as a white solid (0.28 g, 50%).
MS (ESI, pos. ion) *m*/*z:* 657.0 [M+H]⁺;
1H NMR (400 MHz, DMSO-*d₆*) *δ* (ppm) 8.73 - 8.61 (m, 2H), 8.42 (d, *J* = 2.1 Hz, 1H), 8.07 (d, *J* = 8.7 Hz, 1H), 7.97 (dd, *J* = 8.7, 2.4 Hz, 1H), 7.88 (s, 1H), 7.55 - 7.40 (m, 5H), 7.14 (t, *J* = 8.2 Hz, 2H), 6.64 - 6.60 (m, 1H), 6.21 (t, *J* = 5.4 Hz, 1H), 5.30 (s, 2H), 3.60 (s, 2H), 3.18 - 3.05 (m, 2H), 2.05 (s, 6H), 1.06 (t, *J* = 7.1 Hz, 3H).

### Example 7 1-(4-(3-(6-(1H-pyrazol-1-yl)pyrid-3-yl)-1-(2,6-difluorobenzyl)-5-((dimethyl amino)methyl)-2,4-dioxo-1,2,3,4-tetrahydrothieno[2,3-d]pyrimidin-6-yl)phenyl)-3-cyclopropylurea

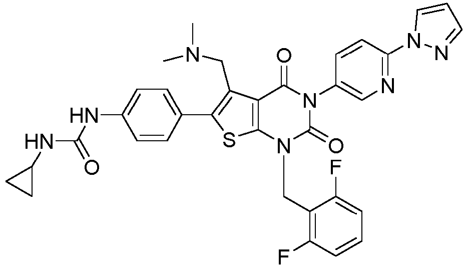

The title compound in this step was prepared according to the method described in **Example 1, Step 10,** *i.e.* 3-(6-(1*H*-pyrazol-1-yl)pyrid-3-yl)-1-(2,6-difluorobenzyl)-5-((dimethyl amino)methyl)-6-(4-aminophenyl)thieno[2,3-*d*]pyrimidine-2,4(1*H*,3*H*)-dione (0.5 g, 0.85 mmol), phenyl *N*-cyclopropylcarbamate (0.755 g, 4.26 mmol), triethylamine (0.59 mL, 4.26 mmol) and 4-dimethylaminopyridine (105 mg, 0.85 mmol) in *N,N*-dimethylformamide (10 mL) reacted together and the reaction mixture was purified by silica gel chromatography eluted with (dichloromethane / methanol (v/v) = 20/1) to give the title compound as a white solid (0.265 g, 46.5%).
MS (ESI, pos. ion) *m*/*z:* 669.2 [M+H]⁺;
1H NMR (400 MHz, DMSO-*d₆*) *δ* (ppm) 8.66 (s, 1H), 8.48 - 8.45 (m, 2H), 8.04 - 8.01 (m, 2H), 7.88 (s, 1H), 7.52 - 7.48 (m, 5H), 7.16 (t, *J =* 6.3 Hz, 2H), 6.63 (s, 1H), 6.47 (s, 1H), 5.31 (s, 2H), 3.60 (s, 2H), 2.05 (s, 6H), 1.24 (t, *J* = 12.6 Hz, 1H), 0.54 - 0.52 (m, 4H).

### Example 8 1-(4-(3-(6-(1H-pyrazol-1-yl)pyrid-3-yl)-1-(2,6-difluorobenzyl)-5-((dimethyl amino)methyl)-2,4-dioxo-1,2,3,4-tetrahydrothieno[2,3-d]pyrimidin-6-yl)phenyl)-3-(2,2-difluoroethyl)urea

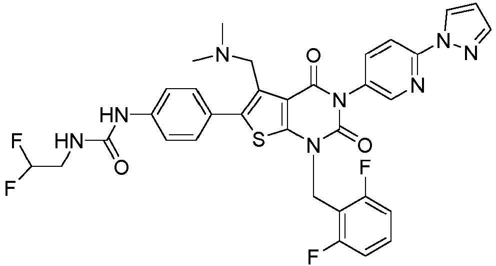

The title compound in this step was prepared according to the method described in **Example 1, Step 10,** *i.e.* 3-(6-(1*H*-pyrazol-1-yl)pyrid-3-yl)-1-(2,6-difluorobenzyl)-5-((dimethyl amino)methyl)-6-(4-aminophenyl)thieno[2,3-*d*]pyrimidine-2,4(1*H*,3*H*)-dione (0.5 g, 0.85 mmol), phenyl *N*-(2,2-difluoroethyl)carbamate (0.857 g, 4.26 mmol), triethylamine (0.59 mL, 4.26 mmol) and 4-dimethylaminopyridine (10.5 mg, 0.085 mmol) in *N*,*N*-dimethylformamide (10 mL) reacted together and the reaction mixture was purified by silica gel chromatography eluted with (dichloromethane / methanol (v/v) = 20/1) to give the title compound as a white solid (0.355 g, 60.1%).
MS (ESI, pos. ion) *m*/*z:* 693.2 [M+H]⁺;
1H NMR (400 MHz, DMSO-*d₆*) *δ* (ppm) 8.92 (s, 1H), 8.66 (d, *J* = 2.3 Hz, 1H), 8.42 (d, *J =* 2.1 Hz, 1H), 8.07 (d, *J* = 8.7 Hz, 1H), 7.97 (dd, *J* = 8.7, 2.4 Hz, 1H), 7.88 (s, 1H), 7.65 - 7.36 (m, 5H), 7.14 (t, *J =* 8.2 Hz, 2H), 6.62 (s, 1H), 6.57 (t, *J* = 5.9 Hz, 1H), 6.07 (tt, *J =* 56.1, 3.6 Hz, 1H), 5.31 (s, 2H), 3.70 - 3.45 (m, 4H), 2.06 (s, 6H).

### Example 9 1-(4-(3-(5-(1H-pyrazol-1-yl)pyrid-2-yl)-1-(2,6-difluorobenzyl)-5-((dimethyl amino)methyl)-2,4-dioxo-1,2,3,4-tetrahydrothieno[2,3-d]pyrimidin-6-yl)phenyl)-3-ethylurea

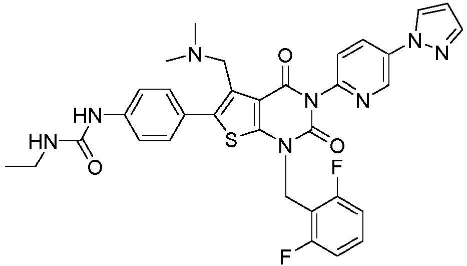

The title compound in this step was prepared according to the method described in **Example 1, Step 10,** *i.e.* 3-(5-(1*H*-pyrazol-1-yl)pyrid-2-yl)-1-(2,6-difluorobenzyl)-5-((dimethyl amino)methyl)-6-(4-aminophenyl)thieno[2,3-*d*]pyrimidine-2,4(1*H*,3*H*)-dione (0.5 g, 0.85 mmol), phenyl *N*-ethylcarbamate (0.7 g, 4.26 mmol), triethylamine (0.59 mL, 4.26 mmol) and 4-dimethylaminopyridine (10.5 mg, 0.085 mmol) in N,N-dimethylformamide (10 mL) reacted together and the reaction mixture was purified by silica gel chromatography eluted with (dichloromethane / methanol (v/v) = 20/1) to give the title compound as a white solid (0.22 g, 39.4%).
MS (ESI, pos. ion) *m*/*z:* 657.2 [M+H]⁺;
¹H NMR (400 MHz, CDCl₃) *δ* (ppm) 8.24 (dd, *J =* 8.5, 2.7 Hz, 1H), 7.98 (d, *J =* 2.3 Hz, 1H), 7.88 (s, 1H), 7.79 (d, *J* = 1.3 Hz, 1H), 7.53 (d, *J* = 8.5 Hz, 1H), 7.46 (d, *J* = 8.5 Hz, 2H), 7.32 (d, *J* = 8.3 Hz, 2H), 7.28 (s, 1H), 6.92 (t, *J =* 8.1 Hz, 2H), 6.54 (d, *J =* 1.9 Hz, 1H), 5.33 (d, *J =* 10.8 Hz, 2H), 3.75 (s, 2H), 3.26 (dt, *J* = 13.9, 7.0 Hz, 2H), 2.17 (s, 6H), 1.13 (t, *J* = 7.2 Hz, 3H).

### Example 10 1-(4-(3-(5-(1H-pyrazol-1-yl)pyrid-2-yl)-1-(2,6-difluorobenzyl)-5-((dimethyl amino)methyl)-2,4-dioxo-1,2,3,4-tetrahydrothieno[2,3-d]pyrimidin-6-yl)phenyl)-3-cyclopropylurea

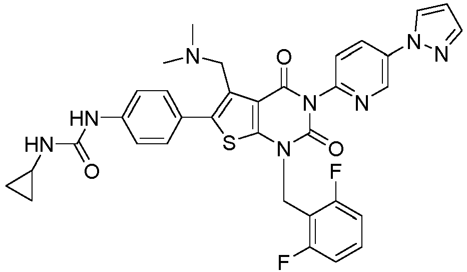

The title compound in this step was prepared according to the method described in **Example 1, Step 10,** *i.e.* 3-(5-(1*H*-pyrazol-1-yl)pyrid-2-yl)-1-(2,6-difluorobenzyl)-5-((dimethyl amino)methyl)-6-(4-aminophenyl)thieno[2,3-*d*]pyrimidine-2,4(1*H*,3*H*)-dione (0.4 g, 0.68 mmol), phenyl *N*-cyclopropylcarbamate (0.5 g, 2.8 mmol), triethylamine (0.4 mL, 3.0 mmol) in *N*,*N-*dimethylformamide (10 mL) reacted together and the reaction mixture was purified by silica gel chromatography eluted with (dichloromethane / methanol (v/v) = 20/1) to give the title compound as a white solid (0.13 g, 28.5%).
MS (ESI, pos. ion) *m*/*z:* 669.3 [M+H]⁺;
¹H NMR (400 MHz, CDCl₃) *δ* (ppm) 8.99 (d, *J =* 2.0 Hz, 1H), 8.21 (dd, *J =* 8.4, 2.3 Hz, 1H), 7.96 (d, *J* = 1.9 Hz, 1H), 7.82 (s, 1H), 7.77 (s, 1H), 7.50 (t, *J =* 8.1 Hz, 3H), 7.39 - 7.30 (m, 3H), 6.92 (t, *J* = 8.0 Hz, 2H), 6.52 (s, 1H), 5.32 (d, *J =* 10.0 Hz, 2H), 3.76 (s, 2H), 2.58 (s, 1H), 2.18 (s, 6H), 0.75 (d, *J =* 5.6 Hz, 2H), 0.54 (brs, 2H).

### Example 11 1-(4-(3-(5-(1H-pyrazol-1-yl)pyrid-2-yl)-1-(2,6-difluorobenzyl)-5-((dimethyl amino)methyl)-2,4-dioxo-1,2,3,4-tetrahydrothieno[2,3-d]pyrimidin-6-yl)phenyl)-3-(2,2-difluoroethyl)urea

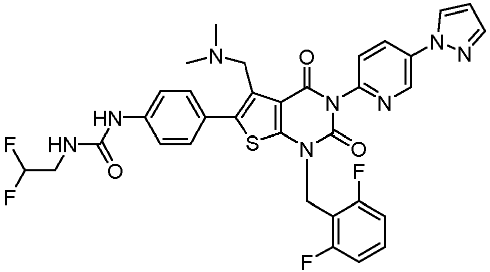

The title compound in this step was prepared according to the method described in **Example 1, Step 10,** *i.e.* 3-(5-(1*H*-pyrazol-1-yl)pyrid-2-yl)-1-(2,6-difluorobenzyl)-5-((dimethyl amino)methyl)-6-(4-aminophenyl)thieno[2,3-*d*]pyrimidine-2,4(1*H*,3*H*)-dione (0.5 g, 0.85 mmol), phenyl N-(2,2-difluoroethyl)carbamate (0.7 g, 3.5 mmol), triethylamine (0.4 mL, 3.0 mmol) in N,N-dimethylformamide (10 mL) reacted together and the reaction mixture was purified by silica gel chromatography eluted with (dichloromethane / methanol (v/v) = 20/1) to give the title compound as a white solid (0.21 g, 35.5%).
MS (ESI, pos. ion) *m*/*z:* 693.2 [M+H]⁺;
¹H NMR (400 MHz, CDCl₃) *δ* (ppm) 8.96 (d, *J =* 2.3 Hz, 1H), 8.74 (s, 1H), 8.19 (dd, *J =* 8.5, 2.5 Hz, 1H), 7.94 (d, *J* = 2.2 Hz, 1H), 7.75 (s, 1H), 7.53 (d, *J =* 8.6 Hz, 1H), 7.49 (d, *J =* 8.3 Hz, 2H), 7.29 (t, *J* = 6.5 Hz, 1H), 7.23 (d, *J =* 8.3 Hz, 2H), 6.90 (t, *J =* 8.1 Hz, 2H), 6.85 (s, 1H), 6.50 (d, *J* = 1.8 Hz, 1H), 5.82 (t, *J* = 56.5 Hz, 1H), 5.31 (s, 2H), 3.81 (s, 2H), 3.48 (s, 2H), 2.30 - 2.10 (m, 6H).

### Example 12 1-(4-(3-(6-(1H-pyrazol-1-yl)pyridazin-3-yl)-1-(2,6-difluorobenzyl)-5-((dimethyl amino)methyl)-2,4-dioxo-1,2,3,4-tetrahydrothieno[2,3-d]pyrimidin-6-yl)phenyl)-3-methoxyurea

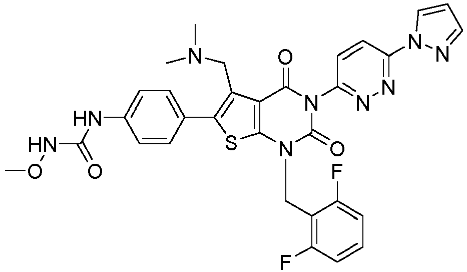

### Step 1) Synthesis of ethyl 2-(3-(6-(1H-pyrazol-1-yl)pyridazin-3-yl)ureido)-4-methyl-5-(4-nitro phenyl)thiophene-3-carboxylate

The title compound in this step was prepared according to the method described in **Example 1, Step 4,** *i.e.* ethyl 4-methyl-5-(4-nitrophenyl)-2-((phenoxycarbonyl)amino) thiophene-3-carboxylate (2.54 g, 5.96 mmol), 6-pyrazolyl-1-pyridazinamine (0.8 g, 4.97 mmol) and triethylamine (2.09 ml, 14.9 mmol) in *N*,*N*-dimethylformamide (20 mL) reacted together to give the title compound as a light yellow solid (1.255 g, 41.6%).
MS (ESI, neg. ion) *m*/*z:* 491.9 [M-H]⁻.

### Step 2) Synthesis of 3-(6-(1H-pyrazol-1-yl)pyridazin-3-yl)-5-methyl-6-(4-nitrophenyl) thieno[2,3-d]pyrimidine-2,4(1H,3H)-dione

The title compound in this step was prepared according to the method described in **Example 1, Step 5,** *i.e.* ethyl 2-(3-(6-(1*H*-pyrazol-1-yl)pyridazin-3-yl)ureido)-4-methyl-5- (4-nitrophenyl)thiophene-3-carboxylate (1.15 g, 2.33 mmol) and sodium methoxide (0.378 g, 6.99 mmol) in ethanol (20 mL) reacted together to give the title compound as a red solid (0.855 g, 82%).
MS (ESI, neg. ion) *m*/*z:* 446.2 [M-H]⁻.

### Step 3) Synthesis of 3-(6-(1H-pyrazol-1-yl)pyridazin-3-yl)-1-(2,6-difluorobenzyl)-5-methyl-6-(4-nitrophenyl)thieno[2,3-d]pyrimidine-2,4(1H,3H)-dione

The title compound in this step was prepared according to the method described in **Example 1, Step 6,** *i.e.* 3-(6-(1*H*-pyrazol-1-yl)pyridazin-3-yl)-5-methyl-6-(4-nitrophenyl) thieno[2,3-*d*]pyrimidine-2,4(1*H*,3*H*)-dione (1.35 g, 3.02 mmol), potassium iodide (0.75 g, 4.52 mmol), potassium carbonate (0.63 g, 4.53 mmol) and 2,6-difluorobenzyl choride (0.736 g, 4.53 mmol) in *N*,*N*-dimethylformamide (20 mL) reacted together to give the title compound as a light yellow solid (1.15 g, 66.5%).
MS (ESI, pos. ion) *m*/*z:* 574.1 [M+H]⁺;
¹H NMR (400 MHz, CDCl₃) *δ* (ppm) 8.82 (d, *J* = 2.6 Hz, 1H), 8.40 (d, *J* = 9.1 Hz, 1H), 8.30 (d, *J* = 8.7 Hz, 2H), 7.83 (d, *J =* 0.9 Hz, 1H), 7.66 (d, *J* = 9.1 Hz, 1H), 7.57 (d, *J =* 8.7 Hz, 2H), 7.39 - 7.28 (m, 1H), 6.95 (t, *J* = 8.1 Hz, 2H), 6.61 - 6.52 (m, 1H), 2.56 (s, 3H).

### Step 4) Synthesis of 3-(6-(1H-pyrazol-1-yl)pyridazin-3-yl)-5-(bromomethyl)-1-(2,6-difluoro benzyl)-6-(4-nitrophenyl)thieno[2,3-d]pyrimidine-2,4(1H,3H)-dione

The title compound in this step was prepared according to the method described in **Example 1, Step** 7, *i.e.* 3-(6-(1*H*-pyrazol-1-yl)pyridazin-3-yl)-1-(2,6-difluorobenzyl)-5-methyl-6-(4-nitrophenyl)thieno[2,3-*d*]pyrimidine-2,4(1*H*,3*H*)-dione (1.1 g, 1.92 mmol), azobisisobutyronitrile (81 mg, 0.48 mmol) and *N*-bromosuccinimide (0.45 g, 2.49 mmol) in chlorobenzene (30 mL) reacted together to give the title compound as a light yellow solid (1.2 g, 95.9%).

### Step 5) Synthesis of 3-(6-(1H-pyrazol-1-yl)pyridazin-3-yl)-1-(2,6-difluorobenzyl)-5-((dimethyl amino)methyl)-6-(4-nitrophenyl)thieno[2,3-d]pyrimidine-2,4(1H,3H)-dione

The title compound in this step was prepared according to the method described in **Example 1, Step 8,** *i.e.* 3-(6-(1*H*-pyrazol-1-yl)pyridazin-3-yl)-5-(bromomethyl)-1-(2,6-difluoro benzyl)-6-(4-nitrophenyl)thieno[2,3-*d*]pyrimidine-2,4(1*H*,3*H*)-dione (0.55 g, 0.84 mmol), dimethylamine hydrochloride (0.417 g, 5.06 mmol) and triethylamine (0.709 mL, 5.06 mmol) in *N*,*N*-dimethylformamide (10 mL) reacted together and the reaction mixture was purified by silica gel chromatography eluted with (dichloromethane / methanol (v/v) = 40/1) to give the title compound as a light yellow solid (0.534 g, 86.6%).
MS (ESI, pos. ion) *m*/*z:* 617.1 [M+H]⁺;
¹H NMR (400 MHz, CDCl₃) *δ* (ppm) 8.82 (d, *J* = 2.5 Hz, 1H), 8.39 (d, *J* = 9.1 Hz, 1H), 8.29 (d, *J =* 8.7 Hz, 2H), 7.88 (d, *J =* 8.7 Hz, 2H), 7.83 (s, 1H), 7.68 (d, *J* = 9.1 Hz, 1H), 7.35 (dt, *J =* 14.5, 7.3 Hz, 1H), 6.96 (t, *J =* 8.1 Hz, 2H), 6.58 - 6.52 (m, 1H), 5.40 (s, 2H), 3.72 (s, 2H), 2.19 (s, 6H).

### Step 6) Synthesis of 3-(6-(1H-pyrazol-1-yl)pyridazin-3-yl)-6-(4-aminophenyl)-1-(2,6-difluoro benzyl)-5-((dimethylamino)methyl)thieno[2,3-d]pyrimidine-2,4(1H,3H)-dione

The title compound in this step was prepared according to the method described in **Example 1, Step 9,** *i.e.* 3-(6-(1*H*-pyrazol-1-yl)pyridazin-3-yl)-1-(2,6-difluorobenzyl)-5-((dimethylamino)methyl)-6-(4-nitrophenyl)thieno[2,3-*d*]pyrimidine-2,4(1*H*,3*H*)-dione (0.28 g, 0.454 mmol), Pd/C (84 mg) and hydrogen chloride-ethyl acetate solution (1.5 mL, 2 M) in methanol (6 mL) reacted together and the reaction mixture was purified by silica gel chromatography eluted with (dichloromethane / methanol (v/v) = 30/1) to give the title compound as a light yellow solid (0.255 g, 95.7%).
MS (ESI, pos. ion) *m*/*z:* 587.0 [M+H]⁺;
¹H NMR (400 MHz, CDCl₃) *δ* (ppm) 8.81 (d, *J* = 2.5 Hz, 1H), 8.38 (d, *J* = 9.1 Hz, 1H), 7.82 (s, 1H), 7.32 (dd, *J* = 15.1, 7.1 Hz, 1H), 7.24 (s, 2H), 6.92 (t, *J* = 8.1 Hz, 2H), 6.72 (d, *J* = 8.3 Hz, 2H), 6.54 (d, *J* = 1.6 Hz, 1H), 5.35 (s, 2H), 3.88 (s, 2H), 2.30 (s, 6H).

### Step 7) Synthesis of 1-(4-(3-(6-(1H-pyrazol-1-yl)pyridaz-3-yl)-1-(2,6-difluorobenzyl)-5-((dimethylamino)methyl)-2,4-dioxo-1,2,3,4-tetrahydrothieno[2,3-d]pyrimidin-6-yl)phenyl)-3-methoxyurea

The title compound in this step was prepared according to the method described in **Example 1, Step 10,** *i.e.* 3-(6-(1*H*-pyrazol-1-yl)pyridazin-3-yl)-6-(4-aminophenyl)-1-(2,6-difluorobenzyl)-5-((dimethylamino)methyl)thieno[2,3-*d*]pyrimidine-2,4(1*H*,3*H*)-dione (0.33 g, 0.563 mmol), phenyl *N*-methoxycarbamate (0.47 g, 2.81 mmol), triethylamine (0.39 mL, 2.81 mmol) and 4-dimethylaminopyridine (7 mg, 0.06 mmol) in *N*,*N*-dimethylformamide (6 mL) reacted together and the reaction mixture was purified by silica gel chromatography eluted with (dichloromethane / methanol (v/v) = 20/1) to give the title compound as a white solid (0.285 g, 76.8%).
MS (ESI, pos. ion) *m*/*z:* 660.2 [M+H]⁺;
¹H NMR (400 MHz, CDCl₃) *δ* (ppm) 8.81 (d, *J* = 2.4 Hz, 1H), 8.38 (d, *J* = 9.1 Hz, 1H), 7.82 (s, 1H), 7.72 (d, *J =* 9.1 Hz, 1H), 7.66 (s, 1H), 7.57 (d, *J =* 8.6 Hz, 2H), 7.49 (d, *J =* 8.5 Hz, 2H), 7.36 - 7.30 (m, 1H), 7.28 (s, 1H), 6.93 (t, *J* = 8.1 Hz, 2H), 6.57 - 6.51 (m, 1H), 5.37 (s, 2H), 3.82 (s, 3H), 3.72 (s, 2H), 2.11 (s, 6H).

### Biological Assay

### Example A: Antagonism of the compound of the present invention on humanized GnRH receptor

### Experimental purposes

The antagonism of the compound on humanized GnRH receptor transfected in RBL-1 cells (Rat Basophilic Leukemia) was evaluated by a fluorescence method for detecting the cytoplasmic calcium flow.

### Experimental procedure

The cells were suspended in HBSS buffer (Invitrogen, USA), 20 mM HEPES buffer (Invitrogen, USA) was added, and then the cells were added at an average density of 1.186 × 10⁴ cells / well into a microwell reaction plate. Fluorescent probe (Fluo8 Direct, AAT Bioquest) was mixed with HBSS buffer (adding 20 mM HEPES buffer, pH = 7.4), and the mixture was added into each well and the cells were incubated at 30 °C for 60 min. Then the reaction plate was placed in a microplate reader (FlipR Tetra, Molecular Device) for testing, the test compound or HBSS buffer (blank control) was added, after 5 min, 8 nM LH-RH was added, and the changes of the fluorescence intensity which is proportional to intracellular calcium ion concentration were measured. The fluorescence intensity of the blank control group without adding the compound was taken as 100% (inhibition rate was 0%), and the inhibition rate of the compound was calculated. The IC₅₀ value was calculated by measuring the inhibition rate of the compound at different concentrations. The results were as shown in Table A:

**Table A Antagonism of the compound of the present invention on humanized GnRH receptor**

| **Example No.** | **IC₅₀ (nM)** |
|---|---|
| Example 2 | 3.003 |
| Example 3 | 1.824 |
| Example 4 | 2.216 |
| Example 5 | 4.102 |
| Example 12 | 3.128 |

The results indicate that the compound of the present invention has a good antagonistic action on humanized GnRH receptor.

### Example B: Pharmacokinetic evaluation after administering a certain amount of the compound of the invention by intravenous or gavage to rats or dogs

The pharmacokinetics of the compound of the invention in rats and / or dogs were evaluated herein, the animal information was listed in Table B.

**Table B Information of the animal subject of the invention**

| Germline | Grade | Sex | Weight | Age | Source |
|---|---|---|---|---|---|
| SD rats | SPF | male | 170-250 g | 6-9 weeks | HUNAN SJA LABORATORY ANIMAL CO.,LTD |
| Beagle dogs | Conventional | male | 8-12 kg | 6-12 months | Beijing Marshall Biotechnology Co., Ltd. |

### Test method

The compound of the present invention was administered to the animal subjects in the form of 10% DMSO + 10% Kolliphor HS15 + 78% Saline + 2% (2% HCl) solution or 78% Saline + 2% (2% HCl) + 20% PEG400 solution. Before administration, the animals were fasted for 12 hours but water was freely allowed. In the IV group, the dose was 1 mg / kg (Rats) or 0.5 mg / kg (dogs), blood samples were taken from vein at the following time points after administration (blood volume was about 0.2 mL): 0.083, 0.25, 0.5, 1.0, 2.0, 4.0, 6.0, 8.0 and 24 h (dogs) or 0.083, 0.25, 0.5, 1.0, 2.0, 5.0, 7.0 and 24 h (Rats), EDTA-K₂ was pre-added to the blood vessels as an anticoagulant. In the gavage group, the dose was 5 mg / kg (Rats) or 2.5 mg / kg (dogs), blood samples were taken from vein at the following time points after administration (blood volume was about 0.2 mL): 0.25, 0.5, 1.0, 2.0, 4.0, 6.0, 8.0 and 24 h (dogs) or 0.25, 0.5, 1.0, 2.0, 5.0, 7.0 and 24 h (Rats), EDTA-K₂ was pre-added to the blood vessels as an anticoagulant. The blood samples were centrifuged at 12,000 for 2 minutes. The plasma was collected and kept at -20 °C or -70 °C.

The above collected plasma samples were processed (after thawing of frozen plasma at room temperature, the thawed plasma was vortexed for 15 s; 10-20 µL of plasma was taken and 120-150 µL of acetonitrile solution containing internal standard was added to the plasma; the mixture was vortexed for 5 min amd centrifuged for 5 min at 4,000 rpm; 100 µL of supernatant was taken and 120-150 µL of methanol / water (v/v = 1/1) was added to the suspernatant, and mixing), and then LC / MS / MS was used to analyze the concentration of the compound in plasma, and non-atrioventricular model was used to calculate the pharmacokinetic parameters.

The results showed that the pharmacokinetic properties of the compounds administered intravenously and intragastrically in rats, dogs were high in exposure and high in bioavailability. The compound of the invention has better drug properties and better clinical application prospect. Wherein the pharmacokinetic parameters of Examples 2 and 3 in rats were detailed in Table B1; the pharmacokinetic parameters of Examples 2 and 3 in dogs were detailed in Table B2.

**Table B1 Pharmacokinetic parameters of Examples 2 and 3 in rats**

| Example No. | Example 2 | | Example 3 | |
|---|---|---|---|---|
| Groups | i.v group | i.g group | i.v group | i.g group |
| Dose (mg/kg) | 1 | 5 | 1 | 5 |
| Tₘₐₓ(h) | 0.083 | 0.667 | 0.083 | 2.33 |
| Cₘₐₓ(ng/ml) | 352 | 177 | 151 | 164 |
| AUCₗₐₛₜ(h*ng/ml) | 261 | 519 | 179 | 1710 |
| AUC_{INF}(h*ng/ml) | 266 | 529 | 184 | 1750 |
| MRT_{INF}(h) | 1.22 | 2.48 | 4.58 | 6.4 |
| T_{1/2}(h) | 1.33 | 1.91 | 2.13 | 4.43 |
| F(%) | -- | 39.8 | -- | 190.6 |
| Cl(ml/min/kg) | 62.7 | -- | 90.7 | -- |

**Table B2 Pharmacokinetic parameters of Examples 2 and 3 in dogs**

| Example No. | Example 2 | | Example 3 | |
|---|---|---|---|---|
| Groups | i.v group | i.g group | i.v group | i.g group |
| Dose(mg/kg) | 0.5 | 2.5 | 0.5 | 2.5 |
| Tₘₐₓ(h) | 0.083 | 1.33 | 0.083 | 1.67 |
| Cₘₐₓ(ng/ml) | 636 | 720 | 493 | 671 |
| AUCₗₐₛₜ(h*ng/ml) | 630 | 4130 | 1330 | 9490 |
| AUC_{INF}(h*ng/ml) | 645 | 4290 | 1530 | 11800 |
| MRT_{INF}(h) | 1.92 | 5.59 | 3.92 | 13.2 |
| T_{1/2}(h) | 1.43 | 4.41 | 2.85 | 9.44 |
| F(%) | -- | 133 | -- | 140.3 |
| Cl(ml/min/kg) | 12.9 | -- | 5.46 | -- |

The results indicate that the compound of the invention has good pharmacokinetic properties in rats and / or dogs, which shows that the compound of the invention has better drug properties and better clinical application prospect.

Reference throughout this specification to "an embodiment", "one embodiment", "some embodiments", "an example," "a specific example," or "some examples," means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example, "in an example," "in a specific example," or "in some examples," in various places throughout this specification are not necessarily referring to the same embodiment or example of the present disclosure. Furthermore, the particular features, structures, materials, or characteristics may be combined in any suitable manner in one or more embodiments or examples.

## Claims

1. A compound having Formula (I) or a stereoisomer, a geometric isomer, a tautomer, an *N-*oxide, a hydrate, a solvate, or a pharmaceutically acceptable salt thereof, 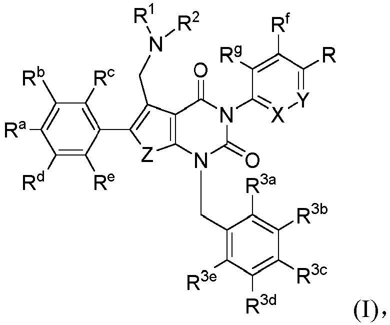 wherein
X is CR^{x} or N; Y is CR^{y} or N;
when Y is N, R is 5-10 membered heteroaryl;
when Y is CR^{y}, R is 5-6 membered heteroaryl, wherein R is optionally substituted with 1, 2, 3 or 4 R⁰; R^{y} is H, D, F, Cl, Br, I, -CN, -NO₂, -NH₂, -OH, -SH, -COOH, -C(=O)NH₂, - C(=O)NHCH₃, -C(=O)N(CH₃)₂, -C(=O)-(C₁-C₆ alkyl), -C(=O)-(C₁-C₆ alkoxy), C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆₋₁₀ aryl or 5-10 membered heteroaryl; or
R is H, D, F, Cl, Br, I, -CN, -NO₂, -NH₂, -OH, -SH, -COOH, -C(=O)NH₂, -C(=O)NHCH₃, - C(=O)N(CH₃)₂, -C(=O)-(C₁-C₆ alkyl), -C(=O)-(C₁-C₆ alkoxy), C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆₋₁₀ aryl or 5-10 membered heteroaryl; R^{y} is 5-6 membered heteroaryl, wherein R^{y} is optionally substituted with 1, 2, 3 or 4 R⁰;
Z is NRⁿ, S or O;
Rⁿ is H, D, C₁₋₆ alkyl, C₂-C₆ alkenyl, C₂-C₆ alkynyl or C₁-C₆ haloalkyl;
each R^{b}, R^{c}, R^{d}, R^{e}, R^{f}, R^{g} and R^{x} is independently H, D, F, Cl, Br, I, -CN, -NO₂, -NH₂, -OH, -SH, -COOH, -C(=O)NH₂, -C(=O)NHCH₃, -C(=O)N(CH₃)₂, -C(=O)-(C₁-C₆ alkyl), -C(=O)-(C₁-C₆ alkoxy), C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆₋₁₀ aryl or 5-10 membered heteroaryl;
R^{a} is -CN, -NO₂, -OR⁴, -NR⁵R⁶, -NR⁷C(=O)R⁸, -NR⁷C(=O)NR⁵R⁶, -NR⁷S(=O)ₜR⁸, - NR⁷S(=O)ₜNR⁵R⁶, -C(=O)R⁸, -C(=O)NR⁵R⁶, -S(=O)ₜNR⁵R⁶ or -S(=O)ₜR⁸; wherein t is 0, 1 or 2;
each R⁰ is independently D, F, Cl, Br, I, -CN, -NO₂, -NH₂, -OH, -SH, -COOH, -C(=O)NH₂, - C(=O)NHCH₃, -C(=O)N(CH₃)₂, -C(=O)-(C₁-C₆ alkyl), -C(=O)-(C₁-C₆ alkoxy), C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino or hydroxy-substituted C₁-C₆ alkyl;
each R¹ and R² is independently H, D, -OH, C₁-C₆ alkyl, C₂-C₆ alkenyl, C₂-C₆ alkynyl, C₁-C₆ haloalkyl, C₁-C₆ alkoxy, C₁-C₆ haloalkoxy, C₃₋₆ cycloalkyl, 3-8 membered heterocyclyl, C₆₋₁₀ aryl, 5-10 membered heteroaryl, C₃₋₆ cycloalkyl-C₁₋₆ alkylene, (3-8 membered heterocyclyl)-C₁₋₆ alkylene, C₆₋₁₀ aryl-C₁₋₆ alkylene or (5-10 membered heteroaryl)-C₁₋₆ alkylene; wherein each R¹ and R² is independently and optionally substituted with 1, 2, 3 or 4 R^{w};
each R^{3a}, R^{3b}, R^{3c}, R^{3d} and R^{3e} is independently H, D, F, Cl, Br, I, -CN, -NO₂, -NH₂, -OH, - SH, -COOH, -C(=O)NH₂, -C(=O)NHCH₃, -C(=O)N(CH₃)₂, -C(=O)-(C₁-C₆ alkyl), -C(=O)-(C₁-C₆ alkoxy), -NHS(=O)₂-(C₁-C₆ alkyl), -N(C₁₋₆ alkyl)S(=O)₂-(C₁-C₆ alkyl), -S(=O)₂-(C₁-C₆ alkyl), C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆₋₁₀ aryl or 5-10 membered heteroaryl;
R⁴ is H, D, C₁-C₆ alkyl, C₂-C₆ alkenyl, C₂-C₆ alkynyl, C₁-C₆ haloalkyl, C₃₋₆ cycloalkyl, 3-8 membered heterocyclyl, C₆₋₁₀ aryl or 5-10 membered heteroaryl; wherein R⁴ is optionally substituted with 1, 2, 3 or 4 R^{w};
each R⁵, R⁶ and R⁷ is independently H, D, C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆₋₁₀ aryl or 5-10 membered heteroaryl; wherein each R⁵, R⁶ and R⁷ is independently and optionally substituted with 1, 2, 3 or 4 R^{w};
R⁸ is -OH, C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆₋₁₀ aryl or 5-10 membered heteroaryl; wherein R⁸ is optionally substituted with 1, 2, 3 or 4 R^{w};
each R^{w} is independently =O, D, F, Cl, Br, I, -CN, -NO₂, -NH₂, -OH, -SH, -COOH, - C(=O)NH₂, -C(=O)NHCH₃, -C(=O)N(CH₃)₂, -C(=O)-(C₁-C₆ alkyl), -C(=O)-(C₁-C₆ alkoxy), - NHS(=O)₂-(C₁-C₆ alkyl), -N(C₁₋₆ alkyl)S(=O)₂-(C₁-C₆ alkyl), -S(=O)₂-(C₁-C₆ alkyl), C₁-C₆ alkyl, C₂-C₆ alkenyl, C₂-C₆ alkynyl, C₁-C₆ haloalkyl, C₁-C₆ alkoxy, C₁-C₆ haloalkoxy, C₁-C₆ alkylthio, C₁-C₆ alkylamino, hydroxy-substituted C₁-C₆ alkyl, C₃₋₆ cycloalkyl, 3-8 membered heterocyclyl, C₆₋₁₀ aryl or 5-10 membered heteroaryl.

2. The compound of claim 1, wherein each R¹ and R² is independently H, D, -OH, C₁-C₄ alkyl, C₂-C₄ alkenyl, C₂-C₄ alkynyl, C₁-C₄ haloalkyl, C₁-C₄ alkoxy, C₁-C₄ haloalkoxy, C₃₋₆ cycloalkyl, 3-6 membered heterocyclyl, C₆₋₁₀ aryl, 5-6 membered heteroaryl, C₃₋₆ cycloalkyl-C₁₋₄ alkylene, (3-6 membered heterocyclyl)-C₁₋₄ alkylene, C₆₋₁₀ aryl-C₁₋₄ alkylene or (5-6 membered heteroaryl)-C₁₋₄ alkylene; wherein each R¹ and R² is independently and optionally substituted with 1, 2, 3 or 4 R^{w};
each R^{3a}, R^{3b}, R^{3c}, R^{3d} and R^{3e} is independently H, D, F, Cl, Br, I, -CN, -NO₂, -NH₂, -OH, - SH, -COOH, -C(=O)NH₂, -C(=O)NHCH₃, -C(=O)N(CH₃)₂, -C(=O)-(C₁-C₄ alkyl), -C(=O)-(C₁-C₄ alkoxy), -S(=O)₂-(C₁-C₄ alkyl), C₁-C₄ alkyl, C₂-C₄ alkenyl, C₂-C₄ alkynyl, C₁-C₄ haloalkyl, C₁-C₄ alkoxy, C₁-C₄ haloalkoxy, C₁-C₄ alkylthio, C₁-C₄ alkylamino, hydroxy-substituted C₁-C₄ alkyl;
each R⁵, R⁶ and R⁷ is independently H, D, C₁-C₄ alkyl, C₂-C₄ alkenyl, C₂-C₄ alkynyl, C₁-C₄ haloalkyl, C₁-C₄ alkoxy, C₁-C₄ haloalkoxy, C₁-C₄ alkylthio, C₁-C₄ alkylamino, hydroxy-substituted C₁-C₄ alkyl, C₃₋₆ cycloalkyl, 3-6 membered heterocyclyl, C₆₋₁₀ aryl or 5-6 membered heteroaryl; wherein each R⁵, R⁶ and R⁷ is independently and optionally substituted with 1, 2, 3 or 4 R^{w}.

3. The compound of claim 1 or 2, wherein each R¹ and R² is independently H, D, -OH, methyl, ethyl, n-propyl, i-propyl, n-butyl, t-butyl, 2-methylpropyl, 1-methylpropyl, vinyl, propenyl, allyl, ethynyl, propynyl, trifluoromethyl, difluoromethyl, methoxy, ethoxy, isopropoxy, trifluoromethoxy, cyclopropyl, cyclopentyl, cyclohexyl, oxiranyl, pyrrolidyl, piperidyl, morpholinyl, piperazinyl, phenyl, naphthyl, pyrrolyl, pyridyl, pyrimidinyl, pyrazolyl, triazolyl, thiazolyl, imidazolyl, tetrazolyl, C₃₋₆ cycloalkylmethylene, C₃₋₆ cycloalkylethylene, C₃₋₆ cycloalkylpropylene, (3-6 membered heterocyclyl)methylene, (3-6 membered heterocyclyl)ethylene, phenylmethylene, phenylethylene, phenylpropylene, pyridylmethylene, pyrimidinylmethylene, pyrrolylmethylene, pyrazolylmethylene, triazolylmethylene or tetrazolylmethylene; wherein each R¹ and R² is independently and optionally substituted with 1, 2, 3 or 4 R^{w};
each R^{3a}, R^{3b}, R^{3c}, R^{3d} and R^{3e} is independently H, D, F, Cl, Br, I, -CN, -NO₂, -NH₂, -OH, - SH, -COOH, -C(=O)NH₂, -C(=O)NHCH₃, -C(=O)N(CH₃)₂, -C(=O)-CH₃, -C(=O)-OCH₃, - S(=O)₂CH₃, methyl, ethyl, *n*-propyl, *i*-propyl, *t*-butyl, vinyl, trifluoromethyl, difluoromethyl, methoxy, trifluoromethoxy, methylamino, dimethylamino or hydroxymethyl;
each R⁵, R⁶ and R⁷ is independently H, D, methyl, ethyl, *n*-propyl, *i*-propyl, *n*-butyl, *t*-butyl, 2-methylpropyl, 1-methylpropyl, vinyl, propenyl, allyl, ethynyl, propynyl, trifluoromethyl, difluoromethyl, 2,2-difluoroethyl, methoxy, ethoxy, *i*-propoxy, *n*-propoxy, *t*-butoxy, trifluoromethoxy, C₁-C₄ alkylthio, C₁-C₄ alkylamino, hydroxy-substituted C₁-C₄ alkyl, cyclopropyl, cyclopentyl, cyclohexyl, 3-6 membered heterocyclyl, C₆₋₁₀ aryl or 5-6 membered heteroaryl; wherein each R⁵, R⁶ and R⁷ is independently and optionally substituted with 1, 2, 3 or 4 R^{w}.

4. The compound of any one of claims 1 to 3, wherein when Y is N, R is pyridyl, pyrimidinyl, thienyl, furyl or 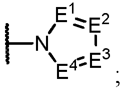
when Y is CR^{y}, R is 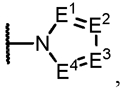
wherein R is optionally substituted with 1, 2, 3 or 4 R⁰; R^{y} is H, D, F, Cl, Br, I, -CN, -NO₂, -NH₂, -OH, -SH, -COOH, -C(=O)NH₂, -C(=O)NHCH₃, - C(=O)N(CH₃)₂, -C(=O)-(C₁-C₄ alkyl), -C(=O)-(C₁-C₄ alkoxy), C₁-C₄ alkyl, C₂-C₄ alkenyl, C₂-C₄ alkynyl, C₁-C₄ haloalkyl, C₁-C₄ alkoxy, C₁-C₄ haloalkoxy, C₁-C₄ alkylthio, C₁-C₄ alkylamino, hydroxy-substituted C₁-C₄ alkyl, C₃₋₆ cycloalkyl, 3-6 membered heterocyclyl, phenyl, naphthyl, pyridyl, pyrimidinyl, thienyl, furyl or 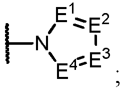 or
R is H, D, F, Cl, Br, I, -CN, -NO₂, -NH₂, -OH, -SH, -COOH, -C(=O)NH₂, -C(=O)NHCH₃, - C(=O)N(CH₃)₂, -C(=O)-(C₁-C₄ alkyl), -C(=O)-(C₁-C₄ alkoxy), C₁-C₄ alkyl, C₂-C₄ alkenyl, C₂-C₄ alkynyl, C₁-C₄ haloalkyl, C₁-C₄ alkoxy, C₁-C₄ haloalkoxy, C₁-C₄ alkylthio, C₁-C₄ alkylamino, hydroxy-substituted C₁-C₄ alkyl, C₃₋₆ cycloalkyl, 3-6 membered heterocyclyl, phenyl, naphthyl, pyridyl, pyrimidinyl, thienyl, furyl or 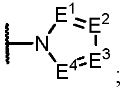 R^{y} is 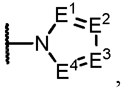 wherein R^{y} is optionally substituted with 1, 2, 3 or 4 R⁰;
wherein each E¹, E², E³ and E⁴ is independently N or CH.

5. The compound of any one of claims 1 to 4, when Y is N, R is pyridyl, pyrimidinyl, thienyl, furyl, 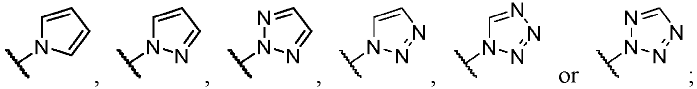
when Y is CR^{y}, R is 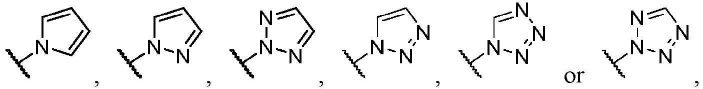 wherein R is optionally substituted with 1, 2, 3 or 4 R⁰; R^{y} is H, D, F, Cl, Br, I, -CN, -NO₂, -NH₂, -OH, -SH, -COOH, -C(=O)NH₂, -C(=O)NHCH₃, -C(=O)N(CH₃)₂, -C(=O)CH₃, -C(=O)OCH₃, methyl, ethyl, *n*-propyl, *i*-propyl, *t*-butyl, vinyl, ethynyl, propynyl, trifluoromethyl, difluoromethyl, methoxy, ethoxy, *i*-propoxy, trifluoromethoxy, methylthio, methylamino, dimethylamino, hydroxymethyl, C₃₋₆ cycloalkyl, 3-6 membered heterocyclyl, phenyl, naphthyl, pyridyl, pyrimidinyl, thienyl, furyl, 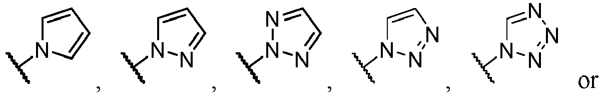 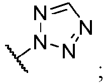 or
R is H, D, F, Cl, Br, I, -CN, -NO₂, -NH₂, -OH, -SH, -COOH, -C(=O)NH₂, -C(=O)NHCH₃, - C(=O)N(CH₃)₂, -C(=O)CH₃, -C(=O)OCH₃, methyl, ethyl, *n*-propyl, *i*-propyl, *t*-butyl, vinyl, ethynyl, propynyl, trifluoromethyl, difluoromethyl, methoxy, ethoxy, *i*-propoxy, trifluoromethoxy, methylthio, methylamino, dimethylamino, hydroxymethyl, C₃₋₆ cycloalkyl, 3-6 membered heterocyclyl, phenyl, naphthyl, pyridyl, pyrimidinyl, thienyl, furyl, 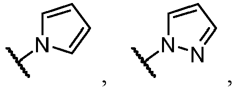 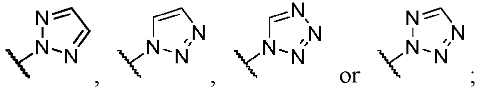 R^{y} is 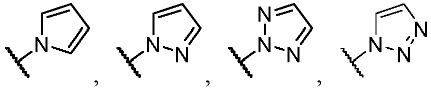 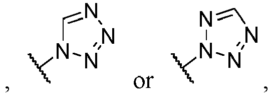 wherein R^{y} is optionally substituted with 1, 2, 3 or 4 R⁰.

6. The compound of any one of claims 1 to 5, wherein each R⁰ is independently D, F, Cl, Br, I, -CN, -NO₂, -NH₂, -OH, -SH, -COOH, -C(=O)NH₂, -C(=O)NHCH₃, -C(=O)N(CH₃)₂, -C(=O)-(C₁-C₄ alkyl), -C(=O)-(C₁-C₄ alkoxy), C₁-C₄ alkyl, C₂-C₄ alkenyl, C₂-C₄ alkynyl, C₁-C₄ haloalkyl, C₁-C₄ alkoxy, C₁-C₄ haloalkoxy, C₁-C₄ alkylthio, C₁-C₄ alkylamino or hydroxy-substituted C₁-C₄ alkyl;
each R^{w} is independently =O, D, F, Cl, Br, I, -CN, -NO₂, -NH₂, -OH, -SH, -COOH, - C(=O)NH₂, -C(=O)NHCH₃, -C(=O)N(CH₃)₂, -C(=O)-(C₁-C₄ alkyl), -C(=O)-(C₁-C₄ alkoxy), - NHS(=O)₂-(C₁-C₄ alkyl), -N(C₁₋₄ alkyl)S(=O)₂-(C₁-C₄ alkyl), -S(=O)₂-(C₁-C₄ alkyl), C₁-C₄ alkyl, C₂-C₄ alkenyl, C₂-C₄ alkynyl, C₁-C₄ haloalkyl, C₁-C₄ alkoxy, C₁-C₄ haloalkoxy, C₁-C₄ alkylthio, C₁-C₄ alkylamino, hydroxy-substituted C₁-C₄ alkyl, C₃₋₆ cycloalkyl, 3-6 membered heterocyclyl, C₆₋₁₀ aryl or 5-6 membered heteroaryl.

7. The compound of any one of claims 1 to 6, wherein each R⁰ is independently D, F, Cl, Br, I, -CN, -NO₂, -NH₂, -OH, -SH, -COOH, -C(=O)NH₂, -C(=O)NHCH₃, -C(=O)N(CH₃)₂, -C(=O)-CH₃, -C(=O)-OCH₃, methyl, ethyl, *n*-propyl, *i*-propyl, *t*-butyl, vinyl, trifluoromethyl, difluoromethyl, methoxy, trifluoromethoxy, methylamino, dimethylamino or hydroxymethyl;
each R^{w} is independently =O, D, F, Cl, Br, I, -CN, -NO₂, -NH₂, -OH, -SH, -COOH, - C(=O)NH₂, -C(=O)NHCH₃, -C(=O)N(CH₃)₂, -C(=O)CH₃, -C(=O)CH₂CH₃, -C(=O)OCH₃, - C(=O)OCH₂CH₃, -NHS(=O)₂CH₃, -N(CH₃)S(=O)₂CH₃, -NHS(=O)₂CH₂CH₃, - N(CH₂CH₃)S(=O)₂CH₂CH₃, -N(CH₂CH₃)S(=O)₂CH₃, -N(CH₃)S(=O)₂CH₂CH₃, -S(=O)₂CH₃, methyl, ethyl, *n*-propyl, *i*-propyl, *n*-butyl, *t*-butyl, 2-methylpropyl, 1-methylpropyl, vinyl, propenyl, allyl, ethynyl, propynyl, trifluoromethyl, difluoromethyl, methoxy, ethoxy, isopropoxy, *n*-propoxy, *t*-butoxy, trifluoromethoxy, C₁-C₄ alkylthio, C₁-C₄ alkylamino, hydroxy-substituted C₁-C₄ alkyl, C₃₋₆ cycloalkyl, 3-6 membered heterocyclyl, C₆₋₁₀ aryl or 5-6 membered heteroaryl.

8. The compound according to any one of claims 1 to 7 having one of the following structures or a stereoisomer, a geometric isomer, a tautomer, an N-oxide, a hydrate, a solvate, or a pharmaceutically acceptable salt thereof:

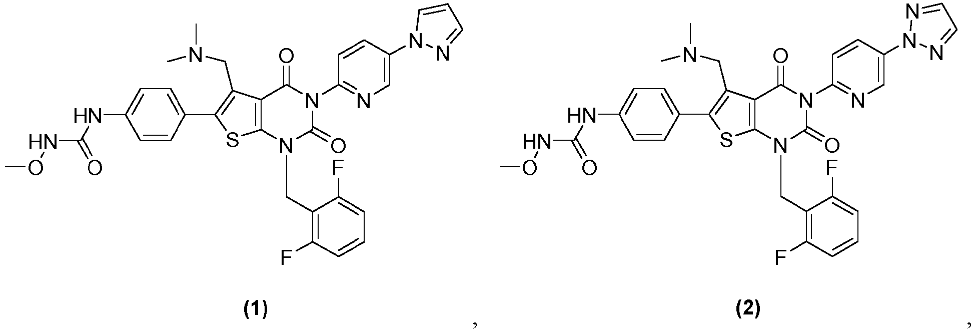

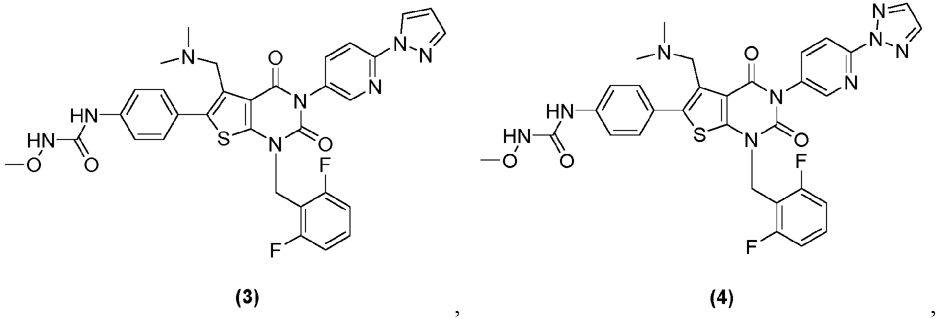

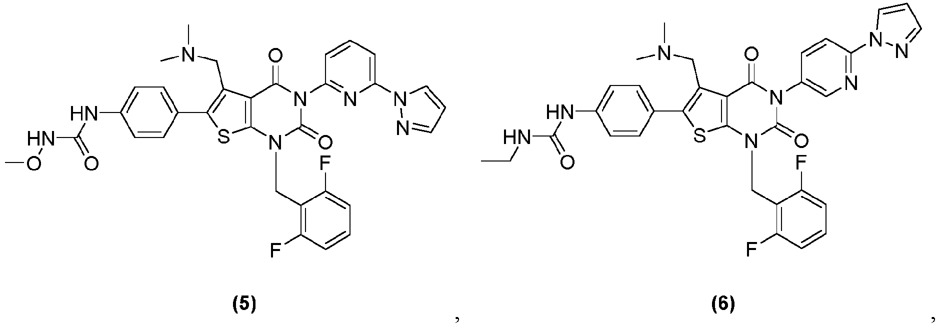

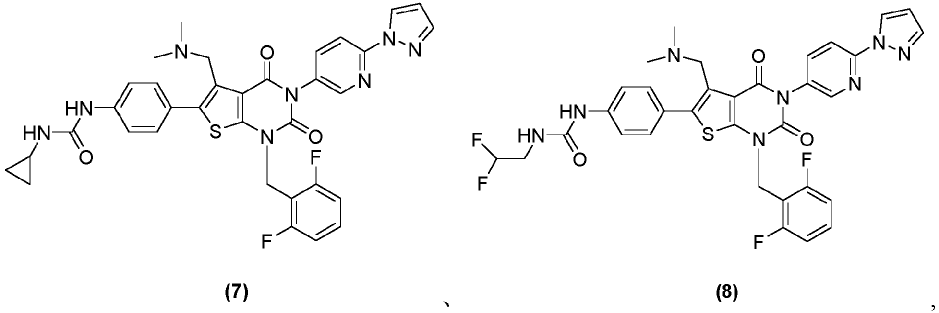

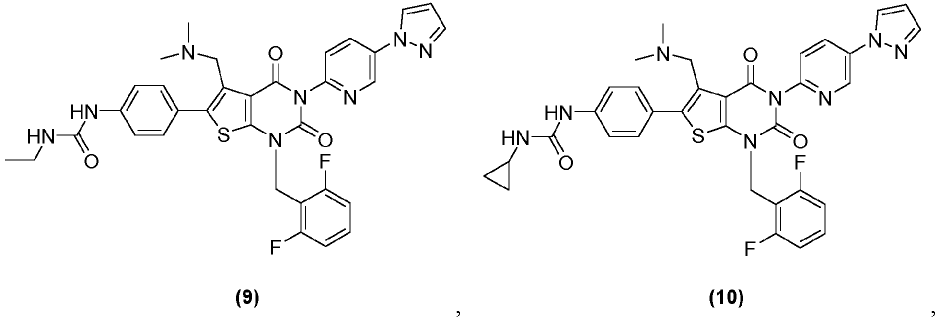

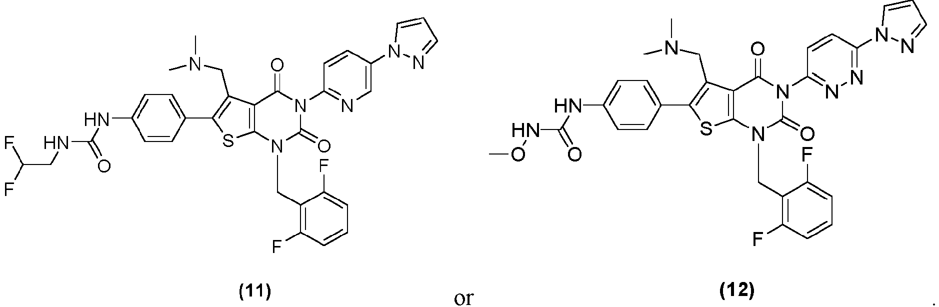

9. A pharmaceutical composition comprising the compound of any one of claims 1 to 8; and
wherein the pharmaceutical composition optionally further comprises a pharmaceutically acceptable excipient, carrier or adjuvant, or a combination thereof.

10. The compound of any one of claims 1-8 or the pharmaceutical composition of claim 9 for use in preventing or treating a sex hormone-dependent disease in a subject.

11. The compound or the pharmaceutical composition for the use of claim 10, wherein the sex hormone-dependent disease is sex hormone-dependent cancer, osseous metastasis from sex hormone-dependent cancer, prostatic hypertrophy, hysteromyoma, endometriosis, uterine fibroids, precocious puberty, amenorrhea, premenstrual syndrome, algomenorrhea, multilocular ovary syndrome, polycystic ovary syndrome, acne, alopecia, infertility or irritable bowel syndrome.

## Patentansprüche

1. Verbindung mit Formel (I) oder ein Stereoisomer, ein geometrisches Isomer, ein Tautomer, ein *N*-Oxid, ein Hydrat, ein Solvat oder ein pharmazeutisch akzeptables Salz davon, 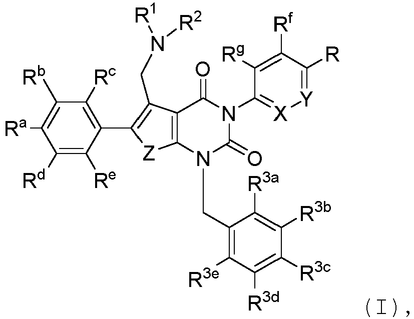 wobei
X CR^{X} oder N ist; Y CRY oder N ist;
wenn Y N ist, ist R ein 5-10-gliedriges Heteroaryl;
wenn Y CRY ist, ist R ein 5-6-gliedriges Heteroaryl, wobei R optional mit 1, 2, 3 oder 4 R⁰ substituiert ist;
RY H, D, F, Cl, Br, I, -CN, -NO₂, -NH₂, -OH, -SH, -COOH, -C(=O)NH₂, -C(=O)NHCH₃, -C(=O)N(CH₃)₂, -C(=O)-(C₁-C₆-Alkyl), -C(=O)-(C₁-C₆-Alkoxy), C₁-C₆-Alkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkylthio, C₁-C₆-Alkylamino, hydroxysubstituiertes C₁-C₆-Alkyl, C₃₋₆-Cycloalkyl, 3-8-gliedriges Heterocyclyl, C₆₋₁₀-Aryl oder 5-10-gliedriges Heteroaryl ist; oder
R H, D, F, Cl, Br, I, -CN, -NO₂, -NH₂, -OH, -SH, -COOH, -C(=O)NH₂, -C(=O)NHCH₃, -C(=O)N(CH₃)₂, -C(=O)-(C₁-C₆-Alkyl), -C(=O)-(C₁-C₆-Alkoxy), C₁-C₆-Alkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkylthio, C₁-C₆-Alkylamino, hydroxysubstituiertes C₁-C₆-Alkyl, C₃₋₆-Cycloalkyl, 3-8-gliedriges Heterocyclyl, C₆₋₁₀-Aryl oder 5-10-gliedriges Heteroaryl ist; RY ein 5-6-gliedriges Heteroaryl ist, wobei RY optional mit 1, 2, 3 oder 4 R⁰ substituiert ist;
Z NRⁿ, S oder O ist;
Rⁿ H, D, C₁₋₆-Alkyl, C₂-C₆-Alkenyl, C₂-C₆-Alkinyl oder C₁-C₆-Halogenalkyl ist;
jedes R^{b}, R^{c}, R^{d}, R^{e}, R^{f}, R^{g} und R^{x} unabhängig voneinander H, D, F, Cl, Br, I, -CN, -NO₂, -NH₂, -OH, - SH, -COOH, -C(=O)NH₂, -C(=O)NHCH₃, -C(=O)N(CH₃)₂, - C(=O)-(C₁-C₆-Alkyl), -C(=O)-(C₁-C₆-Alkoxy), C₁-C₆-Alkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkylthio, C₁-C₆-Alkylamino, hydroxysubstituiertes C₁-C₆-Alkyl, C₃₋₆-Cycloalkyl, 3-8-gliedriges Heterocyclyl, C₆₋₁₀-Aryl oder 5-10-gliedriges Heteroaryl ist;
R^{a} -CN, -NO₂, -OR⁴, -NR⁵R⁶, -NR⁷C(=O)R⁸, -NR⁷C(=O)NR⁵R⁶, -NR⁷S(=O)ₜR⁸, -NR⁷S(=O)ₜNR⁵R⁶, -C(=O)R⁸, -C(=O)NR⁵R⁶, - S(=O)ₜNR⁵R⁶ oder -S(=O)ₜR⁸ ist; wobei t 0, 1 oder 2 ist;
jedes R⁰ unabhängig voneinander D, F, Cl, Br, I, -CN, - NO₂, -NH₂, -OH, -SH, -COOH, -C(=O)NH₂, -C(=O)NHCH₃, - C(=O)N(CH₃)₂, -C(=O)-(C₁-C₆-Alkyl), -C(=O)-(C₁-C₆-Alkoxy), C₁-C₆-Alkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkylthio, C₁-C₆-Alkylamino, oder hydroxysubstituiertes C₁-C₆-Alkyl ist;
jedes R¹ und R² unabhängig voneinander H, D, -OH, C₁-C₆-Alkyl, C₂-C₆-Alkenyl, C₂-C₆-Alkinyl, C₁-C₆-Halogenalkyl, C₁-C₆-Alkoxy, C₁-C₆-Halogenalkoxy, C₃₋₆-Cycloalkyl, 3-8-gliedriges Heterocyclyl, C₆₋₁₀-Aryl, 5-10-gliedriges Heteroaryl, C₃₋₆-Cycloalkyl-C₁₋₆-Alkylen, (3-8-gliedriges Heterocyclyl)-C₁₋₆-Alkylen, C₆₋₁₀-Aryl-C₁₋₆-Alkylen oder (5-10-gliedriges Heteroaryl)-C₁₋₆-Alkylen ist; wobei jedes R¹ und R² unabhängig voneinander und optional mit 1, 2, 3 oder 4 R^{w} substituiert ist;
jedes R^{3a}, R3^{b}, R^{3c}, R^{3d} und R^{3e} unabhängig voneinander H, D, F, Cl, Br, I, -CN, -NO₂, -NH₂, -OH, -SH, -COOH, - C(=O)NH₂, -C(=O)NHCH₃, -C(=O)N(CH₃)₂, -C(=O)-(C₁-C₆-Alkyl), -C(=O)-(C₁-C₆-Alkoxy), -NHS(=O)₂-(C₁-C₆-Alkyl), -N(C₁₋₆ Alkyl)S(=O)₂-(C₁-C₆-Alkyl), -S(=O)₂-(C₁-C₆-Alkyl), C₁-C₆-Alkyl, C₂-C₆-Alkenyl, C₂-C₆-Alkinyl, C₁-C₆-Halogenalkyl, C₁-C₆-Alkoxy, C₁-C₆-Haloalkoxy, C₁-C₆-Alkylthio, C₁-C₆-Alkylamino, hydroxysubstituiertes C₁-C₆-Alkyl, C₃₋₆-Cycloalkyl, 3-8-gliedriges Heterocyclyl, C₆₋₁₀-Aryl oder 5-10-gliedriges Heteroaryl ist;
R⁴ H, D, C₁-C₆-Alkyl, C₂-C₆-Alkenyl, C₂-C₆-Alkinyl, C₁-C₆-Halogenalkyl, C₃₋₆-Cycloalkyl, 3-8-gliedriges Heterocyclyl, C₆₋₁₀-Aryl oder 5-10-gliedriges Heteroaryl ist; wobei R⁴ optional mit 1, 2, 3 oder 4 R^{w} substituiert ist;
jedes R⁵, R⁶ und R⁷ unabhängig voneinander H, D, C₁-C₆-Alkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkylthio, C₁-C₆-Alkylamino, hydroxysubstituiertes C₁-C₆-Alkyl, C₃₋₆-Cycloalkyl, 3-8-gliedriges Heterocyclyl, C₆₋₁₀-Aryl oder 5-10-gliedriges Heteroaryl ist; wobei jedes R⁵, R⁶ und R⁷ unabhängig voneinander und optional mit 1, 2, 3 oder 4 R^{w} substituiert ist;
R⁸ -OH, C₁-C₆-Alkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkylthio, C₁-C₆-Alkylamino, hydroxysubstituiertes C₁-C₆-Alkyl, C₃₋₆-Cycloalkyl, 3-8-gliedriges Heterocyclyl, C₆₋₁₀-Aryl oder 5-10-gliedriges Heteroaryl ist; wobei R⁸ optional mit 1, 2, 3 oder 4 R^{w} substituiert ist;
jedes R^{w} unabhängig =O, D, F, Cl, Br, I, -CN, -NO₂, - NH₂, -OH, -SH, -COOH, -C(=O)NH₂, -C(=O)NHCH₃, - C(=O)N(CH₃)₂, -C(=O)-(C₁-C₆-Alkyl), -C(=O)-(C₁-C₆-Alkoxy), -NHS(=O)₂-(C₁-C₆-Alkyl), -N(C₁₋₆ Alkyl)S(=O)₂-( C₁-C₆-Alkyl), -S(=O)₂-(C₁-C₆-Alkyl), C₁-C₆-Alkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkylthio, C₁-C₆₋ Alkylamino, hydroxysubstituiertes C₁-C₆-Alkyl, C₃₋₆-Cycloalkyl, 3-8-gliedriges Heterocyclyl, C₆₋₁₀ Aryl oder 5-10-gliedriges Heteroaryl ist.

2. Verbindung gemäß Anspruch 1, wobei jedes R¹ und R² unabhängig voneinander H, D, -OH, C₁-C₄-Alkyl, C₂-C₄-Alkenyl, C₂-C₄-Alkinyl, C₁-C₄-Halogenalkyl, C₁-C₄-Alkoxy, C₁-C₄-Halogenalkoxy, C₃₋₆-Cycloalkyl, 3-6-gliedriges Heterocyclyl, C₆₋₁₀-Aryl, 5-6-gliedriges Heteroaryl, C₃₋₆-Cycloalkyl-C₁-₄-Alkylen, (3-6-gliedriges Heterocyclyl)-C₁₋₄-Alkylen, C₆₋₁₀-Aryl-C₁₋₄-Alkylen oder (5-6-gliedriges Heteroaryl)-C₁₋₄-Alkylen ist; wobei jedes R¹ und R² unabhängig voneinander und optional mit 1, 2, 3 oder 4 R^{w} substituiert ist;
jedes R^{3a}, R^{3b}, R^{3c}, R^{3d} und R^{3e} unabhängig voneinander H, D, F, Cl, Br, I, -CN, -NO₂, -NH₂, -OH, -SH, -COOH, - C(=O)NH₂, -C(=O)NHCH₃, -C(=O)N(CH₃)₂, -C(=O)-(C₁-C₄-Alkyl), -C(=O)-(C₁-C₄-Alkoxy), -S(=O)₂-(C₁-C₄-Alkyl), C₁-C₄-Alkyl, C₂-C₄-Alkenyl, C₂-C₄-Alkinyl, C₁-C₄-Halogenalkyl, C₁-C₄-Alkoxy, C₁-C₄-Halogenalkoxy, C₁-C₄-Alkylthio, C₁-C₄-Alkylamino, hydroxysubstituiertes C₁-C₄-Alkyl ist;
jedes R⁵, R⁶ und R⁷ unabhängig voneinander H, D, C₁-C₄-Alkyl, C₂-C₄-Alkenyl, C₂-C₄-Alkinyl, C₁-C₄-Halogenalkyl, C₁-C₄-Alkoxy, C₁-C₄-Halogenalkoxy, C₁-C₄-Alkylthio, C₁-C₄-Alkylamino, hydroxysubstituiertes C₁-C₄-Alkyl, C₃₋₆-Cycloalkyl, 3-6-gliedriges Heterocyclyl, C₆₋₁₀-Aryl oder 5-6-gliedriges Heteroaryl ist; wobei jedes R⁵, R⁶ und R⁷ unabhängig voneinander und optional mit 1, 2, 3 oder 4 R^{w} substituiert ist.

3. Verbindung gemäß Anspruch 1 oder 2, wobei jedes R¹ und R² unabhängig voneinander H, D, -OH, Methyl, Ethyl, *n-*Propyl, *i*-Propyl, *n*-Butyl, *t*-Butyl, 2-Methylpropyl, 1-Methylpropyl, Vinyl, Propenyl, Allyl, Ethinyl, Propinyl, Trifluormethyl, Difluormethyl, Methoxy, Ethoxy, Isopropoxy, Trifluormethoxy, Cyclopropyl, Cyclopentyl, Cyclohexyl, Oxiranyl, Pyrrolidyl, Piperidyl, Morpholinyl, Piperazinyl, Phenyl, Naphthyl, Pyrrolyl, Pyridyl, Pyrimidinyl, Pyrazolyl, Triazolyl, Thiazolyl, Imidazolyl, Tetrazolyl, C₃₋₆-Cycloalkylmethylen, C₃₋₆-Cycloalkylethylen, C₃₋₆-Cycloalkylpropylen, (3-6-gliedriges Heterocyclyl)methylen, (3-6-gliedriges Heterocyclyl)ethylen, Phenylmethylen, Phenylethylen, Phenylpropylen, Pyridylmethylen, Pyrimidinylmethylen, Pyrrolylmethylen, Pyrazolylmethylen, Triazolylmethylen oder Tetrazolylmethylen ist; wobei jedes R¹ und R² unabhängig voneinander und optional mit 1, 2, 3 oder 4 R^{W} substituiert ist;
jedes R^{3a}, R^{3b}, R^{3c}, R^{3d} und R^{3e} unabhängig voneinander H, D, F, Cl, Br, I, -CN, -NO₂, -NH₂, -OH, -SH, -COOH, - C(=O)NH₂, -C(=O)NHCH₃, -C(=O)N(CH₃)₂, -C(=O)-CH₃, - C(=O)-OCH₃, -S(=O)₂CH₃, Methyl, Ethyl, *n*-Propyl, *i-*Propyl, *t*-Butyl, Vinyl, Trifluormethyl, Difluormethyl, Methoxy, Trifluormethoxy, Methylamino, Dimethylamino oder Hydroxymethyl ist;
jedes R⁵, R⁶ und R⁷ unabhängig voneinander H, D, Methyl, Ethyl, *n*-Propyl, *i*-Propyl, *n*-Butyl, *t*-Butyl, 2-Methylpropyl, 1-Methylpropyl, Vinyl, Propenyl, Allyl, Ethinyl, Propinyl, Trifluormethyl, Difluormethyl, 2,2-Difluorethyl, Methoxy, Ethoxy, *i*-Propoxy, *n*-Propoxy, *t-*Butoxy, Trifluormethoxy, C₁-C₄-Alkylthio, C₁-C₄-Alkylamino, hydroxysubstituiertes C₁-C₄-Alkyl, Cyclopropyl, Cyclopentyl, Cyclohexyl, 3-6-gliedriges Heterocyclyl, C₆₋₁₀-Aryl oder 5-6-gliedriges Heteroaryl ist; wobei jedes R⁵, R⁶ und R⁷ unabhängig voneinander und optional mit 1, 2, 3 oder 4 R^{W} substituiert ist.

4. Verbindung gemäß irgendeinem der Ansprüche 1 bis 3, wobei, wenn Y N ist, ist R Pyridyl, Pyrimidinyl, Thienyl, Furyl oder 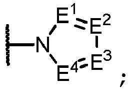 wenn Y CrY ist, ist R 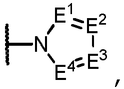 wobei R optional mit 1, 2, 3 oder 4 R⁰ substituiert ist; RY H, D, F, Cl, Br, I, -CN, -NO₂, -NH₂, -OH, -SH, -COOH, -C(=O)NH₂, - C(=O)NHCH₃, -C(=O)N(CH₃)₂, -C(=O)-(C₁-C₄-Alkyl), -C(=O)-(C₁-C₄-Alkoxy), C₁-C₄-Alkyl, C₂-C₄-Alkenyl, C₂-C₄-Alkinyl, C₁-C₄-Halogenalkyl, C₁-C₄-Alkoxy, C₁-C₄-Halogenalkoxy, C₁-C₄-Alkylthio, C₁-C₄-Alkylamino, hydroxysubstituiertes C₁-C₄-Alkyl, C₃₋₆-Cycloalkyl, 3-6-gliedriges Heterocyclyl, Phenyl, Naphthyl, Pyridyl, Pyrimidinyl, Thienyl, Furyl oder 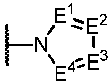 ist; oder R ist H, D, F, Cl, Br, I, -CN, -NO₂, -NH₂, -OH, -SH, - COOH, -C(=O)NH₂, -C(=O)NHCH₃, -C(=O)N(CH₃)₂, -C(=O)-(C1-C₄-Alkyl), -C(=O)-(C₁-C₄-Alkoxy), C₁-C₄-Alkyl, C₂-C₄-Alkenyl, C₂-C₄-Alkinyl, C₁-C₄-Haloalkyl, C₁-C₄-Alkoxy, C₁-C₄-Halogenalkoxy, C₁-C₄-Alkylthio, C₁-C₄-Alkylamino, hydroxysubstituiertes C₁-C₄-Alkyl, C₃₋₆-Cycloalkyl, 3-6-gliedriges Heterocyclyl, Phenyl, Naphthyl, Pyridyl, Pyrimidinyl, Thienyl, Furyl oder 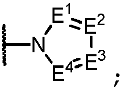 RY ist 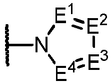 , wobei RY optional mit 1, 2, 3 oder 4 R⁰ substituiert ist;
wobei jedes E¹, E², E³ und E⁴ unabhängig voneinander N oder CH ist.

5. Verbindung gemäß irgendeinem der Ansprüche 1 bis 4, wenn Y N ist, ist R Pyridyl, Pyrimidinyl, Thienyl, Furyl, 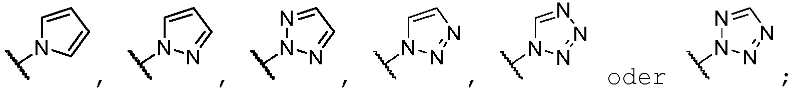 wenn Y CRY ist, ist R 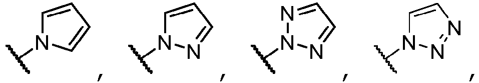 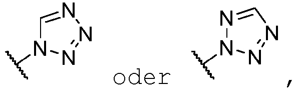 wobei R optional mit 1, 2, 3 oder 4 R⁰ substituiert ist; RY H, D, F, Cl, Br, I, -CN, -NO₂, -NH₂, -OH, -SH, -COOH, -C(=O)NH₂, -C(=O)NHCH₃, -C(=O)N(CH₃)₂, -C(=O)CH₃, - C(=O)OCH₃, Methyl, Ethyl, *n*-Propyl, *i*-Propyl, *t*-Butyl, Vinyl, Ethinyl, Propinyl, Trifluormethyl, Difluormethyl, Methoxy, Ethoxy, i-Propoxy, Trifluormethoxy, Methylthio, Methylamino, Dimethylamino, Hydroxymethyl, C₃₋₆-Cycloalkyl, 3-6-gliedriges Heterocyclyl, Phenyl, Naphthyl, Pyridyl, Pyrimidinyl, Thienyl, Furyl 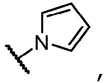 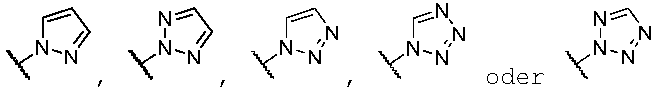 ist;
oder R H, D, F, Cl, Br, I, -CN, - NO₂, -NH₂, -OH, -SH, - COOH, -C(=O)NH₂, -C(=O)NHCH₃, -C(=O)N(CH₃)₂, -C(=O)CH₃, -C(=O)OCH₃, Methyl, Ethyl, *n*-Propyl, *i*-Propyl, *t*-Butyl, Vinyl, Ethinyl, Propinyl, Trifluormethyl, Difluormethyl, Methoxy, Ethoxy, *i*-Propoxy, Trifluormethoxy, Methylthio, Methylamino, Dimethylamino, Hydroxymethyl, C₃₋₆-Cycloalkyl, 3-6-gliedriges Heterocyclyl, Phenyl, Naphthyl, Pyridyl, Pyrimidinyl, Thienyl, Furyl, 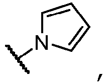 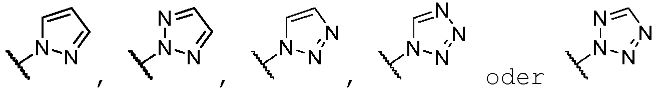 ist; RY 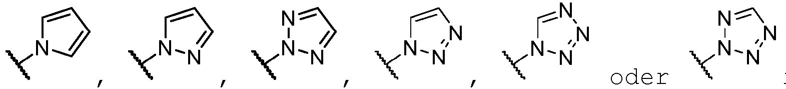 ist, wobei RY optional mit 1, 2, 3 oder 4 R⁰ substituiert ist.

6. Verbindung gemäß irgendeinem der Ansprüche 1 bis 5, wobei jedes R⁰ unabhängig D, F, Cl, Br, I, -CN, -NO₂, - NH₂, -OH, -SH, -COOH, -C(=O)NH₂, -C(=O)NHCH₃, - C(=O)N(CH₃)₂, -C(=O)-(C₁-C₄-Alkyl), -C(=O)-(C₁-C₄-Alkoxy), C₁-C₄-Alkyl, C₂-C₄-Alkenyl, C₂-C₄-Alkinyl, C₁-C₄-Halogenalkyl, C₁-C₄-Alkoxy, C₁-C₄-Halogenalkoxy, C₁-C₄-Alkylthio, C₁-C₄-Alkylamino oder hydroxysubstituiertes C₁-C₄-Alkyl ist;
jedes R^{W} unabhängig =O, D, F, Cl, Br, I, -CN, -NO₂, - NH₂, -OH, -SH, -COOH, -C(=O)NH₂, -C(=O)NHCH₃, - C(=O)N(CH₃)₂, -C(=O)-(C₁-C₄-Alkyl), -C(=O)-(C₁-C₄-Alkoxy), -NHS(=O)₂-(C₁-C₄-Alkyl), -N(C₁₋₄-Alkyl)S(=O)₂-(C₁-C₄-Alkyl), -S(=O)₂-(C₁-C₄-Alkyl), C₁-C₄-Alkyl, C₂-C₄-Alkenyl, C₂-C₄-Alkinyl, C₁-C₄-Halogenalkyl, C₁-C₄-Alkoxy, C₁-C₄-Halogenalkoxy, C₁-C₄-Alkylthio, C₁-C₄-Alkylamino, hydroxysubstituiertes C₁-C₄-Alkyl, C₃₋₆₋Cycloalkyl, 3-6-gliedriges Heterocyclyl, C₆₋₁₀-Aryl oder 5-6-gliedriges Heteroaryl ist.

7. Verbindung gemäß irgendeinem der Ansprüche 1 bis 6, wobei jedes R⁰ unabhängig D, F, Cl, Br, I, -CN, -NO₂, - NH₂, -OH, -SH, -COOH, -C(=O)NH₂, -C(=O)NHCH₃, - C(=O)N(CH₃)₂, -C(=O)-CH₃, -C(=O)-OCH₃, Methyl, Ethyl, n-Propyl, i-Propyl, t-Butyl, Vinyl, Trifluormethyl, Difluormethyl, Methoxy, Trifluormethoxy, Methylamino, Dimethylamino oder Hydroxymethyl ist;
jedes R^{W} unabhängig =O, D, F, Cl, Br, I, -CN, -NO₂, - NH₂, -OH, -SH, -COOH, -C(=O)NH₂, -C(=O)NHCH₃, - C(=O)N(CH₃)₂, -C(=O)CH₃, -C(=O)CH₂CH₃, -C(=O)OCH₃, - C(=O)OCH₂CH₃, -NHS(=O)₂CH₃, -N(CH₃)S(=O)₂CH₃, - NHS(=O)₂CH₂CH₃, -N(CH₂CH₃)S(=O)₂CH₂CH₃, - N(CH₂CH₃)S(=O)₂CH₃, -N(CH₃)S(=O)₂CH₂CH₃, -S(=O)₂CH₃, Methyl, Ethyl, n-Propyl, i-Propyl, n-Butyl, t-Butyl, 2-Methylpropyl, 1-Methylpropyl, Vinyl, Propenyl, Allyl, Ethinyl, Propinyl, Trifluormethyl, Difluormethyl, Methoxy, Ethoxy, Isopropoxy, n-Propoxy, t-Butoxy, Trifluormethoxy, C₁-C₄-Alkylthio, C₁-C₄-Alkylamino, hydroxysubstituiertes C₁-C₄-Alkyl, C₃₋₆-Cycloalkyl, 3-6-gliedriges Heterocyclyl, C₆₋₁₀-Aryl oder 5-6-gliedriges Heteroaryl ist.

8. Verbindung gemäß irgendeinem der Ansprüche 1 bis 7, die eine der folgenden Strukturen oder ein Stereoisomer, ein geometrisches Isomer, ein Tautomer, ein N-Oxid, ein Hydrat, ein Solvat, ein Metabolit, oder ein pharmazeutisch akzeptables Salz davon hat: 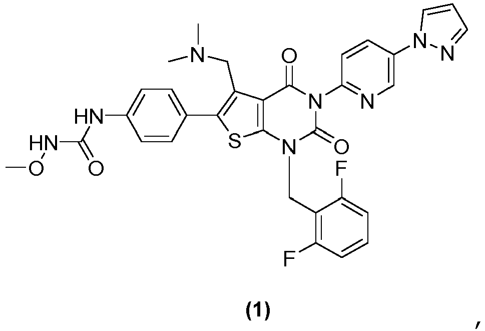 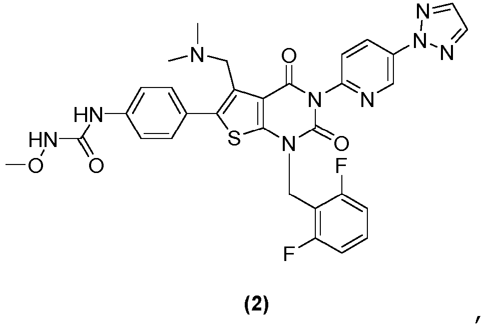 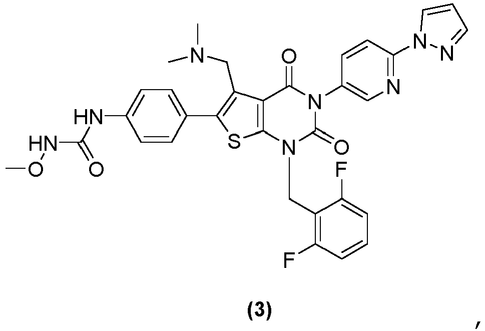 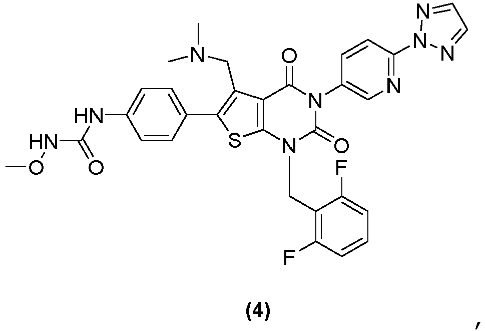 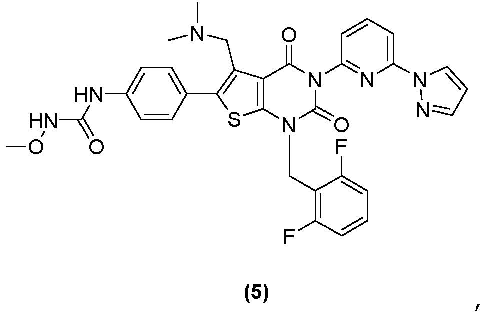 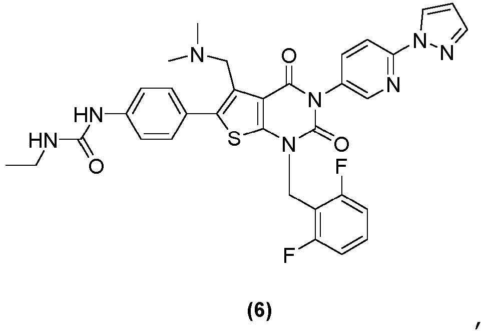 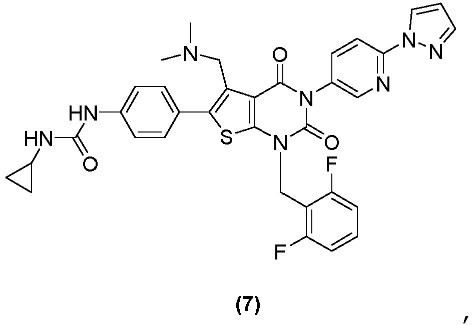 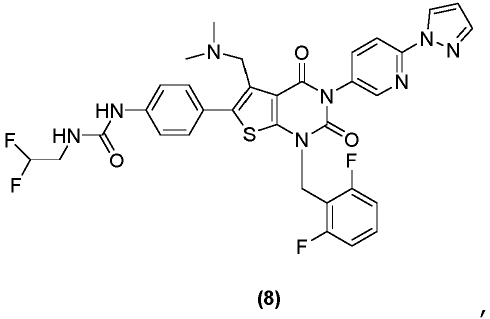 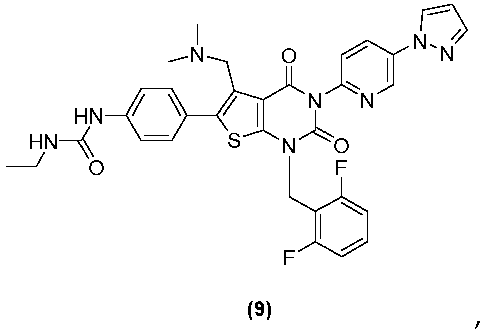 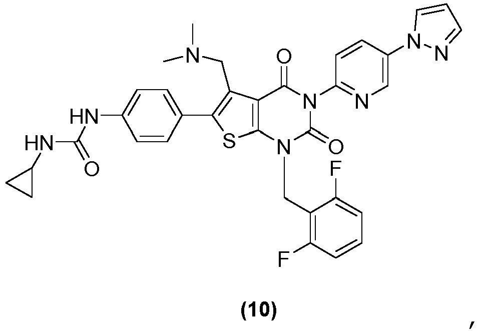 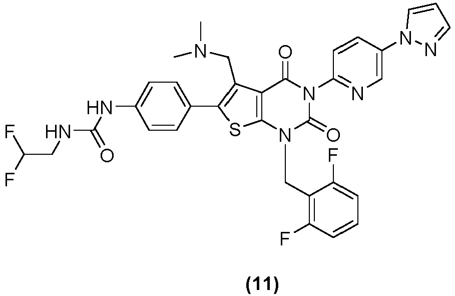 oder

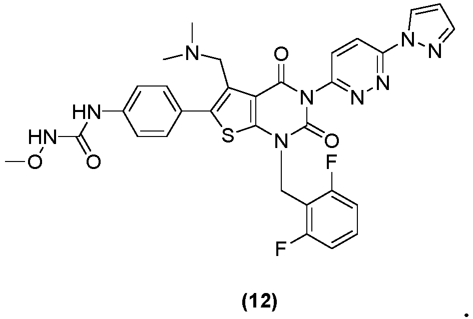

9. Pharmazeutische Zusammensetzung, umfassend die Verbindung gemäß irgendeinem der Ansprüche 1 bis 8; und wobei die pharmazeutische Zusammensetzung optional ferner einen pharmazeutisch akzeptablen Trägerstoff, Träger oder Hilfsstoff oder eine Kombination davon umfasst.

10. Verbindung gemäß irgendeinem der Ansprüche 1 bis 8 oder pharmazeutische Zusammensetzung gemäß Anspruch 9 zur Verwendung bei der Vorbeugung oder Behandlung einer sexualhormonabhängigen Erkrankung bei einem Patienten.

11. Verbindung oder pharmazeutische Zusammensetzung zur Verwendung gemäß Anspruch 10, wobei die sexualhormonabhängige Erkrankung sexualhormonabhängiger Krebs, Knochenmetastase aus einem sexualhormonabhängigen Krebs, Prostatahypertrophie, Hysteromyom, Endometriose, Uterusmyome, vorzeitige Pubertät, Amenorrhö, prämenstruelles Syndrom, Algomenorrhö, multilokuläres Ovarialsyndrom ("multilocular ovary syndrome"), polyzystisches Ovarialsyndrom, Akne, Alopezie, Unfruchtbarkeit oder Reizdarmsyndrom ist.

## Revendications

1. Composé présentant la Formule (I) ou stéréoisomère, isomère géométrique, tautomère, N-oxyde, hydrate, solvate ou sel pharmaceutiquement acceptable de celui-ci, 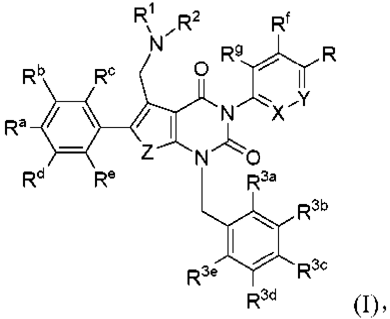 dans lequel
X est CR^{X} ou N ; Y est CR^{y} ou N ;
quand Y est N, R est un hétéroaryle à 5 à 10 chaînons ;
quand Y est CR^{y}, R est un hétéroaryle à 5 à 6 chaînons, dans lequel R est facultativement substitué par 1, 2, 3 ou 4 R° ; R^{y} est H, D, F, Cl, Br, I, -CN, -NO₂, -NH₂, - OH, -SH, -COOH, -C(=O)NH₂, - C(=O)NHCH₃, -C(=O)N(CH₃)₂, -C(=O)-(C₁-C₆ alkyle), - C(=O)-(C₁-C₆ alcoxy), C₁-C₆ alkyle, C₂- Ce alcényle, C₂-Ce alcynyle, C₁-C₆ haloalkyle, C₁-C₆ alcoxy, C₁-C₆ haloalcoxy, C₁-C₆ alkylthio, C₁-C₆ alkylamino, alkyle Ci-Ce substitué par un hydroxy, C₃-₆ cycloalkyle, hétérocyclyle à 3 à 8 chaînons, aryle C_{6- 10} ou hétéroaryle à 5 à 10 chaînons ; ou
R est H, D, F, Cl, Br, I, -CN, -NO₂, -NH₂, -OH, -SH, -COOH, -C(=O)NH₂, - C(=O)NHCH₃, - C(=O)N(CH₃)₂, -C(=O)-( C₁-C₆ alkyle), -C(=O)-( C₁-C₆ alcoxy), C₁-C₆ alkyle, C₂-C₆ alcényle, C₂-C₆ alcynyle, C₁-C₆ haloalkyle, C₁-C₆ alcoxy, C₁-C₆ haloalcoxy, C₁-C₆ alkylthio, C₁-C₆ alkylamino, C₁-C₆ alkyle substitué par un hydroxy, C₃-₆ cycloalkyle, hétérocyclyle à 3 à 8 chaînons, C₁₋₁₀ aryle ou hétéroaryle à 5 à 10 chaînons ; R^{y} est un hétéroaryle à 5 à 6 chaînons, dans lequel R^{y} est facultativement substitué par 1, 2, 3 ou 4 R⁰ ;
Z est NR", S ou O ;
Rⁿ est H, D, C₁₋₆ alkyle, C₂-C₆ alcényle, C₂-C₆ alcynyle ou C₁-C₆ haloalkyle ;
chaque R^{b}, R^{c}, R^{d}, R^{e}, R^{f}, R^{g} et R^{x} est indépendamment H, D, F, Cl, Br, I, -CN, -NO₂, -NH₂, -OH, -SH, -COOH, -C(=O)NH₂, -C(=O)NHCH₃, -C(=O)N(CH₃)₂, -C(=O)-(C₁-C₆ alkyle), -C(=O)-(C₁- C₆ alcoxy), C₁-C₆ alkyle, C₂-C₆ alcényle, C₂-C₆ alcynyle, C₁-C₆ haloalkyle, C₁-C₆ alcoxy, C₁-C₆ haloalcoxy, C₁-C₆ alkylthio, C₁-C₆ alkylamino, C₁-C₆ alkyle substitué par un hydroxy, C₃-₆ cycloalkyle, hétérocyclyle à 3 à 8 chaînons, C₆₋₁₀aryle ou hétéroaryle à 5 à 10 chaînons ;
R^{a} est -CN, -NO₂, -OR⁴, -NR⁵R⁶, -NR⁷C(=O)R⁸, -NR⁷C(=O)NR⁵R⁶, -NR⁷S(=O)ₜR⁸, - NR⁷S(=O)ₜNR⁵R⁶, -C(=O)R⁸, -C(=O)NR⁵R⁶, -S(=O)ₜNR⁵R⁶ ou -S(=O)ₜR⁸ ; dans lequel t vaut 0, 1 ou 2 ;
chaque R⁰ est indépendamment D, F, Cl, Br, I, -CN, -NO₂, -NH₂, -OH, -SH, -COOH, -C(=O)NH₂, - C(=O)NHCH₃, -C(=O)N(CH₃)₂, -C(=O)-(C₁-C₆ alkyle), -C(=O)-(C₁-C₆ alcoxy), C₁-C₆ alkyle, C₂- C₆ alcényle, C₂-C₆ alcynyle, C₁-C₆ haloalkyle, C₁-C₆ alcoxy, C₁-C₆ haloalcoxy, C₁-C₆ alkylthio, C₁- C₆ alkylamino ou C₁-C₆ alkyle substitué par un hydroxy ;
chaque R¹ et R² est indépendamment H, D, -OH, C₁-C₆ alkyle, C₂-C₆ alcényle, C₂-C₆ alcynyle, C₁-C₆ haloalkyle, C₁-C₆ alcoxy, C₁-C₆ haloalcoxy, C₃-₆ cycloalkyle, hétérocyclyle à 3 à 8 chaînons, C₆₋₁₀ aryle, hétéroaryle à 5 à 10 chaînons, C₃-₆ cycloalkyle-C₁₋₆ alkylène, C₁₋₆ alkylène-(hétérocyclyle à 3 à 8 chaînons), C₆₋₁₀ aryle-C₁₋₆ alkylène ou C₁₋₆ alkylène-(hétéroaryle à 5 à 10 chaînons) ; dans lequel chaque R¹ et R² est indépendamment et facultativement substitué par 1, 2, 3 ou 4 R^{w} ;
chaque R^{3a}, R^{3b}, R^{3c}, R^{3d} et R^{3e} est indépendamment H, D, F, Cl, Br, I, -CN, -NO₂, - NH₂, -OH, - SH, -COOH, -C(=O)NH₂, -C(=O)NHCH₃, -C(=O)N(CH₃)₂, -C(=O)-(Cl-C₆ alkyle), -C(=O)-(C₁-C₆ alcoxy), -NHS(=O)₂-(C₁-C₆ alkyle), -N(C₁-₆ alkyle)S(=O)₂-(C₁-C₆ alkyle), - S(=O)₂-(C₁-C₆ alkyle), C₁-C₆ alkyle, C₂-C₆ alcényle, C₂-C₆ alcynyle,
C₁-C₆ haloalkyle, C₁-C₆ alcoxy, C₁-C₆ haloalcoxy, C₁-C₆ alkylthio, C₁-C₆ alkylamino, C₁-C₆ alkyle substitué par un hydroxy, C₃-₆ cycloalkyle, hétérocyclyle à 3 à 8 chaînons, C₆₋₁₀ aryle ou hétéroaryle à 5 à 10 chaînons ;
R⁴ est H, D, C₁-C₆ alkyle, C₂-C₆ alcényle, C₂-C₆ alcynyle, C₁-C₆ haloalkyle, C₃-₆ cycloalkyle, hétérocyclyle à 3 à 8 chaînons, C₆₋₁₀ aryle ou hétéroaryle à 5 à 10 chaînons ; dans lequel R⁴ est facultativement substitué par 1, 2, 3 ou 4 R^{w} ;
chaque R⁵, R⁶ et R⁷ est indépendamment H, D, C₁-C₆ alkyle, C₂-C₆ alcényle, C₂-C₆ alcényle, C₁-C₆ haloalkyle, C₁-C₆ alcoxy, C₁-C₆ haloalcoxy, C₁-C₆ alkylthio, C₁-C₆ alkylamino, C₁-C₆ alkyle substitué par un hydroxy, C₃-₆ cycloalkyle, hétérocyclyle à 3 à 8 chaînons, C₆₋₁₀ aryle ou hétéroaryle à 5 à 10 chaînons ; dans lequel chaque R⁵, R⁶ et R⁷ est indépendamment et facultativement substitué par 1, 2, 3 ou 4 R^{w} ;
R⁸ est -OH, C₁-C₆ alkyle, C₂-C₆ alcényle, C₂-C₆ alcynyle, C₁-C₆ haloalkyle, C₁-C₆ alcoxy, C₁-C₆ haloalcoxy, C₁-C₆ alkylthio, C₁-C₆ alkylamino, C₁-C₆ alkyle substitué par un hydroxy, C₃-₆ cycloalkyle, hétérocyclyle à 3 à 8 chaînons, C₆₋₁₀ aryle ou hétéroaryle à 5 à 10 chaînons ; dans lequel R⁸ est facultativement substitué par 1, 2, 3 ou 4 R^{w} ;
chaque R^{w} est indépendamment =0, D, F, Cl, Br, I, -CN, -NO₂, -NH₂, -OH, -SH, - COOH, - C(=O)NH₂, -C(=O)NHCH₃, -C(=O)N(CH₃)₂, -C(=O)-(C₁-C₆ alkyle), -C(=O)-( C₁-C₆ alcoxy), - NHS(=O)₂-(Cl-C₆ alkyle), -N(C₁-₆ alkyle)S(=O)₂-( C₁-C₆ alkyle), -S(=O)₂-( C₁-C₆ alkyle), C₁-C₆ alkyle, C₂-C₆ alcényle, C₂-C₆ alcynyle, C₁-C₆ haloalkyle, C₁-C₆ alcoxy, C₁-C₆ haloalcoxy, C₁-C₆ alkylthio, C₁-C₆ alkylamino, C₁-C₆ alkyle substitué par un hydroxy, C₃-₆ cycloalkyle, hétérocyclyle à 3 à 8 chaînons, C₆₋₁₀ aryle ou hétéroaryle à 5 à 10 chaînons.

2. Composé selon la revendication 1, dans lequel chaque R¹ et R² est indépendamment H, D, -OH, C₁-C₄alkyle, C₂-C₄ alcényle, C₂-C₄ alcynyle, C₁-C₄ haloalkyle, C₁-C₄ alcoxy, C₁-C₄ haloalcoxy, C₃-₆ cycloalkyle, hétérocyclyle à 3 à 6 chaînons, C₆₋₁₀ aryle, hétéroaryle à 5 à 6 chaînons, C₃-₆ cycloalkyle-C₁-₄ alkylène, (hétérocyclyle à 3 à 6 chaînons)-C₁-₄ alkylène, C₆₋₁₀ aryle-C₁₋₄ alkylène ou C₁-₄ alkylène-(hétéroaryle à 5 à 6 chaînons) ; dans lequel chaque R¹ et R² est indépendamment et facultativement substitué par 1, 2, 3 ou 4R^{w} ;
chaque R^{3a}, R^{3b}, R^{3c}, R^{3d} et R^{3e} est indépendamment H, D, F, Cl, Br, I, -CN, -NO₂, - NH₂, -OH, - SH, -COOH, -C(=O)NH₂, -C(=O)NHCH₃, -C(=O)N(CH₃)₂, -C(=O)-(C₁-C₄ alkyle), -C(=O)-(C₁-C₄ alcoxy), -S(=O)₂-( C₁-C₄ alkyle), C₁-C₄ alkyle, C₂-C₄ alcényle, C₂-C₄ alcynyle, C₁-C₄ haloalkyle, C₁-C₄ alcoxy, C₁-C₄ haloalcoxy, C₁-C₄ alkylthio, C₁-C₄ alkylamino, C₁-C₄ alkyle substitué par un hydroxy ;
chaque R⁵, R⁶ et R⁷ est indépendamment H, D, C₁-C₄ alkyle, C₂-C₄ alcényle, C₂-C₄ alcynyle, C₁-C₄ haloalkyle, C₁-C₄ alcoxy, C₁-C₄ haloalcoxy, C₁-C₄ alkylthio, C₁-C₄ alkylamino, C₁-C₄ alkyle substitué par un hydroxy, C₃-₆ cycloalkyle, hétérocyclyle à 3 à 6 chaînons, C₆₋₁₀ aryle ou hétéroaryle à 5 à 6 chaînons ; dans lequel chaque R⁵, R⁶ et R⁷ est indépendamment et facultativement substitué par 1, 2, 3 ou 4R^{W}.

3. Composé selon la revendication 1 ou revendication 2, dans lequel chaque R¹ et R² est indépendamment H, D, -OH, méthyle, éthyle, *n*-propyle, *l*-propyle, *n*-butyle, *t*-butyle, 2-méthylpropyle, 1-méthylpropyle, vinyle, propényle, allyle, éthynyle, propynyle, trifluorométhyle, difluorométhyle, méthoxy, éthoxy, isopropoxy, trifluorométhoxy, cyclopropyle, cyclopentyle, cyclohexyle, oxiranyle, pyrrolidyle, pipéridyle, morpholinyle, pipérazinyle, phényle, naphtyle, pyrrolyle, pyridyle, pyrimidinyle, pyrazolyle, triazolyle, thiazolyle, imidazolyle, tétrazolyle, C₃-₆ cycloalkylméthylène, C₃-₆ cycloalkyléthylène, C₃-₆ cycloalkylpropylène, méthylène(hétérocyclyle à 3 à 6 chaînons), éthylène(hétérocyclyle à 3 à 6 chaînons), phénylméthylène, phényléthylène, phénylpropylène, pyridylméthylène, pyrimidinylméthylène, pyrrolylméthylène, pyrazolylméthylène, triazolylméthylène ou tétrazolylméthylène ; dans lequel chaque R¹ et R² est indépendamment et facultativement substitué par 1, 2, 3 ou 4 R^{w} ;
chaque R^{3a}, R^{3b}, R^{3e}, R^{3d} et R^{3e} est indépendamment H, D, F, Cl, Br, I, -CN, -NO₂, - NH₂, -OH, - SH, -COOH, -C(=O)NH₂, -C(=O)NHCH₃, -C(=O)N(CH₃)₂, -C(=O)-CH₃, -C(=O)-OCH₃, - S(=O)₂CH₃, méthyle, éthyle, n-propyle, i-propyle, t-butyle, vinyle, trifluorométhyle, difluorométhyle, méthoxy, trifluorométhoxy, méthylamino, diméthylamino ou hydroxyméthyle ;
chaque R⁵, R⁶ et R⁷ est indépendamment H, D, méthyle, éthyle, n-propyle, i-propyle, n-butyle, t-butyle, 2-méthylpropyle, 1-méthylpropyle, vinyle, propényle, allyle, éthynyle, propynyle, trifluorométhyle, difluorométhyle, 2,2-difluoroéthyle, méthoxy, éthoxy, z-propoxy, «-propoxy, /-butoxy, trifluorométhoxy, C₁-C₄ alkylthio,
C₁-C₄ alkylamino, C₁-C₄ alkyle substitué par un hydroxy, cyclopropyle, cyclopentyle, cyclohexyle, hétérocyclyle à 3 à 6 chaînons, C₆₋₁₀ aryle ou hétéroaryle à 5 à 6 chaînons ; dans lequel chaque R⁵, R⁶ et R⁷ est indépendamment et facultativement substitué par 1, 2, 3 ou 4 R^{w}.

4. Composé selon l'une quelconque des revendications 1 à 3, dans lequel quand Y est N, R est pyridyle, pyrimidinyle, thiényle, furyle ou 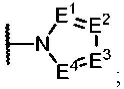
quand Y est CR^{y}, R est 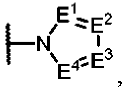 dans lequel R est facultativement substitué par 1, 2, 3 ou 4 R° ; R^{y} est H, D, F, Cl, Br, I, -CN, -NO₂, -NH₂, -OH, -SH, -COOH, -C(=O)NH₂, - C(=O)NHCH₃, - C(=O)N(CH₃)₂, -C(=O)-(C₁-C₄ alkyle), -C(=O)-(C₁-C₄ alcoxy), C₁-C₄ alkyle, C₂-C₄ alcényle, C₂-C₄ alcynyle, C₁-C₄ haloalkyle, C₁-C₄ alcoxy, C₁-C₄ haloalcoxy, C₁-C₄ alkylthio, C₁-C₄ alkylamino, C₁-C₄ alkyle substitué par un hydroxy, C₃-₆ cycloalkyle, hétérocyclyle à 3 à 6 chaînons, phényle, naphtyle, pyridyle, pyrimidinyle, thiényle, furyle ou 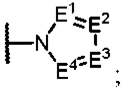 or
R est H, D, F, Cl, Br, I, -CN, -NO₂, -NH₂, -OH, -SH, -COOH, -C(=O)NH₂, - C(=O)NHCH₃, - C(=O)N(CH₃)₂, -C(=O)-(C₁-C₄ alkyle), -C(=O)-(C₁-C₄ alcoxy), C₁-C₄ alkyle, C₂-C₄ alcényle, C₂-C₄ alcynyle, C₁-C₄ haloalkyle, C₁-C₄ alcoxy, C₁-C₄ haloalcoxy, C₁-C₄ alkylthio, C₁-C₄ alkylamino, C₁-C₄ alkyle substitué par un hydroxy, C₃-₆ cycloalkyle, hétérocyclyle à 3 à 6 chaînons, phényle, naphtyle, pyridyle, pyrimidinyle, thiényle, furyle ou 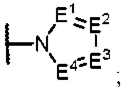 R^{y} est 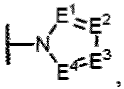 dans lequel R^{y} est facultativement substitué par 1, 2, 3 ou 4 R° ;
dans lequel chaque E¹, E², E³ et E⁴ est indépendamment N ou CH.

5. Composé selon l'une quelconque des revendications 1 à 4, quand Y est N, R est pyridyle, pyrimidinyle, thiényle, furyle, 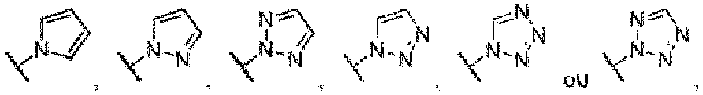
quand Y est CR^{y}, R est 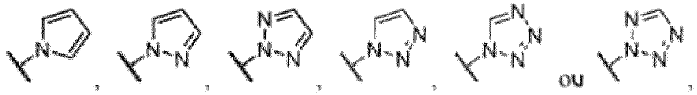
dans lequel R est facultativement substitué par 1, 2, 3 ou 4 R⁰ ; R^{y} est H, D, F, Cl, Br, I, -CN, -NO2, -NH2, -OH, -SH, -COOH, -C(=O)NH₂, -C(=O)NHCH₃, -C(=O)N(CH₃)₂, - C(=O)CH₃, -C(=O)OCH₃, méthyle, éthyle, n-propyle, i-propyle, t-butyle, vinyle, éthynyle, propynyle, trifluorométhyle, difluorométhyle, méthoxy, éthoxy, z-propoxy, trifluorométhoxy, méthylthio, méthylamino, diméthylamino, hydroxyméthyle, C₃-6 cycloalkyle, hétérocyclyle à 3 à 6 chaînons, phényle, naphtyle, pyridyle, pyrimidinyle, thiényle, furyle, 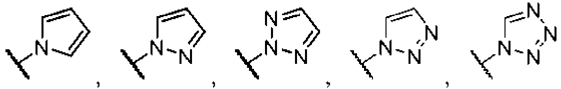 ou 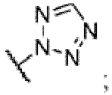 ou
R est H, D, F, Cl, Br, I, -CN, -NO₂, -NH₂, -OH, -SH, -COOH, -C(=O)NH₂, - C(=O)NHCH₃, - C(=O)N(CH₃)₂, -C(=O)CH₃, -C(=O)OCH₃, méthyle, éthyle, *n*-propyle, *i-*propyle, t-butyle, vinyle, éthynyle, propynyle, trifluorométhyle, difluorométhyle, méthoxy, éthoxy, z-propoxy, trifluorométhoxy, méthylthio, méthylamino, diméthylamino, hydroxyméthyle, C₃-₆ cycloalkyle, hétérocyclyle à 3 à 6 chaînons, phényle, naphtyle, pyridyle, pyrimidinyle, thiényle, furyle, 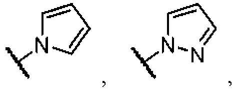 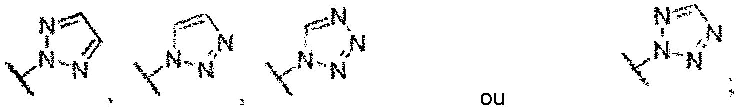 R^{y} est 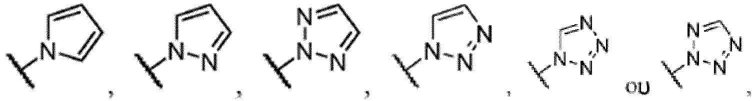 dans lequel R^{y} est facultativement substitué par 1, 2, 3 ou 4 R⁰.

6. Composé selon l'une quelconque des revendications 1 à 5, dans lequel chaque R⁰ est indépendamment D, F, Cl, Br, I, -CN, -NO₂, -NH₂, -OH, -SH, -COOH, -C(=O)NH₂, - C(=O)NHCH₃, -C(=O)N(CH₃)₂, -C(=O)- (C₁-C₄ alkyle), -C(=O)-( C₁-C₄ alcoxy), C₁-C₄ alkyle, C₂-C₄ alcényle, C₂-C₄ alcynyle, C₁-C₄ haloalkyle, C₁-C₄ alcoxy, C₁-C₄ haloalcoxy, C₁-C₄ alkylthio, C₁-C₄ alkylamino ou C₁-C₄ alkyle substitué par un hydroxy ;
chaque R^{w} est indépendamment =O, D, F, Cl, Br, I, -CN, -NO₂, -NH₂, -OH, -SH, - COOH, - C(=O)NH₂, -C(=O)NHCH₃, -C(=O)N(CH₃)₂, -C(=O)-(C₁-C₄ alkyle), -C(=O)-( C₁-C₄ alcoxy), - NHS(=O)₂-(C₁-C₄ alkyle), -N(C₁-C₄ alkyle)S(=O)₂-( C₁-C₄ alkyle), -S(=O)₂-( C₁-C₄ alkyle), C₁-C₄ alkyle, C₂-C₄ alcényle, C₂-C₄ alcynyle, C₁-C₄ haloalkyle, C₁-C₄ alcoxy, C₁-C₄ haloalcoxy, C₁-C₄ alkylthio, C₁-C₄ alkylamino, C₁-C₄ alkyle substitué par un hydroxy, C₃-₆ cycloalkyle, hétérocyclyle à 3 à 6 chaînons, C₆₋₁₀ aryle ou hétéroaryle à 5 à 6 chaînons.

7. Composé selon l'une quelconque des revendications 1 à 6, dans lequel chaque R⁰ est indépendamment D, F, Cl, Br, I, -CN, -NO₂, -NH₂, -OH, -SH, -COOH, -C(=O)NH₂, - C(=O)NHCH₃, -C(=O)N(CH₃)₂, -C(=O)- CH₃, -C(=O)-OCH₃, méthyle, éthyle, n-propyle, *i-*propyle, t-butyle, vinyle, trifluorométhyle, difluorométhyle, méthoxy, trifluorométhoxy, méthylamino, diméthylamino ou hydroxyméthyle ;
chaque R^{w} est indépendamment =O, D, F, Cl, Br, I, -CN, -NO₂, -NH₂, -OH, -SH, - COOH, - C(=O)NH₂, -C(=O)NHCH₃, -C(=O)N(CH₃)₂, -C(=O)CH₃, -C(=O)CH₂CH₃, - C(=O)OCH₃, - C(=O)OCH₂CH₃, -NHS(=O)₂CH₃, -N(CH₃)S(=O)₂CH₃, -NHS(=O)₂CH₂CH₃, N(CH₂CH₃)S(=O)₂CH₂CH₃, -N(CH₂CH₃)S(=O)₂CH₃, -N(CH₃)S(=O)₂CH₂CH₃, -S(=O)₂CH₃, méthyle, éthyle, *n*-propyle, *i*-propyle, *n*-butyle, *t*-butyle, 2-méthylpropyle, 1-méthylpropyle, vinyle, propényle, allyle, éthynyle, propynyle, trifluorométhyle, difluorométhyle, méthoxy, éthoxy, isopropoxy, *n*-propoxy, *t*-butoxy, trifluorométhoxy, C₁-C₄ alkylthio, C₁-C₄ alkylamino, C₁-C₄ alkyle substitué par un hydroxy, C₃-₆ cycloalkyle, hétérocyclyle à 3 à 6 chaînons, C₆₋₁₀ aryle ou hétéroaryle à 5 à 6 chaînons.

8. Composé selon l'une quelconque des revendications 1 à 7 présentant l'une des structures suivantes ou stéréoisomère, isomère géométrique, tautomère, N-oxyde, hydrate, solvate ou sel pharmaceutiquement acceptable de celui-ci :

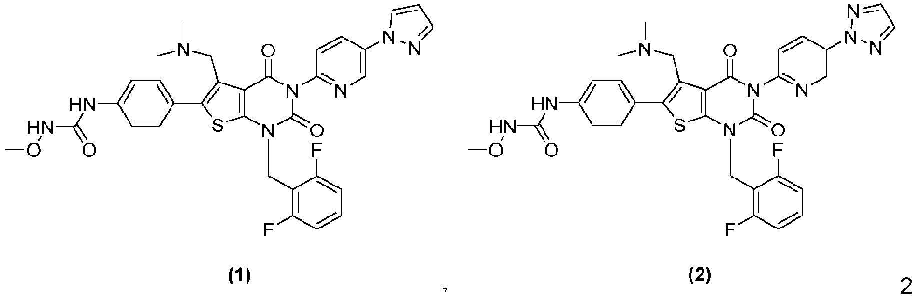

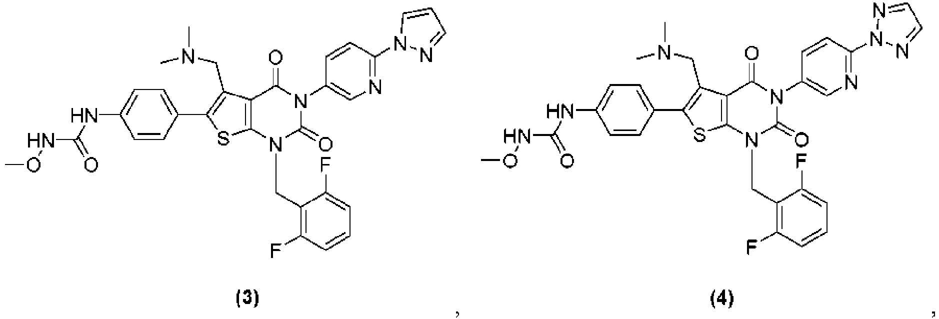

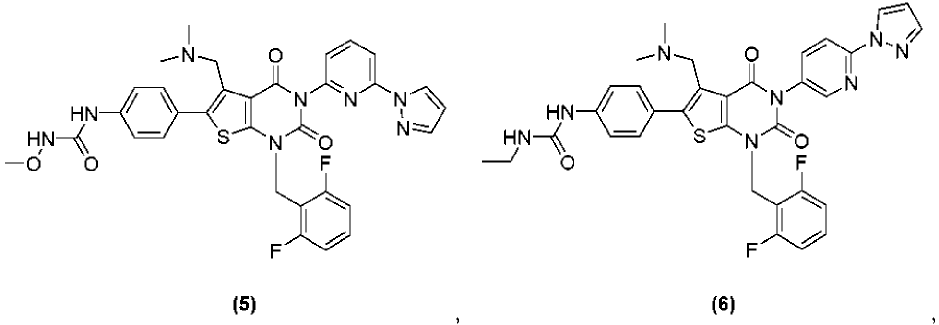

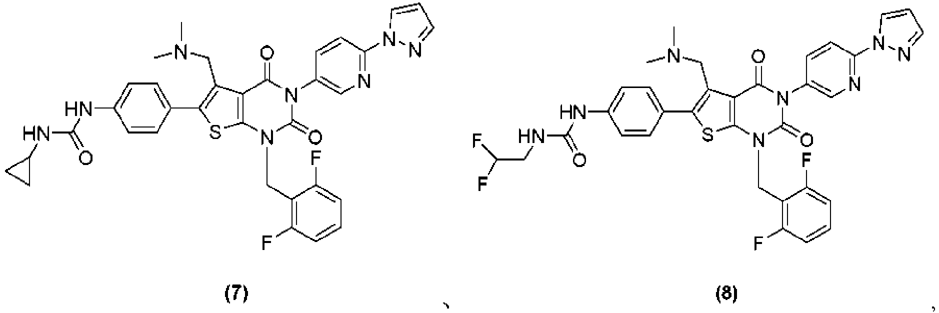

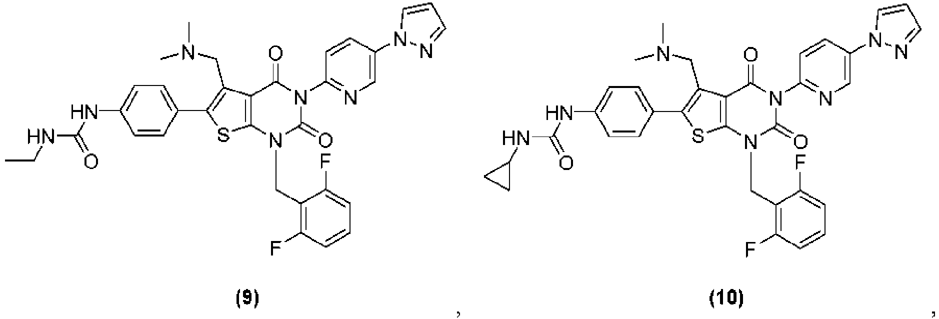

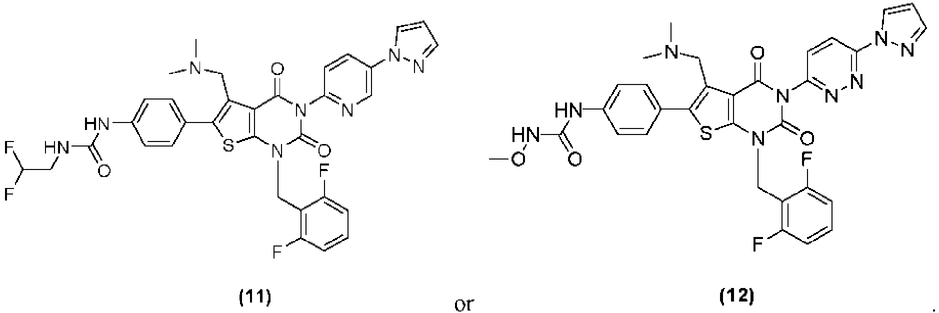

9. Composition pharmaceutique comprenant le composé selon l'une quelconque des revendications 1 à 8 ; et
dans laquelle la composition pharmaceutique comprend en outre facultativement un excipient, un vecteur ou un adjuvant pharmaceutiquement acceptable, ou une combinaison de ceux-ci.

10. Composé selon l'une quelconque des revendications 1 à 8 ou composition pharmaceutique selon la revendication 9 pour utilisation dans la prévention ou le traitement d'une maladie dépendant des hormones sexuelles chez un patient ;

11. Composé ou composition pharmaceutique pour l'utilisation selon la revendication 10, dans lequel la maladie dépendant des hormones sexuelles est un cancer dépendant des hormones sexuelles, une métastase osseuse issue d'un cancer dépendant des hormones sexuelles, une hypertrophie prostatique, l'hystéromyome, l'endométriose, des fibromes utérins, la puberté précoce, l'aménorrhée, le syndrome prémenstruel, l'algoménorrhée, le syndrome des ovaires multiloculaires, le syndrome des ovaires polykystiques, l'acné, l'alopécie, l'infertilité ou le syndrome du côlon irritable.
